# EUROPÄISCHE PATENTANMELDUNG

(11) **EP 3 875 146 A1**
(43) Veröffentlichungstag der Anmeldung: **08.09.2021**
(21) Anmeldenummer: 21160275.0
(22) Anmeldetag: 02.03.2021
(51) Int. Cl.: A61N 5/06

(54) **BELEUCHTUNGSMITTEL, INSBESONDERE ZUR REDUKTION VON KEIMEN, MIT VORZUGSWEISE WENIGSTENS ZWEI LICHTQUELLEN, SOWIE VERFAHREN ZUR NUTZUNG DES BELEUCHTUNGSMITTELS ALS MOBILES DESINFEKTIONSMITTEL**

(30) Priorität: 02.03.2020 DE 102020105567; 03.04.2020 DE 202020101826 U; 28.04.2020 EP 20171941; 14.05.2020 AT 5010120 U
(71) Anmelder: RP-Technik GmbH, 63110 Rodgau (DE)
(72) Erfinder: Pasedag, Roland, 63110 Rodgau (DE); Pasedag, Reinald, 63110 Rodgau (DE)
(74) Vertreter: Cremer & Cremer

(57) **Zusammenfassung**

Ein Bestrahlungssteckstück kann derart gestaltet sein, dass es wie ein Schnuller im vorderen Mundraum, z. B. an Zunge und Gaumen, anliegen kann. Der nicht für die Anlage vorgesehene Bereich des Bestrahlungssteckstücks, genauer des transluzenten Einführteils, ist für die Abgabe von Licht wenigstens in einen posterioren Mundhöhlenbereich aus wenigstens einer Lichtquelle, die im Inneren des Einführteil angeordnet ist, vorgesehen.

Gem. einem vorteilhaften Verfahren lässt sich das Bestrahlungssteckstück mit einem solchen Einführteil herstellen, der eine abgestimmte Länge für eine ganz bestimmte Lage oder Position in einer Mundhöhle eines Nutzers hat. Der ausgewählte Einführteil ist aus einem Satz von Einführteilen auswählbar. Der Einführteil ist über einen Steckverbinder an eine Steuerplatine in einem blockartigen Körper des Bestrahlungssteckstücks anzuschließen.

Durch Verwendung eines solchen Bestrahlungssteckstücks ist es möglich, die Keimzahl, insbesondere im Rachenbereich eines Menschen, aber auch auf Sitzflächen durch Lichtbestrahlung mit Licht keimtötend wirkender Wellenlängen zu reduzieren. So ist es möglich, ein tragbares Desinfektionsgerät an der Hand zu haben.

## Beschreibung

Die vorliegende Erfindung behandelt ein Bestrahlungssteckstück, das teilweise in eine Körperhöhle eines Lebewesens wie eines Menschen eingeführt werden kann, um insbesondere den Gesundheitszustand des Menschen zu fördern oder zu verbessern.

Des Weiteren behandelt die vorliegende Erfindung ein Verfahren zur Verwendung des Bestrahlungssteckstücks als Lichtquelle zur Einwirkung eines Lichts auf einen Bereich einer Körperhöhle wie z. B. einen Rachenbereich eines Lebewesens.

Mit anderen Worten, die vorliegende Erfindung behandelt ein Bestrahlungssteckstück nach dem Oberbegriff von Anspruch 1 und ein Verfahren zur Anpassung des Bestrahlungssteckstücks an eine Körperöffnung oder Körperhöhle eines Lebewesens nach Anspruch 18.

### Technisches Gebiet

Aus zahlreichen medizinischen Untersuchungen ist bekannt, dass mit Hilfe von Licht ausgewählter Wellenlängen positive Effekte bei äußerlich bestrahlten Personen sowie bei einer Effusion von Körperflüssigkeiten wie Blut oder Plasma (siehe z. B. "A new proof of concept in bacterial reduction" von Michelle Maclean et al. in Journal of Blood Transfusion, Volume 2016) herbeigeführt werden kann. Sowohl aus der Fachliteratur als auch aus der Patentliteratur lassen sich Hinweise entnehmen, dass nicht nur ultraviolettes Licht und infrarotes Licht positive Effekte bei dem Bestrahlen von Lebewesen, wie Menschen oder Tieren, sondern auch einzelne Frequenzen und Frequenzbänder im optischen bzw. sichtbaren Wellenspektrum ebenfalls positive Effekte hervorrufen können.

Das für Menschen sichtbare Wellenspektrum wird üblicherweise in einem Wellenlängenbereich zwischen 400 nm und einem Wellenlängenbereich unterhalb von 750 nm angesiedelt. Die Breite und die exakte Lage des Frequenzbandes, also welche Wellenlängen tatsächlich sichtbarem Licht entsprechen, bzw. die Wellenlängengrenzwerte des Wellenlängenbandes variieren etwas von Mensch zu Mensch. Auch gilt bei manchen Tieren ein etwas anderes Wellenband als sichtbares Lichtwellenband, d. h. in dem jene Tiere Licht optisch wahrnehmen können (z. B. ist das Wellenlängenband bei Honigbienen um ca. 150 nm in Richtung auf das kurzwelligere UV-Licht verschoben). Generell kann aber gesagt werden, dass als optisch sichtbares Wellenlängenband ein Wellenlängenband angesehen werden kann, das zwar in den Randbereichen bzw. bezüglich seiner Grenzwerte um einige nm variieren kann, das jedoch eine Bandbreite von ca. 350 nm überstreicht. Dieser Wellenlängenbereich wird gemeinhin als sichtbarer Wellenlängenbereich bezeichnet, dessen Grenzwerte auf das jeweils adressierte Lebewesen konkret anzupassen sind.

Außerhalb des sichtbaren Wellenlängenbereichs ist der ultraviolette Wellenlängenbereich angesiedelt. Ultraviolettes Licht in vernünftigen Dosen hat medizinisch positive Wirkung. Es ist aber auch aus der Fachliteratur genauso wie aus der Patentliteratur bekannt, dass der Sonnenbestrahlung der Effekt der aktinischen Keratose zugeordnet wird.

Auch lassen sich in der Fachliteratur Stimmen finden, die erläutern, dass gewisse Wellenlängen im blauen Spektrum und/oder violetten Spektrum verschiedene positive Effekte beim Menschen hervorrufen können sollen.

So erläutert z. B. der Fachaufsatz "New Proof-of-Concept in Viral Inactivation: Virucidal Efficacy of 405 nm Light Against Feline Calicivirus as a Model for Norovirus Decontamination" von Rachael M. Tomb, Michelle Maclean, John E. Coia, Elizabeth Graham, Michael McDonald, Chintamani D. Atreya, Scott J. MacGregor und John G. Anderson, veröffentlicht am 31. Dezember 2016 in "Food and Environmental Virology", dass mit Hilfe eines Lichts der Wellenlänge 405 nm bakterizide, fungizide und auch viruzide Inaktivierungen durch oxidative Schädigung der Bakterien, Pilze und Viren herbeigeführt werden kann.

Der Fachaufsatz von der University of Arizona mit dem Titel "Blue light can help heal mild traumatic brain injury", veröffentlicht am 15. Januar 2020 auf Science Daily, schlägt vor, morgens eine Blaulichttherapie Leuten zuteilwerden zu lassen, die ein Gehirntrauma erlitten haben.

Aus verschiedenen Quellen lässt sich folglich ableiten, dass Blaulicht dem Gesundheitszustand von Menschen zuträglich ist, nicht zuletzt, wenn humoristische oder karnevalistische Aspekte hinzukommen.

### Stand der Technik

Gegenstände mit Leuchtmitteln, die speziell für Lichtabgabe im Nahbereich des Mundes eines Menschen vorgesehen sind, lassen sich z. B. der EP 1 273 208 B1 (Inhaber: Mermaz, Christophe et al.; Erteilungstag: 14.01.2004) entnehmen, die einen leuchtenden Lutscher vorstellt. Als ein Ausführungsbeispiel ist vorgesehen, einem Lutscher eine gefärbte Zuckerhülle zu geben, welche transparent oder transluzent ein Leuchtmodul umgibt.

Aus Zahnarztpraxen und aus der DE 10 2018 119 423 A1 (Anmelderin: Schott AG; Offenlegungstag: 13.02.2020) sind Lichthärtegeräte für Zahnplombierungsmaterial (den so genannten "composites") bekannt, die Licht auf Zähne mit Kunststofffüllungen aussenden sollen, um eine Kunststofffüllung (bzw. ein "composite") in einem reparierten Zahn zu härten ("Lichtpolymerisationsgeräte"). Hierbei schlägt die DE 10 2018 119 423 A1 vor, blaues Licht mit der Wellenlänge von 405 nm zu verwenden, um z. B. im Bereich der Backenzähne eine Aushärtung flüssiger Materialien, z. B. von Klebern, durchzuführen.

Ein weiteres Gerät für die Photopolymerisation von Zahnreparaturmaterial wie einem aushärtbaren Harz wird in der Figur 2 der US 2017/035 539 A1 (Anmelder: Joseph F. Bringley; Veröffentlichungstag: 09. Februar 2017) vorgestellt. Eine lichtleitende Spitze ist gebogen ausgeführt, damit das Licht zielgerichteter auf einen Zahn mit einer organischen Kunststoffmatrix als Füllung zu ihrer Härtung aufgebracht werden kann.

Für eine orale Anwendung wird in der WO 2018/213 893 A1 (Anmelderin: Cosmoaesthetics Pty Ltd; Veröffentlichungstag: 29.11.2018) ein mit LEDs ausgestatteter Griff beschrieben, an den ein austauschbares Mundstück angesteckt werden kann, nachdem durch einen Schnell-Auslöse-Mechanismus das nicht mehr benötigte Mundstück von dem Griff getrennt worden ist.

Ein weiteres Gerät für eine Anwendung im Mundbereich einer Person wird in der US 2008/065 175 A1 (Erfinder: Russell J. Redmond et al.; Veröffentlichungstag: 13.03.2008) beschrieben, das eine UV-Lichtquelle für die Abgabe von UV-Strahlen hat.

Weitere stabartige Geräte, die entweder für den Einsatz in einem Mund eines Menschen bestimmt sind oder hierfür verwendet werden können oder auch in anderen Körperhöhlen zum Einsatz kommen können, sind der US 2015/030 989 A1 (Anmelder: Nikolaos S. Soukos et al.; Veröffentlichungstag: 29.01.2015), der US 2007/073 362 A1 (Erfinder: Noel Rodney Campbell; Veröffentlichungstag: 29.03.2007), der US 6 159 236 A (Anmelderin: Advanced Photodynamic Technologies, Inc.; Veröffentlichungstag: 12.12.2000), der US 2010/076 526 A1 (Erfinder: Yosef Krespi et al.; Veröffentlichungstag: 25.03.2010) und der US 2010/274 328 A1 (Erfinder: Robert Morgan; Veröffentlichungstag: 28.10.2010) zu entnehmen.

Alle diese Geräte scheinen für eine Nutzung durch fachkundiges Personal wie z. B. einen Zahnarzt gestaltet zu sein. Bei einigen Geräten soll ein Bediener das Gerät an dem Griff während seines Einsatzes in einer Körperhöhle eines Menschen, wie einem Mund der Person, fortwährend festhalten und so an die richtige Stelle, zum Beispiel in den Bereich einer zu schließenden Zahnkavität, führen.

Die zuvor genannten Druckschriften gelten mit ihrer Benennung als vollumfänglich in vorliegende Erfindungsbeschreibung inkorporiert. Hierdurch soll vermieden werden, nicht mehr erneut und wiederholt allgemein bekannte Zusammenhänge zwischen Licht ausgewählter Wellenlängen und den Möglichkeiten des Bestrahlens einer Körperöffnung oder einer Körperhöhle wie die Bestrahlung eines Mundes zu erörtern, sondern durch Verweis auf die Druckschriften als ebenfalls definiert für vorliegende Erfindung ansehen zu dürfen.

### Aufgabenstellung

Viele Menschen, die Sorge vor Infektionskrankheiten wie einem grippalen Infekt haben, nutzen häufig gerne medizinische Tüchlein oder Mundschutze, die sie sich vor den Mund binden, weil sie glauben, diese Maßnahme stelle eine wirksame Barriere für Viren und Bakterien aus der Umwelt dar und durch das Tüchlein sei ein wirksamer Schutz für ihre Atemwege sichergestellt. In der Fachliteratur gibt es zahlreiche Stimmen, die die medizinische Wirkung in Abrede stellen und behaupten, solche Mundschutze stellten nur ein psychologisches Massenphänomen dar.

Ähnlich verhält es sich mit den übrigen Körperhöhlen und Körperöffnungen bei Lebewesen wie Menschen. Z. B. wird in medizinischen Lehrbüchern zu Hals-Nasen-Ohrenkrankheiten die Auffassung vertreten, dass eine Mittelohrendzündung durch Pneumokokken (Streptococcus pneumoniae), Haemophilus influenzae, Streptococcus pyogenes, Staphylococcus aureus oder Moraxella catarrhalis hervorgerufen wird. Eine Chlamydieninfektion oder Gonorrhö und auch Genitalherpes sowie Kandidose sollen für unangenehme Genitalerkrankungen verantwortlich sein. In alle diesen Fällen wird in der Regel durch Antibiotika, antiviralen Medikamenten oder durch oral einzunehmende Antimykotika versucht, dem Viren-, Bakterien- oder Pilzbefall Herr zu werden.

Tatsächlich wäre es aber wünschenswert, bio-physikalische, mechanische oder chemischpharmazeutische Barrieren und Behandlungsmaßnahmen zu kennen, wie Infekte, Ansteckungen und vor allem über Atemwege oder sonst durch Körperkontakt übertragene Krankheiten in ihren Wirkungen und Schädigungen verringert werden können. Mit anderen Worten, ein geeigneter Ersatz für solche, wenigstens teilweise heftig kritisierten (angeblichen) Schutzmaßnahmen wie Mundschutz oder Abstinenz sollte durch effektivere Maßnahmen und Geräte ermöglicht werden können.

In diesem Zusammenhang wäre es besonders günstig, einen Entwurf für ein Gerät zu kennen, der aufgrund seiner Gestaltung besonders leicht zu bedienen ist, idealerweise auch von weniger geübten Personen.

### Erfindungsbeschreibung

Die erfindungsgemäße Aufgabe wird durch ein Bestrahlungssteckstück nach Anspruch 1 gelöst; ein geeignetes Verfahren, durch das das Bestrahlungssteckstück durch einen Nutzer als mobiles Desinfektionsgerät anpassbar ist, lässt sich Anspruch 18 entnehmen. Vorteilhafte Weiterbildungen lassen sich den abhängigen Ansprüchen entnehmen.

Ein Bestrahlungssteckstück ist ein Gegenstand, der zum einen dazu vorgesehen ist, in eine Körperöffnung einer Person oder in die Körperöffnung, wie z. B. in das Maul (bzw. Mund), eines Tieres gesteckt zu werden, wobei es erwünscht ist, dass das Bestrahlungssteckstück nicht in Gänze im das Bestrahlungssteckstück aufnehmenden Menschen oder Tier verschwindet. Idealerweise ist zum anderen das Bestrahlungsteckstück aber auch so gestaltet, dass es als mobiles Desinfektionsgerät für Oberflächen, wie z. B. von Sitzflächen im öffentlichen Nah- und Fernverkehr, benutzt werden kann.

Für den Teil, der in der Körperöffnung, z. B. in einem Mund, z. B. in einem Nasenloch (Nares), z. B. in einer Ohrmuschel (Auricula auris), z. B. in einem After, z. B. in einer Vagina, verschwinden soll, hat das Bestrahlungssteckstück ein Einführteil. Das Bestrahlungssteckstück ist besonders geformt. Hierdurch ist es möglich, dass das Einführteil eine Anlagerung, insbesondere großflächige Anlagerung, in der Körperhöhle herstellen kann, genauer ausgeführt, an einer Innenwand der Körperhöhle, z. B. am Gehörgang, z. B. an einer Zunge, z. B. an einem Gaumen oder z. B. an einer Innenseite von Nüstern eines Pferdes. Darüber hinaus ist es für manche Einsatzbereiche vorteilhaft, wenn das Einführteil in einem weiteren Bereich seines Körpers so gestaltet ist, dass eine zweite, insbesondere eine zur ersten Aufnahme, gegengleiche Aufnahme des Einführteils, insbesondere durch einen anderen Bereich des Einführteils, realisierbar ist. Mit anderen Worten, das Einführteil soll zwischen einer ersten Zellwand des Lebewesens (z. B. einer Zunge) und einer zweiten Zellwand des Lebewesens (z. B. Gaumen), ggf. hinter anderen Körperteilen wie Zähnen oder einem äußeren Schließmuskel, aufgenommen werden. Es kann auch gesagt werden, dass das Einführteil aufgrund seiner Länge und seiner Breite auf die Körperhöhle, in der das Einführteil einzuführen sein wird bzw. ist, abgestimmt ist (z. B. für ein Einführen in eine Mundhöhle eine Länge in einem Bereich von 5 cm bis zu 15 cm, z. B. für ein Einführen in eine Nase eine maximale Breite von 1 cm und eine Länge von weniger als 5 cm (Nase einer europäischen Frau) oder 6 cm (Nase eines europäischen Mannes)).

Als Gegengewicht zum Einführteil kann ein Verwahrkasten einen Akkumulator und/oder sonstige Teile des Bestrahlungssteckstücks beinhalten. Das längliche Einführteil erfährt seinen Momentenausgleich bzw. sein Austarieren durch einen gewichtsmäßig konzentrierteren (durch einen mit höherer Dichte ausgestatteten) Verwahrkasten. Ein Gewichtsausgleich durch einen kompakten, das Gewicht des Einführteils ausgleichenden Verwahrkasten steigert einen Tragekomfort.

Neben dem Einführteil hat das Bestrahlungssteckstück eine Anlagefläche, die auch als Schild zu bezeichnen ist. Die Anlagefläche erstreckt sich in eine andere Richtung als das Einführteil sich erstreckt. Die Anlagefläche erstreckt sich quer zu einer Längserstreckung des Bestrahlungssteckstücks. Vorteilhaft ist es, wenn die Anlagefläche zur abdeckenden, abdichtenden bzw. verschließenden Aufnahme (bzw. Auflage) an dem Rand der Körperhöhle (Körperhöhlenkranz wie z. B. Lippen) anliegen kann (z. B. durch eine Oberflächenführung wie geschwungen, gebogen und/oder zurückgezogen).

In einer vorteilhaften Ausgestaltung ist die Anlagefläche als Anschlag, Bremse bzw. Stoppelement ausgestaltet, damit ein Teil des Bestrahlungssteckstücks außerhalb der Körperhöhle verbleibt. Zeigt die Anlagefläche eine Wölbung bzw. einen Bogen (z. B. einen Kreisbogenausschnitt), so kann eine Anlagefläche besonders vorteilhaft einen gebogenen Rand bzw. einen gewölbten Rand einer Körperöffnung eines Menschen, insbesondere vollflächig, abdecken und zugleich den Eintritt von Fremdkörpern und Fremdpartikeln, wie Viren oder Bakterien, reduzieren oder sogar verhindern.

Das Bestrahlungssteckstück insgesamt bzw. globaler betrachtet kann eine beliebige Formgebung haben, z. B. abschnittsweise oval oder sphärisch geformt sein. Das Bestrahlungssteckstück hat aber insgesamt eine längliche Erstreckung. Die längliche Erstreckung kann mit einer Längserstreckung angegeben werden. Hierzu ist die Anlagefläche im (nahezu) 90-Grad-Winkel angeordnet. In diesem Fall verläuft die Anlagefläche quer zur Längserstreckung. Die Längserstreckung ist auf eine typische Körperhöhlenlänge (z. B. Mundraumtiefe, z. B. Gehörgangtiefe, z. B. Nasenlänge) abgestimmt.

Die Anlagefläche ist zum Aufliegen auf einem Teil des sich an die Körperöffnung anschließenden Körpers, z. B. zum Aufliegen auf dem Gesicht, insbesondere auf dem Amorbogen (bzw. der Schnauze, z. B. eines Hundes), gestaltet. Ist das Bestrahlungssteckstück ein für einen Mund vorgesehenes Mundsteckstück, so liegt die Anlagefläche beim Menschen zumindest auf dem Amorbogen an. Typischerweise wird die Anlagefläche jedoch auf Teilbereichen, z. B. bei einem Mund sowohl auf der Oberlippe als auch auf der Unterlippe, anliegen. Ist das Bestrahlungssteckstück als Analsteckstück zu verwenden, liegt die Anlagefläche wenigstens zum Teil auf dem Perineum, ein anderer Teil der Anlagefläche ist für die Anlage an den Gesäßbacken bestimmt. Hierdurch wird die Auflage bzw. Anlagefläche verteilt, wenn das Bestrahlungssteckstück in der Körperhöhle eingesetzt ist.

Das Einführteil hat eine Hülle bzw. eine Komponente, die zumindest in einem Bereich transluzent ausgestaltet ist. Der transluzente Körper bzw. die transluzente Hülle hat einen Abschnitt, durch den Licht durchtreten kann. Natürlich kann der gesamte Körper bzw. die gesamte Hülle als transluzenter Körper realisiert sein. Besonders vorteilhaft ist es, wenn zumindest der vordere Bereich des stabartigen Bestrahlungssteckstücks transluzent ist, z. B. aus einem transluzenten Silikon hergestellt ist.

Im Inneren des Bestrahlungssteckstücks ist eine Lichtquelle angeordnet. Die Lichtquelle ist für die Abgabe von Licht bestimmter Wellenlängen bzw. für Wellenlängenbereiche konzipiert. Die Wirkung lässt sich noch steigern, wenn neben dem Licht auch noch eine Abgabe von UV-Strahlung und/oder einem Laserstrahl vorgesehen ist, z. B. durch eine Anordnung von LEDs, die Licht abgeben können, und der zusätzlichen Anordnung von wenigstens einer LED, die eine UV-Strahlung oder einen Laser-Strahl abgeben kann. Das Licht, das aus der Lichtquelle im Einführteil austreten kann, ist vorzugsweise ein gerichtetes Licht, das vorzugsweise in dem posterioren Körperhöhlenbereich eine Beleuchtung sicherstellen kann.

Das von der Lichtquelle abgegebene Licht liegt vorteilhafterweise in einem Wellenlängenbereich, in dem krankheitsverursachende Fremdkörper wie Bakterien, Viren und/oder Pilze geschädigt, abgetötet oder zumindest in ihrer Keimzahl verringert werden können (hierbei wird der Begriff "Keime" im medizinischen Sinne als Oberbegriff für Viren, Bakterien und Pilze sowie für ähnliche Parasiten, Fremdkörper und Schädiger von Lebewesen wie Menschen benutzt). Günstige Wellenlängen liegen z. B. im Blaulichtbereich (mittlerer und oberer 400 nm-Bereich), im Violettlichtbereich (unterer 400 nm-Bereich) und/oder im Ultraviolettlichtbereich (unterhalb von 400 nm, in einem Bereich von bis zu 100 nm). Hierbei sind vorzugsweise solche Wellenlängenbereiche ausgenommen, durch die eine unerwünschte Strahlungsbelastung für körpereigenes Gewebe entsteht, die also als ungünstig angesehen werden, wie eine Wellenlänge von 248 nm. In der medizinischen Fachliteratur sind Meinungen zu finden, dass eine längere Belastung durch Wellen im Wellenlängenbereich um 248 nm zu Krebs bzw. unkontrollierter Zellteilung führen könne. Solche Wellenlängen und Wellenlängenbereiche werden in dem Bestrahlungssteckstück idealerweise nicht erzeugt. Außerdem wird die Lichtquelle unterhalb einer maximalen Energieumsetzung betrieben, um die frequenzabhängigen Leuchtdichtengrenzwerte einzuhalten.

Mit einem solchen Bestrahlungssteckstück ist es möglich, die Keimzahl in einer Körperhöhle, wie z. B. in einem Bereich des Munds oder auch im Rachenbereich, eines das Bestrahlungssteckstück aufnehmenden Lebewesens, z. B. eines Menschen, zu reduzieren. Hierzu wird das Bestrahlungssteckstück in die Körperhöhle des Lebewesens eingeführt. Anschließend wird das Bestrahlungssteckstück eingeschaltet, sodass es Licht, insbesondere über seinen transluzenten Bereich des Einführteils, abgeben kann.

Mit anderen Worten, das Bestrahlungssteckstück kann als keimtötendes Werkzeug, insbesondere in einer Körperhöhle eines Nutzers, verwendet werden. Ein solches Gerät kann als Desinfektionsgerät bezeichnet werden. Mit einem solchen Gerät können aber auch Keime an sonstigen Oberflächen, z. B. auf Sitzflächen in Straßenbahnen, Zügen und Bussen, reduziert werden. Es handelt sich somit um ein mobiles Desinfektionsgerät, das von einer Person mitgeführt den unmittelbaren Bereich der Person (z. B. einen Fahrgastsitz) entkeimen kann (im Sinne eines Nahbereichsentkeimungsgeräts).

Aus einem vorderen Bereich des Bestrahlungssteckstücks, d. h. aus einem Bereich des Einführteils, kann das Licht austreten. Es kann auch davon gesprochen werden, dass ein gerichtetes Licht aus einem Spitzenbereich des Einführteils austritt. Das Licht ist idealerweise so gerichtet, dass es noch tiefer in die das Einführteil aufnehmende Mundhöhle eindringen kann ("posteriore Beleuchtungsbereich").

Das Licht soll auf den hinteren Bereich einstrahlen. Bei einer Anwendung in einem Mund sollte also der Rachenbereich, insbesondere sollte auf einen Mundschleimhautbereich und vorzugsweise auf einen Zungenbereich der Mundhöhle eingestrahlt werden. Es ist besonders vorteilhaft, wenn das Licht gerichtet in den Posteriori-Bereich der Mundhöhle strahlt. Bei einer rektalen Anwendung eines hierfür vorgesehenen Bestrahlungssteckstücks im After sollte Licht auch zumindest bis zum Mastdarmausgang gelangen.

Das Bestrahlungssteckstück ist dazu bestimmt, in unterschiedlichen Verwendungsweisen genutzt zu werden. Für verschiedene Verwendungsarten und Anwendungsfälle kann das zuvor vorgestellte Bestrahlungssteckstück genutzt werden, dessen Einführteil idealerweise auf den Anwendungsfall angepasst ist (z. B. mit einem Bissbereich, z. B. mit einem Einklemmbereich, z. B. mit einem Schließmuskelbereich, z. B. mit einem Muskelanpassbereich).

Durch ein Einschalten einer Lichtquelle, z. B. mehrere LEDs umfassend, im Inneren des Einführteils, das in einer Körperhöhle einer Person steckt, kann zunächst einmal Licht im Inneren der Körperhöhle einer Person erzeugt werden.

Die Lichtquelle schafft ein gerichtetes Licht, das von dem Einführteil abstrahlt und somit in einer Richtung weg von dem Einführteil leuchtet. Durch eine anschließende Nutzung der Reflexion bzw. der Streuung zumindest eines Teils des Lichts in Richtung auf den Körperöffnungsbereich, insbesondere an einer Zellwand im posterioren Bereich der Körperhöhle der Person, kann das Licht auf den Rand bzw. die Begrenzung der Körperöffnung der Person scheinen und hierdurch eine Verfärbung hervorrufen. Eine Reflektion von Licht kann z. B. an Zähnen oder Zahnkronen erfolgen. Das Licht, das vorzugsweise reflektiertes Licht ist, tritt aus der Körperhöhle über die Ränder der Körperöffnung aus. Mithilfe von aufgetragenen fluoreszierenden Hautcremes lassen sich in Verbindung mit blauem Licht oder UV-Licht z. B. Lichteffekte auf der Haut generieren.

Das Bestrahlungssteckstück ist als mobiles Desinfektionsgerät gestaltet, bei dem mittels Licht Keime in der Körperhöhle, wie z. B. in einem Mund, insbesondere im Rachen, eines Lebewesens, wie im Rachen eines Menschen, oder wie in einer Nase eines Menschen, abgetötet werden können. Die Bestrahlung erfolgt bis in eine Tiefe der Körperhöhle hinein, die größer ist als eine Erstreckung des Bestrahlungssteckstücks. Wird mit einer Wellenlänge gearbeitet, die an Körperteilen wie Zähnen, Nasenhaaren oder Schamlippen reflektiert werden kann, so kann die Wirkung des ausgestrahlten Lichts gesteigert werden.

Nachfolgend werden vorteilhafte Ausgestaltungen und Weiterbildungen dargelegt, die für sich gesehen, sowohl einzeln als auch in Kombination, ebenfalls erfinderische Aspekte offenbaren können.

Das Bestrahlungssteckstück kann schnullerartig ausgestaltet sein, insbesondere in dem Fall, in dem es als Mundsteckstück genutzt werden soll. Man könnte also auch sagen, dass das erfindungsgemäße Mundsteckstück eine schnullerartige Vorrichtung mit Beleuchtung für den Innenraum des Mundes ist.

In einer weiteren alternativen Ausführungsform ist das Bestrahlungssteckstück zapfenartig ausgestaltet. Auch kann das schnullerartige Bestrahlungssteckstück zapfenartig gestaltet sein. Es kann auch gesagt werden, dass das Bestrahlungssteckstück einen stab- oder stilartigen Teil hat, der als Einführteil gedacht ist.

Vorteilhafterweise hat das Bestrahlungssteckstück auch eine Energiequelle oder wird von einer ihm zur Verfügung stehenden Energiequelle versorgt. Das Bestrahlungssteckstück ist im Betriebszustand mit einer Energiequelle verbunden. Eine Energiequelle kann Teil des Bestrahlungssteckstücks sein oder zumindest für das Bestrahlungssteckstück zur Verfügung stehen.

Die Lichtquelle ist idealerweise eine Niedervoltlichtquelle, um z. B. das Risiko für einen Nutzer des Bestrahlungssteckstücks zu reduzieren. Die Energiequelle ist idealerweise für eine Niedervoltversorgung ausgelegt.

Die Lichtquelle kann aus einer oder mehreren LEDs geschaffen bzw. hergestellt sein. Mehrere LEDs können zusammen die Lichtquelle bieten oder bilden. Als LEDs eignen sich Rot-, Grün-, Blau-LEDs (RGB-LEDs) bzw. Rotlicht-LEDs, Grünlicht-LEDs und/oder Blaulicht-LEDs. Auch können dezidierte Blaulicht-LEDs Teil der Lichtquelle sein oder die Lichtquelle bilden. Weitere LEDs, die geeignet sind, Teil der Lichtquellen darzustellen oder die Lichtquelle zu bilden, sind UV-Licht-LEDs, z. B. UV-Licht-LEDs eines ganz bestimmten UV-Lichtbandes wie z. B. UV-C. Eine oder mehrere weitere LEDs können Laser-LEDs sein.

Die Energiequelle kann in einer Ausgestaltung auf einem Spannungsniveau Strom abgeben, der der Versorgungsspannung der Lichtquellen, insbesondere der LEDs, entspricht. Liefert die Energiequelle eine höhere Spannung, so kann mit Hilfe einer elektronischen Schaltung (Tiefsetzsteller, Z-Dioden-Schaltung, Zerhacker mit Transformator oder durch ähnliche Schaltungen) die Spannung auf das Versorgungsspannungsniveau der Lichtquelle reduziert werden.

Zur zeitlichen Steuerung des Betriebs des Bestrahlungssteckstücks sollte ein vorteilhaft gestaltetes Bestrahlungssteckstück eine Zeitsteuerung umfassen. Durch die Zeitsteuerung kann die Beleuchtungsdauer zeitlich begrenzt werden. Nur während der Beleuchtungsdauer kann die Lichtquelle ein Licht abgeben. Das Bestrahlungssteckstück ist zeitlich in seinem Betrieb begrenzt. Ein übermäßiger Lichtkonsum wird so vermieden.

Es ist vorteilhaft, wenn die Dauer der Abgabe von entsprechendem Licht zeitlich beschränkt wird. Eine Zeitsteuerung, durch die die Dauer der Beleuchtung beschränkt wird, trägt zur Schädigungsfreiheit durch das Bestrahlungssteckstück bei.

Das Bestrahlungssteckstück sollte vorteilhafterweise ein Batteriefach aufweisen. Dieses Batteriefach sollte aber in einem Abschnitt des Bestrahlungssteckstücks angeordnet sein, der nicht für die Aufnahme in der Körperhöhle, z. B. dem Mund, eines Menschen gedacht ist. Das Batteriefach sollte außerhalb eines menschlichen Körpers verbleiben. Der anzusiedelnde Bereich sollte außerhalb des menschlichen Körpers liegen. In das Batteriefach kann - idealerweise - nach Gusto des Benutzers entweder ein Akkumulator oder eine Batterie eingebaut werden, sofern beide die gleiche Niedervoltversorgungsspannung liefern können.

Die Lichtquelle, die Licht abgeben soll, sollte in einem Wellenlängenbereich zwischen 400 nm und 490 nm liegen. Eine geeignete Wellenlänge ist z. B. bei ungefähr 405 nm. Es hat sich gezeigt, dass eine Wellenlänge um 453 nm positive Effekte hervorrufen kann. Auch eine Wellenlänge im Bereich um 470 nm kann positive Wirkung entfalten.

Ein geeigneter Wellenlängenbereich für UV-Licht ist ein Wellenlängenbereich, der im Bereich um 222 nm liegt.

Wird das Licht aus der Lichtquelle gerichtet durch das Einführteil ausgestrahlt, kann die Wirkung des Bestrahlungssteckstücks gesteigert werden, wobei zugleich der Energieverbrauch niedrig gehalten werden kann. Vorzugsweise ist der Spitzenbereich des Einführteils für eine Abgabe des Lichtes gestaltet. Der Spitzenbereich kann in einer Ausgestaltung mit vier LEDs ausgestattet sein. Die vier LEDs können mit je zwei unmittelbar nebeneinander angeordneten LEDs die Spitze bedecken. Die vier LEDs können in zwei Reihen zu je zwei LEDs angeordnet sein. Hierbei ist es möglich, dass von den vier LEDs zwei zu der einen Art LEDs gehören (z. B. Blaulicht-LEDs) und zwei zu der anderen Art LEDs gehören (z. B. Rotlicht-LEDs oder Laser-LEDs).

Ist ein Teil des Bestrahlungssteckstücks knopfartig gestaltet, z. B. durch einen knopfartigen Korpus, der vorteilhafterweise als Griff fungiert, so kann der knopfartige Korpus jenseits der Anlagefläche positioniert sein. Der knopfartige Korpus kann als Elektronikgehäuse ausgestaltet sein. Der knopfartige Korpus kann flach sein, wodurch die Erstreckung jenseits der Körperhöhle möglichst kurz ist. Die Anlagefläche trennt das Einführteil von dem knopfartigen Korpus. Der knopfartige Korpus ist als Außengehäuse gestaltet, weil der Korpus dafür vorgesehen ist, außerhalb des menschlichen Körpers zu bleiben, während das Einführteil in die Körperhöhle eingebracht ist. Der knopfartige Korpus kann als elektrischer Versorgungskörper ausgestaltet sein, z. B. über elektrische Anschlüsse. Somit ist der Korpus eine Elektronikbox, insbesondere mit einem Akkumulator und kann in diesem Fall auch als Akkukasten bezeichnet werden. Als elektrische Anschlüsse können speziell vorgesehene Kabel, aber auch Stecker, an die ein Gegenstecker mit einem entsprechenden Kabel angeschlossen werden kann, vorgesehen sein. Auch kann innerhalb des knopfartigen Korpus, wie bereits angeklungen, ein Batteriefach existieren, in das eine Batterie (oder auch ein Akkumulator) als Energiequelle für den Betrieb der Lichtquelle eingebaut werden kann.

Vorteilhaft ist es, wenn das Bestrahlungssteckstück bzw. Teile desselben, z. B. das Einführteil, nachgiebig ausgestaltet ist. Auch ist es vorteilhaft, wenn das Bestrahlungssteckstück flüssigkeitsdicht ausgestaltet ist. Das Bestrahlungssteckstück kann, insbesondere aufgrund geeigneter Materialwahlen, sowohl nachgiebig als auch flüssigkeitsdicht ausgestaltet sein. In einigen Gestaltungen mag es ausreichen, wenn das Bestrahlungssteckstück, insbesondere dessen Einführteil, nur nachgiebig ist. In anderen Ausgestaltungen mag es ausreichen, wenn das Bestrahlungssteckstück, insbesondere dessen Einführteil, flüssigkeitsdicht ausgestaltet ist. Als Materialien für die Schaffung des Bestrahlungssteckstücks, insbesondere für dessen flüssigkeitsdichten und/oder nachgiebigen Teile wie dem Einführteil, bieten sich Silikone oder Latex an. Das Bestrahlungssteckstück, zumindest aber sein Einführteil kann entweder aus einer Kunststoffhülle hergestellt sein oder aus einer Latex-Hülle.

Das Bestrahlungssteckstück kann von außen betrachtet wie ein zwei Halbschalen umfassender größerer Körper mit einem dahinter angeordneten, kleineren Körper ausgestaltet sein. Der oval gestaltete Querkörper und das Einführteil sind idealerweise symmetrisch gestaltet. In dem Fall, dass das Bestrahlungssteckstück als Mundsteckstück ausgestaltet ist, ist vorzugsweise eine Langachse des Ovals entlang einer Bissebene ausgerichtet. Bei der Ausgestaltung als Mundsteckstück ist es möglich, solche Raumgestaltungen zu wählen, dass sowohl eine dentogingivale als auch eine linguale Anlagerung des Mundhöhlenkörpers im Mund des das Mundsteckstück nutzenden Menschen gegeben ist. Im Falle einer Realisierung des Bestrahlungssteckstücks als Analstab, ist eine Formgebung in einem Bereich des Einführteils zur Anlagerung des Schließmuskels an das Einführteil besonders vorteilhaft. Im Falle einer Realisierung des Bestrahlungssteckstücks als Ohrsteckstück ist das Einführteil idealerweise auf die Formgebung eines Gehörgangs und an die Formgebung des Mittelohrs mit einem Trommelfell angepasst. Ein Körper, der in Bezug auf seine Ober- und Unterschale symmetrisch gestaltet ist, erleichtert die geeignete Platzierung in einer Körperhöhle wie z. B. im Mund, wie im After, wie in der Nase, wie in der Vagina oder wie im Ohr. Auch lässt sich so ein Gegenstand leichter herstellen.

Ist das Bestrahlungssteckstück nachgiebig gestaltet, so kann es so nachgiebig gestaltet sein, dass es formadaptiv in einem Abschnitt der Körperhöhle, z. B. auf dem Gaumen und/oder der Zunge eines Mundes anliegen kann. Eine formadaptive Oberfläche folgt den Druckverhältnissen in der Körperhöhle, z. B. einem Druck durch eine Zunge oder einem Unterdruck durch Atemvorgänge. Die formadaptive Oberfläche ist zur Nachbildung der Körperhöhle eines Nutzers sinnvoll, so können z. B. bei einem Analstab die rektalen Körperformen nachgeformt sein, insbesondere die Verengung durch den äußeren und ggf. auch durch den inneren Schließmuskel.

Die Wirkung, dass durch das Bestrahlungssteckstück Keime reduziert werden, kann dadurch gesteigert werden, dass das Einführteil, z. B. der Mundhöhlenkörper, verschiebbar ausgestaltet ist. Das Einführteil kann in einer vorteilhaften Ausgestaltung gegenüber der Anlagefläche bzw. der Auflage räumlich bewegt werden. Das Einführteil kann z. B. ausziehbar gestaltet sein. Ist das Einführteil verlängerbar, so kann das Licht aus dem Einführteil möglichst gut an die zu bestrahlende Stelle, wie z. B. den Rachenraum eines Menschen, gelangen. Bekanntermaßen sind die diversen Körperhöhlen bei verschiedenen Menschen unterschiedlich groß - Kinder haben z. B. eine sehr viel kleinere Mundhöhle als Erwachsene. Hierfür können Bestrahlungssteckstücke unterschiedlicher Größe bereitgestellt werden oder es wird ein Universal-Einführteil bereitgestellt, bei dem das Einführteil in seiner Größe, insbesondere Längserstreckung, veränderbar ist. Es ist also die Länge des Einführteils durch eine verschiebliche Anordnung in dem oder an dem Einführteil veränderbar.

Sollte die Länge des Bestrahlungssteckstücks nicht passen, z. B. weil eine Körperhöhle, wie der After oder der Mund eines Nutzers des Bestrahlungssteckstücks, kürzer ist als das Einführteil lang ist, so kann durch wenige Handgriffe aus einem ersten Bestrahlungssteckstück ein zweites, passenderes Bestrahlungssteckstück werden. Hierzu kann der Mundhöhlenkörper von dem Mundsteckstück getrennt werden. Eine besonders einfache Methode des Trennens ist dadurch geschaffen, dass durch ein Herausbiegen des Einführteils bzw. seines Endes aus Befestigungsnasen die beiden markantesten bzw. größten Teile des Bestrahlungssteckstücks zu trennen sind (sogenannte Klippnasen). Die Befestigungsnasen können im Randbereich eines blockartigen Anschlusskörpers des Bestrahlungssteckstücks vorhanden sein. Ein Ende des Einführteils kann als flache Platte ausgestaltet sein. Die flache Platte bildet eine Seite des hinteren Körpers des Bestrahlungssteckstücks. Die Platte wird durch die Befestigungsnasen gehalten.

Ist das erste Bestrahlungssteckstück so lang, dass es als unangenehm von einem Menschen empfunden wird, z. B. in der Verwendung als Mundsteckstück einen Würgereiz bei einem Menschen hervorruft, so kann durch Austausch des Einführteils, z. B. des Mundhöhlenkörpers, mit einem kurzen stabartigen Licht- bzw. Strahlenspender ein Einführteil geschaffen werden, das besser auf die Anatomie der jeweiligen Körperhöhle, wie z. B. auf die Anatomie des Mundraums, des Nutzers angepasst ist.

Sind die Klippnasen genau entgegengesetzt zueinander orientiert, so kann durch zwei Bewegungen, idealerweise zeitgleich auszuführende Bewegungen, ein Block an dem Einführteil so in seiner Breite oder Höhe verändert werden, dass er sich von dem Körper bzw. dem Außengehäuse, der bzw. das außerhalb des Mundes verbleiben soll, trennt. Solche Bewegungen können als verengende bzw. zusammendrückende Bewegungen oder als aufspreizende Bewegungen vorgesehen sein.

Die vorteilhaften Weiterbildungen können auch wie folgt in der Ausgestaltung als Mundsteckstück vorgestellt werden.

Der Mundhöhlenkörper hat vorteilhafterweise eine längliche Form. In einer vorteilhaften Weiterbildung erinnert der Mundhöhlenkörper an einen Schnuller. Wird der Mundhöhlenkörper schlanker gestaltet, so ist er eher durch den Begriff "stabartig" zu beschreiben. Auch eine Zapfenform ist eine geeignete Form für den Mundhöhlenkörper. Ist der Mundhöhlenkörper nicht nur gerade gestaltet, sondern leicht geschwungen bzw. gebogen ausgestaltet, so kann der vordere Teil des Mundsteckstücks der Gaumenwölbung und/oder der Zungenwölbung nachgeführt sein, allgemeiner ausgedrückt, der Mundwölbung, hervorgerufen durch Gaumen und/oder Zunge, nachempfunden sein. In einer solchen Gestaltung kann das eine Ende des Mundhöhlenkörpers niedriger liegen als ein mittlerer Bereich des Mundhöhlenkörpers. Das andere Ende des Mundhöhlenkörpers kann ebenfalls niedriger liegen. Beide Enden des Mundhöhlenkörpers können auf ungefähr der gleichen Höhe angesiedelt sein. In einem mittleren Bereich des, insbesondere stabartigen, Mundhöhlenkörpers ist dieser, z. B. im Vergleich zu den Rand- oder Endbereichen des Mundhöhlenkörpers, angehoben. Werden LED-Stripes als Lichtquellen verwendet, so kann der Mundhöhlenkörper sehr schmal, z. B. weniger als 5 cm in seiner Breite, gestaltet sein. Werden Stripes verwendet, die aus LEDs mit einer Breite von 3 mm aufgebaut sind, so kann der stab- oder zapfenähnliche Mundhöhlenkörper so dünn sein, dass er gerade einmal 5 mm beträgt. Je schmaler der Mundhöhlenkörper ausgeführt ist, desto geringer fällt seine hindernde oder beeinflussende Eigenschaft in das Gewicht.

In dem Fall, dass das Bestrahlungssteckstück für eine Anwendung als Analstab oder Vaginastab ausgestaltet ist, ist das Einführteil, insbesondere im Vergleich mit einem Mundsteckstück, vorteilhafterweise breiter ausgelegt. Der zusätzliche Raumgewinn bzw. das zusätzliche Volumen kann durch Luftführungskanäle genutzt werden, über die Luft in oder aus der entsprechenden Körperhöhle ein- oder ausgeleitet werden kann. Mithilfe einer Luftförderung (leichter Überdruck oder leichter Unterdruck, z. B. in einem Bereich zwischen 10 mbar und 50 mbar) kann die Benutzung des Anal- oder des Vaginastabs angenehmer werden.

Idealerweise hat das Bestrahlungssteckstück eine eigene Energiequelle, z. B. in sich integriert bzw. eingebaut. Die Energiequelle ist, wenn es sich um einen Akkumulator handelt, nach einigen Betriebsstunden wieder aufzuladen. Hierzu bietet sich ein integrierter, insbesondere standardisierter Anschluss. Z. B. kann ein USB-Anschluss wie ein USB-C-Anschluss oder ein USB 3.0-Anschluss die Buchse für den Anschluss eines Kabels zur Stromversorgung und zum Aufladen des integrierten Akkumulators in den hinteren Teil des Bestrahlungssteckstücks eingearbeitet sein. In dem Bereich, der nicht für die Aufnahme in der Körperhöhle bestimmt ist (Elektronikbox, Außengehäuse, kastenartiger Korpus), kann eine Buchse für den Anschluss eines Kabels vorgesehen sein.

Eine Lichtquelle, die sich im Inneren des Einführteils befindet, kann sich aus einem Satz verschiedener LED-Typen zusammensetzen. Mehrere LEDs, insbesondere LEDs des Typs Stripe-LED, können eine Beleuchtungsgruppe bilden, die benachbart zu anderen LEDs angeordnet mit den weiteren LEDs eine Beleuchtungsgruppe bilden, z. B. mehrere Blaulicht-LEDs mit einer LASER-LED und mit einer UV-LED. So können Sätze von LEDs zusammengestellt werden, die sich aus LEDs wenigstens zwei verschiedener Typen zusammensetzen.

Mehrere, insbesondere typgleiche LEDs, z. B. Blaulicht-LEDs, können auf einem LED-Träger angeordnet sein. Werden die LEDs entlang des Einführteils für unterschiedliche Lichtausstrahlungen platziert, z. B. durch einen Betrieb mit verschiedenen Leistungen oder z. B. durch dichtere oder weiter entferntere Platzierungen der LEDs, können Bereiche mit helleren und Bereiche mit weniger helleren Ausleuchtungen erzeugt werden. Durch vorzugsweise unterschiedliche Lichtdichten in verschiedenen Abschnitten des Einführteils sind hellere und dunklere Bereiche herstellbar. So ist es möglich, von einer LED zu einer anderen LED mit untereinander abweichenden Versorgungsströmen zu operieren. Auch ist es möglich, in einem ersten Bereich LEDs dichter anzuordnen als in einem zweiten Bereich. In dem zweiten Bereich können die LEDs weiter beabstandet sein. In einem ersten Bereich sind die LEDs dichter platziert als in einem zweiten Bereich. Der zweite Bereich hat LEDs, die weiter beabstandet platziert sind als in dem ersten Bereich. Entlang des Einführteils können LEDs unterschiedlich dicht platziert sein. Wird in einem Rachenbereich oder in einem sonstigen posterioren Körperhöhlenbereich eine höhere Helligkeit benötigt, können die LEDs, die das Licht für jenen Bereich ausstrahlen sollen, mit einem stärkeren Versorgungsstrom versorgt werden.

Ist das Bestrahlungssteckstück mit einer Zeitsteuerung ausgestattet, so können die Betriebsphasen der Lichtquelle gesteuert werden. Ist ein Temperatursensor in dem Bestrahlungssteckstück, insbesondere im Einführteil integriert, so ist eine zusätzliche Sicherheitseinrichtung vorhanden. LEDs, die Licht oder eine elektromagnetische Welle abgeben sollen, können nur dann ausstrahlen, wenn sowohl die Zeitsteuerung es zulässt als auch der Temperatursensor keine erhöhte oder unzulässig hohe Temperatur registriert. Mit einer Zeitsteuerung für unterschiedliche Lichtquellen oder unterschiedliche Beleuchtungskörper, z. B. für einen LED-Stripe mit Blaulicht-LEDs und für eine LASER-LED, kann der Stripe über eine andere Dauer betrieben werden als die LASER-LED. Ähnliche Abweichungen können zwischen Blaulicht-LEDs und UV-(Licht-)LEDs eingestellt werden, z. B. kann die eine UV-LED oder es können die mehreren UV-LEDs nur kürzer betrieben werden als die Blaulicht-LEDs.

Eine LED, die eine Wellenfront oder eine Strahlung jenseits des sichtbaren Wellenspektrums aussendet, ist z. B. eine UV-Licht-LED. Eine Blaulicht-LED kann länger betrieben werden als eine UV-Licht-LED oder eine sonstige LED, die eine Wellenfront jenseits des sichtbaren Wellenspektrums aussendet, wenn Zellschäden möglichst unterbunden bleiben sollen.

Durch eine Temperaturüberwachung, z. B. mittels des oben beschriebenen Temperatursensors, kann sichergestellt werden, dass nur Licht bzw. eine elektromagnetische Welle von dem Bestrahlungssteckstück abgegeben wird, wenn das Bestrahlungssteckstück, insbesondere sein Einführteil, unterhalb einer Temperaturschwelle, wie z. B. 38 °C, liegt.

Ist in dem Bestrahlungssteckstück ein Akkumulator oder eine Batterie eingebaut, insbesondere auf der Steuerungsplatine (elektronische Steuerung) eingelötet, so kann das Bestrahlungssteckstück auch ohne Anschluss an eine Netzversorgung betrieben werden. Ein mechanisch fest verbundener Energiespeicher wie ein Akkumulator, z. B. durch eingelötete Lötfahnen des Akkumulators, kann zur besseren Gleichgewichtslage, insbesondere des Einführteils, in der Körperhöhle des Nutzers beitragen. Handelt es sich bei dem Bestrahlungssteckstück um ein Mundsteckstück, so kann ein austariertes Mundsteckstück mittig im Mund verlaufen. Druckstellen, insbesondere durch ein Ende des Einführteils, z. B. an einer Zunge oder an einem Gaumen, sind unwahrscheinlicher.

Der Teil des Bestrahlungssteckstücks, der außerhalb der Körperhöhle verbleiben soll, kann als Halteknopf, kastenförmiger Griff oder blockartiger Korpus ausgestaltet sein, um ein Gegengewicht zum Einführteil zu bilden und darüber hinaus eine Angriffsstelle zu bieten, durch die das Einführteil beliebig (von außen) in der Körperhöhle bewegt werden kann.

Vorteilhafterweise hat das Bestrahlungssteckstück an seinem seitlichen Bereich einen Einschaltknopf. Der Einschaltknopf kann mit einem Einschalttaster kooperieren. Der Einschalttaster kann z. B. im Inneren des Korpus platziert sein. Die Steuerung des Bestrahlungssteckstücks kann so gestaltet sein, dass der Einschalttaster nur zu ganz bestimmten Zeitpunkten oder nach ganz bestimmten Zeitdauern wieder betätigbar ist bzw. erneut Signale zur Verfügung stellen kann. Der Einschalttaster kann ein zeitlich verriegelnder Taster sein. Nur nach bestimmten Zeiten steht er wieder zu Verfügung. Nur wenn eine gewisse Zeit, wie z. B. 30 Minuten, abgelaufen bzw. vorbei sind, kann ein neuer Schaltimpuls ausgelöst werden.

Das Bestrahlungssteckstück hat in seinem Inneren vorteilhafterweise einen LED-Träger. Das Bestrahlungssteckstück weist in seinem solchen Fall einen LED-Träger auf, auf dem LEDs angeordnet sind. Die LEDs müssen nicht alle gleich ausgerichtet sein. Die LEDs können in unterschiedliche Richtungen weisen. So ist es möglich, zwei Richtungen für das Licht vorzusehen, sozusagen zumindest eine bidirektionale Ausleuchtung zu haben. Noch bevorzugter sind Einführteile, die mehr als zwei Belichtungsrichtungen abdecken können, also mit tridirektional, tetradirektional oder pentadirektional leuchtende Einführteile ausgestattet sind.

Das Bestrahlungssteckstück kann mit einem Einführteil ausgestattet sein, das einen Einklemmbereich hat, wie ein Bissbereich, ein Schließmuskelbereich oder ein Muskelanpassbereich. Dieser besondere Bereich, in dem z. B. der Stab verjüngt und/oder gebogen bzw. gewölbt ausgeführt sein kann, ist vorteilhafterweise näher bei einem Körperöffnungsschutz als bei einem (äußeren) Ende des Einführteils angesiedelt. Dieser Bereich kann dafür bestimmt sein, das Einführteil in der Körperöffnung besonders leicht und angenehm zu halten, z. B. durch ein Schließen des Mundes oder durch ein Anspannen des Schließmuskels. Der Einklemmbereich kann als eine Verstärkung und/oder eine Einschnürung der transluzenten Hülle ausgebildet sein.

Das Bestrahlungssteckstück kann mit einem Set Einführteile ausgestattet sein. Zu einem Bestrahlungssteckstück kann ein modulares Set mit mehreren Einführteilen gehören. In einem solchen Fall ist es möglich, die Einführteile des Sets gegeneinander auszuwechseln. Ein solches Set kann insbesondere so zusammengestellt sein, dass zu so einem Set mindestens ein Mundhöhleneinführteil, mindestens ein Analeinführteil und vorzugsweise mindestens ein Vaginaleinführteil oder mindestens ein Ohreinführteil und/oder mindestens ein Naseneinführteil gehören. Das erste Einführteil des Sets kann sich dadurch auszeichnen, dass es einen kleineren Einführdurchmesser aufweist als ein Einführdurchmesser eines zweiten Einführteils des Sets.

Die zuvor beschriebenen Ausgestaltungen des Bestrahlungssteckstücks können auch wie folgt zusammengefasst werden.

Ist das Bestrahlungssteckstück als Mundsteckstück zu verwenden, so kann es derart gestaltet sein, dass es wie ein Schnuller im vorderen Mundraum, z. B. an Zunge und Gaumen, anliegen kann. Der nicht für die Anlage vorgesehene Bereich des Mundsteckstücks, genauer des transluzenten Mundhöhlenkörpers, ist für die Abgabe von Licht wenigstens in einen posterioren Mundhöhlenbereich aus wenigstens einer Lichtquelle, die im Inneren des Mundhöhlenkörper angeordnet ist, vorgesehen.

Gem. einem vorteilhaften Verfahren lässt sich das Bestrahlungssteckstück mit einem solchen Einführteil herstellen bzw. konfigurieren, das eine abgestimmte Länge für eine ganz bestimmte Lage oder Position in einer Körperhöhle eines Nutzers hat. Das ausgewählte Einführteil ist aus einem Satz von Einführteilen auswählbar (z. B. einem ersten Anal-Einführteil, einem ersten Vagina-Einführteil, einem ersten Oral-Einführteil, einem zweiten Anal-Einführteil, einem zweiten Vagina-Einführteil, einem zweiten Oral-Einführteil usw.). Das Einführteil ist über einen Steckverbinder an eine Steuerplatine in einem blockartigen Korpus des Bestrahlungssteckstücks anzuschließen. Durch Verwendung eines solchen Bestrahlungssteckstücks ist es möglich, die Keimzahl, insbesondere in einem hinteren Körperhöhlenbereich wie z. B. in einem Rachenbereich eines Menschen, durch Lichtbestrahlung mit Licht keimtötend wirkender Wellenlängen zu reduzieren. So ist es möglich, ein tragbares Desinfektionsgerät an der Hand zu haben, das z. B. in Bussen und Bahnen, sozusagen mal eben, im Sinne eines Schnullers, Lutschers oder Sauggeräts eingeführt werden kann, insbesondere bei hoher Fluktuation oder engem Gedränge.

Weitere vorteilhafte Ausgestaltungen sollen nachfolgend vorgestellt werden.

Die beabsichtigte Keimzahlreduktion kann noch weiter verbessert werden, indem ein lichtaktivierbarer Inhibitor, Wirkstoffverstärker, Wirkverstärker oder Energieumsetzer in die Körperhöhle eingebracht wird, z. B. über das Bestrahlungssteckstück oder über eine Mundspülung. Werden Zusatzstoffe, wie z. B. ein Reaktant, zusätzlich in die Körperhöhle gegeben, so kann die Wirkung des Bestrahlungssteckstücks noch verbessert werden. Der Reaktant, der Inhibitor, der Wirkstoffverstärker bzw. der Wirkverstärker können auf die Wellenlänge des von dem Bestrahlungssteckstück ausgestrahlten Lichts bzw. der von dem Bestrahlungssteckstücks abgestrahlten elektromagnetischen Welle abgestimmt sein. Der lichtaktivierbare Inhibitor kann auch kurz als Lichtinhibitor bezeichnet werden. Der Lichtinhibitor reagiert idealerweise auf genau die Wellenlänge, die von dem Bestrahlungssteckstück ausgesendet wird. Beispielsweise kann der Inhibitor ein Hemmstoff sein, der Stoffwechselaktivitäten verlangsamt. Der Inhibitor kann auch ein Hemmstoff sein, der die Vermehrung von Bakterien oder Viren verlangsamt oder unterdrückt. Auch kann der Inhibitor so ausgewählt sein, dass er Moleküle oder eine Molekülmischung umfasst, die das Eindringen von Viren in Zellen unterdrückt. Ein Beispiel für einen Inhibitor ist ein Enzym. Ein Enzym, das durch blaues Licht aktivierbar ist, ist z. B. ein speziell gestaltetes Enzym des Typs Adenylatzyklase (Adenylylcyclasen). Ein anderes Beispiel für einen Inhibitor ist ein Ion, wie ein Silberion. Durch eine geeignete Lichtwellenlänge kann ein Inhibitor durch eine Freisetzung in der entsprechenden Körperhöhle, z. B. im Mundraum, z. B. im Speichel, aktivierbar sein, indem z. B. eine chemische Bindung an einen Inhibitorträger aufgebrochen wird, insbesondere photochemisch gebrochen wird. Ein weiteres Beispiel ist Silbernitrat, mit dem Wucherungen und Geschwüre im Rachenbereich und im hinteren Mundbereich (oder auch im After oder auch in der Vagina) behandelt werden können. Eine andere Möglichkeit der Aktivierung eines Inhibitors besteht in der photochemischen Abspaltung einer molekularen Schutzeinheit, z. B. eines Moleküls. Außerdem kann ein Inhibitor durch Aufnahme elektromagnetischer Energie in einen elektronisch angeregten Zustand verbracht werden, der ein Aktivierungszustand ist. Durch die Vorgabe einer Lichtintensität und einer Bestrahlungsdauer ist eine bereitgestellte Menge des reaktiv oder katalytisch wirkenden Inhibitors genau dosierbar.

Das Bestrahlungssteckstück weist vorzugsweise eine Steuereinheit auf, durch die eine Abgabe einer Lichtdosis, insbesondere einer auswählbaren Lichtwellenlänge, z. B. sensorisch, kontrollierbar ist.

Besonders vorteilhaft ist ein Mundsteckstück, ein Analsteckstück, ein Vaginalsteckstück, ein Nasalsteckstück bzw. ein Auralsteckstück, das den Inhibitor, Wirkstoffverstärker, Wirkverstärker oder Energieumsetzer dosiert in der entsprechenden Körperhöhle, wie im Mundraum, wie im After, wie in der Vagina, wie in der Nase oder wie im Ohr, abgeben kann, z. B. über einen Docht oder über eine Dosierkanüle.

Wie gesagt, bieten sich zur Erzeugung des Lichts unterschiedliche Wellenlängenbereiche an. Ein besonders bevorzugter Wellenlängenbereich liegt in einem Bereich zwischen 400 nm und 480 nm, wobei sich z. B. besonders ausgewählte Wellenlängen wie 405 nm, 453 nm oder 470 nm als vorteilhaft herausgestellt haben. Daneben ist es vorteilhaft, wenn ein UV-Licht-Wellenlängenbereich ebenfalls von dem Bestrahlungssteckstück abgedeckt werden kann. Ein solcher Bereich kann z. B. ein UV-C-Wellenlängenbereich sein, wie z. B. zwischen 100 nm und 280 nm. Hierbei versteht ein Fachmann, dass von den breit angegebenen Wellenlängenbereichen wiederum einzelne, engere Wellenlängenbereiche ausgeschlossen sein können, um insbesondere schädigende Wirkungen für menschliche Organismen auszuschließen.

Der Weg des Lichts kann zur Erzeugung eines besonderen visuellen Effekts genutzt werden, der z. B. zu besonderen Anlässen wie Karneval bzw. Fasching oder einer Feier eine positive, zumindest aber markante Erscheinung hervorrufen kann.

Mit dem Verfahren kann eine Verfärbung durchgeführt werden. Diese Verfärbung kann bedarfsabhängig, insbesondere nur in besonderen temporären Phasen, gewählt werden, z. B. durch ein kurz aufeinander folgendes Ein- und Ausschalten.

Besonders markant kann es in bestimmten Umständen sein, wenn eine Blaulichtverfärbung eines Mundbereichs oder eines Unterleibsbereichs einer Person hervorgerufen wird. So können z. B. die Lippen einer Person durch das Mundsteckstück nur vorrübergehend visuell wahrnehmbar umgefärbt werden (von kurzer zeitlicher Dauer).

Die Hülle des Mundhöhlenkörpers, sofern das Einführteil als Mundhöhlenkörper zu verwenden ist, kann mit Geschmacks- und/oder Aromastoffen, z. B. einem Erdbeergeschmack, veredelt sein. Dadurch lässt sich die Akzeptanz steigern. Wenn der Mundhöhlenkörper nicht mehr nur nach Silikon schmeckt, sondern nach einer angenehmen Geschmacksnote, wie Whiskey oder Trüffel, steigert es die Bereitschaft, das Mundsteckstück in den Mund einzuführen.

Der Mundhöhlenkörper kann mit einer zweigeteilten Spitze ausgestattet sein, sodass ein Ende des Mundhöhlenkörpers das Gaumenzäpfchen im Mund, die Uvula, seitlich passieren kann. Wenn eine zweigeteilte Spitze vorhanden ist, wird vorzugsweise auch ein im Spitzenbereich zweigeteilter LED-Träger benutzt, dessen erste und zweite LED-Träger-Spitze mit jeweils mindestens einer LED ausgestattet ist.

Das mobile Desinfektionsgerät ist so klein, dass es leicht in Handtaschen mitgeführt werden kann. Möchte sich eine Person auf einen Sitz im öffentlichen Personenverkehr niederlassen, kann die Person den Sitz vor der Benutzung durch ein Ableuchten desinfizieren. So können Keime auf Oberflächen abgetötet werden. Das Abtöten von Keimen kann sitzplatzindividuell vorgenommen werden. Das Gerät, das überwiegend für die Verwendung als z. B. Mundsteckstück, Nasensteckstück oder Ohrsteckstück gedacht ist, kann außerdem als Oberflächendesinfektionsgerät genutzt werden. So kann die Spitze, die üblicherweise auf einen Rachen eines Menschen ausgerichtet wird, entlang der Oberfläche eines Sitzes in einem Zug, einer Straßenbahn oder einem Bus geführt werden.

Es kann auch gesagt werden, vorteilhafterweise strahlt die Lichtquelle, die sich aus mehreren LEDs bilden kann, in einem Wellenlängenbereich mit Wellenlängen, die höherwellig sind als 390 nm (also 400nm, 450 nm, 500 nm oder dergleichen).

Als besonders vorteilhaft für die Keimzahlreduktion hat sich LED-Licht in einem Wellenlängenbereich um 410 nm erwiesen. Wird der daran anschließende Spektralbereich auf seine Wirksamkeit hin untersucht, kann zu längeren Wellenlängen (als 410 nm) bzw. zu niedrigeren Energien (als 24.390 cm⁻¹, d.h. Wellenzahlen) der Lichtquanten hin die Keimzahlreduktion durch das Licht weniger effizient sein bzw. absinken. Zu kürzeren Wellenlängen bzw. zu höheren Energien der Lichtquanten hin kann die Lichteinstrahlung auf körpereigene Zellen, insbesondere bei andauernder Bestrahlung ohne Begrenzung der Dosis, zumindest zu einer verstärkten Zellalterung führen. Die Wellenlänge von 410 nm wird vorzugsweise von geeigneten LEDs mit einer Lichtintensität innerhalb des Wellenlängenbereichs der Halbwertsbreite der spektralen Lichtintensitätsverteilung bereitgestellt. Die Wellenlänge von 410 nm kann das spektrale Intensitätsmaximum einer für die Behandlung vorteilhaften LED sein. Ein für eine effektive Behandlung mit Licht vorteilhaftes Intensitätsmaximum einer Lichtquelle wie einer LED kann auch bei 400 nm oder bei 390 nm oder in einem Wellenlängenbereich zwischen 390 nm und 440 nm liegen. Ein für eine effektive Desinfektion besonders interessanter Wellenlängenbereich, insbesondere für ein spektrales Intensitätsmaximum einer geeigneten LED, erstreckt sich von 290 nm bis 390 nm (auf einer Wellenlängenskala). Mit Licht aus einem Spektralleuchtmittel, wie eine oder mehrere LEDs, als Lichtquelle, das von einem Bestrahlungssteckstück bis z. B. in den Rachenansatz der Mundhöhle eingestrahlt wird, lassen sich u. a. zahlreiche Bakterien in der Körperhöhle (z. B. im Mundraum) und auf den Schleimhäuten eliminieren, insbesondere wenn das Licht als Blau-Licht einen Wellenlängenbereich von 400 nm bis 460 nm, wie 440 nm bis 460 nm, umfasst, z. B. mit einem Intensitätsmaximum bei 455 nm und/oder einem Intensitätsmaximum bei 410 nm. Eine Lichtquelle, die ein vorteilhaftes Breitband-Spektralleuchtmittel aufweist, ist mit einer relativ einfachen Sicherheitsvorkehrung, wie einer druck- und/oder temperatur-sensorischen Sicherheitsschaltung, handhabbar, wenn das Breitband-Spektralleuchtmittel Licht im Bereich von UV-B-Licht und/oder UV-A-Licht und/oder Blaulicht in dem Mundsteckstück generiert.

Der Wellenlängenbereich des Lichts ist mit bekannter Messtechnik, wie einem Gitterspektrometer, bestimmbar. Mithilfe eines Strahlungsmessgeräts, wie einem Leistungs- oder Energiemesser, kann sichergestellt werden, dass eine in den Mundraum eingestrahlte Lichtintensität, anders gesagt, die Energie- bzw. Leistungsdichte der auf die Mundschleimhaut auftreffenden optischen Strahlung für den zu behandelnden Menschen und/oder das Tier unschädlich ist.

Die LEDs können für eine Abgabe von Licht in einer Bestrahlungsdosis von 430 J*cm⁻² ausgelegt sein. Noch bessere bzw. schnellere Wirkungen werden erzielt, wenn das Licht mit einer Bestrahlungsdosis von 1.300 J*cm⁻² in den bestrahlten Bereich abgestrahlt wird.

Eine Halbwertsbreite einer geeigneten LED kann bis zu 200 nm betragen. Besonders vorteilhaft sind LEDs mit einer Halbwertsbreite von 5 nm bis 50 nm. Bei kleinerer Halbwertsbreite der spektralen Intensitäten einer LED, z. B. zwischen 2,5 nm und 25 nm, kann die eingesetzte elektrische Energie aus einem Energiespeicher besonders energieeffizient in therapeutisch wirksames Licht umgewandelt werden. Die begrenzte Halbwertsbreite der Lichtquelle erlaubt es, eine Blaulicht-LED von LEDs für andere Lichtfarben, wie von einer Grünlicht-LED, zu unterscheiden. Besonders schmal ist die Halbwertsbreite von Blaulicht-Laser-LEDs, die mitunter zur Behandlung bzw. zur Desinfektion bevorzugt eingesetzt werden. Solche LEDs können als Blaulicht(-Laser)-LEDs bezeichnet werden, auch wenn ein weiterer Teil ihres Spektralbereichs, insbesondere jenseits der Halbwertsbreite, außerhalb des Blaulichtbereichs liegt. Die Halbwertsbreite umfasst den Wellenlängenbereich, in dem die spektrale Lichtintensität ausgehend von einem spektralen Maximum um weniger als eine Hälfte (bis zu einer Intensität mit einem Faktor 0,5 des spektralen Maximums) abfällt.

Besonders vorteilhaft ist ein Akkumulator, der mechanisch fest über eingelötete Lötfahnen mit der Steuerungsplatine (elektronische Steuerung) verbunden ist, weil damit eine dauerhaft flüssigkeitsdichte Einkapselung des Akkumulators bzw. der elektronischen Komponenten in dem Bestrahlungssteckstück möglich ist. Der Energiespeicher kann zur besseren Gleichgewichtslage des Bestrahlungssteckstücks im Mund des Nutzers beitragen.

Wenn das Bestrahlungssteckstück mit einer elektromagnetischen Empfangseinheit ausgestattet ist, die das Laden des Energiespeichers des Bestrahlungssteckstücks durch Induktion ermöglicht, kann das gesamte Bestrahlungssteckstück, oder zumindest alle elektronischen Komponenten des Bestrahlungssteckstücks, von der Hülle eingeschlossen sein. Das elektrische Laden des Energiespeichers kann z. B. an einem mit einer elektromagnetischen Sendestation ausgestatteten Halter für das Bestrahlungssteckstück erfolgen. Solche Bestrahlungssteckstücke sind besonders leicht zu sterilisieren.

Der knopfartige Korpus kann einen Fortsatz, wie einen Halteknopf, insbesondere für die Aufnahme von knopfartigen Batteriezellen, aufweisen. Anders gesagt, kann der Halteknopf zylinderartig von dem Korpus abstehen. Der Fortsatz ist als Griff ausgebildet. Wenn an dem knopfartigen Fortsatz, insbesondere an dessen Oberfläche, zwei einander gegenüberliegende Fingergriffmulden vorhanden sind, lässt sich das Bestrahlungssteckstück besonders gut zwischen Daumen und Zeigefinger halten. Ein Fortsatz eignet sich besser zum Tragen eines Bestrahlungssteckstücks als ein umklappbarer Ring.

Ein Griff des Bestrahlungssteckstücks befindet sich also, vorzugsweise in axialer Verlängerung entlang einer Achse des Einführteils bzw. des Einführteils, z. B. in einer bezüglich einer Auflage gespiegelten Richtung, die von dem Einführteil wegweist, an dem Bestrahlungssteckstück. Durch eine von dem Einführteil beabstandete Anordnung des Griffs an dem Bestrahlungssteckstück können Verunreinigungen des Einführteils durch Fingerabdrücke weitgehend ausgeschlossen werden. Eine Person, die das Bestrahlungssteckstück handhabt, kommt, bei korrekter, sicherer Handhabung am Griff, nicht in Berührung mit Körperflüssigkeiten der behandelten Person oder des behandelten Tiers.

Das Einführteil, das durch den Mundhöhlenkörper gebildet sein kann, ist nach außen von einem gesundheitsverträglichen Material, wie mundspeichel-, nahrungsmittel- und getränkekompatiblen Material, z. B. einem Kunststoff, begrenzt. Der (wenigstens bereichsweise) transluzente Körper bildet die äußere Hülle des Einführteils, das z. B. im Mund positionierbar ist. Anders gesagt, ist der transluzente Bereich des Mundsteckstücks biokompatibel für den Organismus, wie für den menschlichen Organismus, für den das Mundsteckstück bestimmt ist. Das Einführteil kann aber auch eine kleinere Längserstreckung als der Mundhöhlenkörper aufweisen, wenn an dem Mundhöhlenkörper zusätzliche Befestigungs- bzw. Halteteile vorgesehen sind, wie eine Auflage bzw. eine Anlagefläche.

Der vordere, eine Hüllenlänge abschließende Bereich bzw. ein die Hülle beendender Bereich kann auch als Hüllenkappe bezeichnet werden. Die Hülle als Teil des Einführteils schließt die im Inneren des Einführteils angeordneten Komponenten ein. In dem Inneren können sich elektronische bzw. elektrooptische Komponenten, Energieleitungen für elektrischen Strom und/oder Wärme abführende Teile sowie vorzugsweise ein Träger für die einzelnen Komponenten befinden, die miteinander vergossen sein können. Die Hülle bildet aufgrund ihrer Materialeigenschaften einen flüssigkeitsdichten Abschluss für die in ihrem Inneren angeordneten Komponenten, z. B. einen Abschluss gegenüber dem Mundraum, der bis zu einem Gehäuse reicht. Der die Hülle bildende Körper kann aus einem Kunststoff, z. B. aus einem Silikon, gefertigt sein.

Das Bestrahlungssteckstück, zumindest aber sein Einführteil kann entweder eine Kunststoffhülle umfassen oder eine Latex-Hülle. Naturkautschuk-Latex bietet vorteilhafterweise eine ausreichende Festigkeit für ein Einführteil. Ein Beispiel für einen geeigneten synthetischen Latex ist Polyisopren-Latex, der insbesondere keine allergenen Proteine enthält. Je nach Herstellungsverfahren können bei der Produktion von Polyisopren-Latex unterschiedliche Katalysatoren zum Einsatz kommen, die sich mitunter günstig auf Eigenschaften des synthetischen Latex, wie dessen Reißbeständigkeit, auswirken. Beispiele solcher Latex-Varianten sind Titan-Polyisopren-Latex, Lithium-Polyisopren-Latex und Neodyn-Polyisopren-Latex. Eine Hülle, die biegsam und weich ist, fördert den Tragekonform. Die Kunststoffhülle sollte vorteilhafterweise aus einem Kunststoff gefertigt sein, so dass die Kunststoffhülle als weiche, nachgiebige und biegsame Hülle wahrgenommen wird, insbesondere bei einer Schließkraft, die üblicherweise von Schneidezähnen oder einem Schließmuskel ausgeübt wird.

Weitere Materialien, die sich für die Herstellung zumindest von Komponenten des Bestrahlungssteckstücks bzw. des Einführteils eignen, sind Polypropylen, Ethen-Vinyl-Acetat, Acrylonitril-Butadien-Styren und thermoplastische Elastomere bzw. Gummis. Außerdem können Polyethylen, insbesondere als sog. "Low-Density-Polyethylen" sowie Polyethylenterephtalat, insbesondere in der nichtkristallinen bzw. amorphen Variante, als vorteilhafte transparente Materialien zum Einsatz kommen. Zumindest einzelne Komponenten des Mundsteckstücks, wie Gehäuse, LED-Träger oder Hülle des Mundhöhlenkörpers, können aus einem Kunststoff wie Polyurethan gefertigt sein. Weitere vorteilhafte transparente Materialien sind Polycarbonat und Polyamid. Vorteilhafterweise kommen aber nur solche Materialien zum Einsatz, die keine hormonartige Wirkung entfalten. Die Abgabe hormonartiger Wirkstoffe in den Speichel lässt sich ggf. durch eine Beschichtung mit einem inerten, transparenten Material, wie einem Latex, weitgehend verhindern. Transparente Materialien, die zumindest Komponenten eines Mundsteckstücks bilden, können eine lichtabsorbierende oder lichtabblockende Substanz, wie ein Titanoxid oder ein Graphit oder einen Farbstoff, als Beimischung in einem Segment (bzw. einer Komponente) enthalten, z. B. im Bereich der Auflage des Mundsteckstücks, des Ohrsteckstücks oder des Nasensteckstücks, damit kein UV-Licht zu den Lippen oder in die Umgebung austreten kann. Ein Material geeigneter Elastizität lässt sich beispielsweise mithilfe von Testverfahren nach Shore spezifizieren bzw. auswählen, wie ein Shore-A-Testverfahren. Vorteilhafte elastische Materialien für ein Mundsteckstück haben eine Härte in einem Bereich von 10 Shore bis 70 Shore, vorzugsweise von 20 Shore bis 50 Shore. Damit ist ein günstiger Kompromiss zwischen Elastizität und Beständigkeit getroffen.

Es ist auch möglich, einen transluzenten bzw. transparenten Körper als ein Mehrschichtsystem herzustellen, indem z. B. das Mundsteckstück durch Eintauchen eines transparenten Grundkörpers in einen Latex, wie in einen synthetischen Latex und/oder wie in eine Polymerlösung, geschaffen wird.

Je nach Betrachtungsweise kann das Einführteil auch als ein Stiel oder Stab des Bestrahlungssteckstücks bezeichnet werden, an dem, wenn es sich um ein Mundsteckstück bei dem Bestrahlungssteckstück handelt, das Mundsteckstück freihändig im Mund gehalten werden kann. Mit den Eigenschaften schnullerartig, stabartig bzw. zapfenartig werden gängige Formgebungen für ein Einführteil benannt. Das Einführteil kann z. B. entlang der Länge des Einführteils bezüglich einer Vertikalebene spiegelsymmetrisch, bezüglich einer Horizontalebene spiegelsymmetrisch, bezüglich einer Zentralachse rotationssymmetrisch gerade erstreckt oder auch bezüglich einer Zentralachse rotationssymmetrisch gekrümmt sein. Sowohl bei dem Begriff "Stiel" als auch bei dem Begriff "Stab" assoziieren viele Menschen einen länglichen, schmalen, häufig auch dünnen, Gegenstand. Folglich kann der Begriff "Stiel" auch als Synonym für "Stab" in vielen Fällen verwendet werden.

Vorzugsweise ist mindestens eine LED (oder es sind zwei, drei oder vier LEDs) an einer LED-Träger-Spitze, die zu dem Spitzenbereich des Bestrahlungssteckstücks bzw. des Einführteils gehört, angeordnet. Eine Flächengröße der LED-Träger-Spitze kann größer als eine Querschnittsfläche des LED-Trägers, insbesondere in einer Nähe der Anlagefläche, sein, damit mehrere LEDs an der Spitze befestigt sein können. Vorzugsweise sind an der LED-Träger-Spitze LEDs für unterschiedliche Wellenlängenbereiche, insbesondere mit unterschiedlichen Abstrahlorientierungen, befestigt. Anders gesagt, durch eine Rundung oder einen Winkel der LED-Träger-Spitze ist mindestens eine, vorzugsweise sind (zwei oder mehr) Abstrahlorientierung(en) der an dem LED-Träger angebrachten Spitzen-LEDs vorgebbar.

Der Einschaltknopf ist vorzugsweise von einer Stelle, die sich weiterhin außerhalb der Körperhöhle (z. B. der Mundhöhle) befindet, betätigbar. D. h., der Einschaltknopf sollte an einer Stelle des Bestrahlungssteckstücks angeordnet sein, sodass der Einschaltknopf zugänglich bleibt, obwohl das Bestrahlungssteckstück in der Körperhöhle (z. B. im Mund) einer Person steckt. Im Falle, dass es sich bei dem Bestrahlungssteckstück um ein Mundsteckstück handelt, hat es einen Teil, der nicht für die Aufnahme in einem Mund vorgesehen ist. Z. B. ist es vorteilhaft, wenn ein griffartiger Körper (Griff, Knopf, Handling-Block oder Ähnliches) vorhanden ist, der so groß ist, dass er nicht in einem (normal dimensionierten) Mund aufgenommen werden kann. An diesem griffartigen Körper kann dann auch der Einschaltknopf vorhanden sein.

Ein blockartiger Griff kann z. B. in dem Fall, dass das Bestrahlungssteckstück als Mundsteckstück gestaltet ist, ein mit der Auflage des Mundhöhlenkörpers verbundener, vorzugsweise quaderartiger, Korpus sein, der zumindest einen Länge, wie eine Länge in einer Vertikalrichtung und/oder eine Länge in einer Horizontalrichtung, zwischen 5 mm und 50 mm aufweist. Wird von einem blockartigen Korpus gesprochen, so unterscheidet sich dieser von einem kastenartigen Korpus durch die fehlende Klappbarkeit. Ein kastenartiger Korpus ist vorteilhafterweise aufklappbar, um den Energiespeicher, also mindestens eine Batterie bzw. mindestens einen Akkumulator, aus dem Batteriefach herausnehmen zu können oder in das Batteriefach einsetzen zu können. Der blockartige Korpus ist vorteilhafterweise hermetisch abgeschlossen. Ein Öffnen des blockartigen Korpus, ggf. zum Austausch von Elektronikplatinen oder von Energiespeichern, ist nicht (z. B. weil es ein verschweißter Kunststoffkasten ist) oder nur mithilfe eines Werkzeugs möglich (Sicherungsfunktion).

Handelt es sich bei der Hülle um eine Silikonhülle für ein erstes Mundsteckstück, so hat die Silikonhülle im Bereich des Mundhöhlenkörpers eine äußere Breite von 16 mm, eine äußere Länge von 84 mm und eine äußere Höhe von ca. 12 mm. In einer Nähe zur Auflagefläche an der Silikonhülle weist die Silikonhülle zur Aufnahme des LED-Trägers in ihrem Inneren eine innere Breite von etwa 30 mm auf. Diese innere Breite dient der Aufnahme eines Steckverbinders des LED-Trägers. Im Übrigen hat die Hülle eine innere Breite, in die der LED-Träger aufgrund seiner Breite passt. Eine innere Höhe der Hülle beträgt etwa 9 mm. Im Bereich der Auflagefläche hat die Silikonhülle eine äußere Breite von 74 mm und eine äußere Höhe von 37 mm. Ein Neigungswinkel an einer Endschräge der LED-Träger-Spitze beträgt, von einer Oberseite des LED-Trägers kommend, etwa 315 ± 5°. Die Spitze der Silikonhülle weist eine entsprechend abgewinkelte Fläche auf. Durch diesen abgewinkelten Flächenbereich der Silikonhülle hindurch sowie durch benachbarte Flächenbereiche der Silikonhülle hindurch kann blaues Licht von den LED-Paaren in den posterioren Bereich einer Mundhöhle eines Benutzers gelangen. Der LED-Träger hat von seiner Spitze bis zu seinem Steckverbinder eine Länge von 70 mm und eine Breite von 12 mm. Eine maximale Höhe des LED-Trägers beträgt an den Enden jeweils 6 mm bis 7 mm und in der Mitte etwa 2 mm. Der LED-Träger ist wie ein Bogen gewölbt bzw. gekrümmt. Die aufgezogene, flexible Silikonhülle folgt dem Verlauf des Bogens. Unter dem Bogen findet die Zunge eines Nutzers des Mundsteckstücks Platz. An dem Mundhöhlenkörper kann das Mundsteckstück wie an einem Stiel zum Halten zwischen Zunge und Gaumendach bzw. zwischen den Schneidezähnen eingeklemmt werden. Anders gesagt, weist der Mundhöhlenkörper einen oberen Stielbogen bzw. oberen Stabbogen und einen unteren Stielbogen bzw. Stabbogen auf, der insbesondere durch die Krümmung des LED-Trägers vorgegeben ist. Die LEDs sind zusammen mit einer Stromzuleitungsschaltung in ein auf dem LED-Träger befestigtes Band integriert. Eine Breite des Korpus im Bereich der Anlagefläche beträgt 69 mm und seine Höhe beträgt im Bereich der Anlagefläche etwa 33 mm. Der blockartige Korpus im Anschluss an den kragenartigen Bereich der Anlagefläche hat eine Höhe von etwa 21 mm und eine Breite von etwa 63 mm sowie eine maximale Längserstreckung von etwa 40 mm. In den Korpus ist auf halber Breite eine Betriebszustandsanzeige-LED integriert, die hinter einer blauen Lichtfilterabdeckung sitzt. Das Gewicht des Mundsteckstücks beträgt etwa 70 g (Gramm). Eine Genauigkeit der Längenangaben beträgt ± 1 mm und eine Genauigkeit der Gewichtsangabe beträgt ± 1 g.

Die zuvor dargestellten Kombinationen und Ausführungsbeispiele lassen sich auch in zahlreichen weiteren Verbindungen und Kombinationen betrachten.

Neben den zuvor dargestellten Varianten und Ausführungsformen ist es auch vorstellbar, dass das Bestrahlungssteckstück eine Kombination unterschiedlicher Lichtquellen beinhaltet, z. B. eine UV-Lichtquelle neben einer Blaulicht-Lichtquelle. Ist die Steuerung des Bestrahlungssteckstücks etwas raffinierter gestaltet, so kann zwischen dem Betrieb der UV-Lichtquelle und der Blaulichtquelle mit einem zeitlichen Muster hin- und hergeschaltet werden. Hierbei ist es besonders vorteilhaft, wenn die Phasen der einen Lichtbestrahlung und die Phasen der anderen Lichtbestrahlung nicht gleich lang, sondern in Abhängigkeit des zu erreichenden Ziels in unterschiedlichen prozentualen Anteilen betrieben werden.

Darüber hinaus ist es möglich, an dem Anschlag bzw. an der Anlage einen Leuchtkranz zu haben, der einen Betrieb des Bestrahlungssteckstücks optisch anzeigt, insbesondere dann leuchtet, wenn zumindest eine LED des Einführteils leuchtet bzw. betrieben wird.

Überraschenderweise hat sich gezeigt, dass mit dem Corona-Virus infizierte Menschen, die Halsschmerzen und andere corona-typische Krankheitssymptome aufweisen, durch wenige Behandlungen bzw. Eigenbehandlungen mit einem zuvor beschriebenen Bestrahlungssteckstück in der Ausformung als Mundsteckstück geheilt werden können. So hat sich in einem Fall gezeigt, dass eine Person, die Krankheitssymptome zeigte und einen positiven Corona-Test vorweisen musste, nach nur einer einstelligen Anzahl Behandlungen, z. B. nach fünf Behandlungen, geheilt war. Ein zweiter Corona-Test nach nur fünf Tagen fiel negativ aus. Die Person hatte nach dem ersten positiven Corona-Test und bis zu dem zweiten Corona-Test einmal täglich während einer Gesamtperiode von fünf Tagen ein Mundsteckstück in ihren Mund eingeführt und für ca. 15 Minuten das Mundsteckstück betrieben bzw. blau leuchten lassen.

Ein autonom versorgtes, d. h. mit einem Speicher für elektrische Energie ausgestattetes Mundsteckstück kann auch als mobiles Gerät von Personen in öffentlichen Gebäuden und Verkehrsmitteln mitgeführt werden. Sollte eine Person feststellen, dass sie sich einer erhöhten Gefährdungslage aussetzen musste, z. B. aufgrund von unterschrittenen Mindestabständen oder z. B. aufgrund von unerwünschten Ereignissen (wie Anhusten, wie Anspucken, wie Anhauchen durch einen Dritten), kann sie unverzüglich Gegenmaßnahmen ergreifen, indem sie sich das Bestrahlungssteckstück in den Mund steckt und für eine gewisse Dauer, z. B. für 10 Minuten oder 15 Minuten einschaltet, um insbesondere eingetretene oder eintretende Keime durch Licht oder sonst geeignete Wellenlängen abzutöten.

Eine andere Person, mit der zusammen ein erster Prototyp eines Analstabs getestet wurde, berichtete über einen Reizdarm. Diese Person nutzte einen Analstab jeweils einmal täglich über eine Dauer von mehreren Tagen und fühlte sich nach einer relativ kurzen Periode der Benutzung deutlich besser.

Das erfindungsgemäße Bestrahlungssteckstück kann als Wohlbefindenssteigerungsmittel, selbst von ungeübten Nutzern, verwendet werden.

### Figurenkurzbeschreibung

Die vorliegende Erfindung kann noch besser verstanden werden, wenn Bezug auf die beiliegenden Figuren genommen wird, die beispielhaft besonders vorteilhafte Ausgestaltungsmöglichkeiten darlegen, ohne die vorliegende Erfindung auf diese einzuschränken. Mit anderen Worten,
- Figur 1: zeigt eine erste Ausführungsform eines erfindungsgemäßen Bestrahlungssteckstücks, das als Mundsteckstück verwendbar ist,
- Figur 2: zeigt in einer perspektivischen Ansicht eine zweite Ausführungsform eines erfindungsgemäßen Bestrahlungssteckstücks, das ebenfalls als Mundsteckstück verwendbar ist,
- Figur 3: zeigt eine erste Seitenansicht der Ausführungsform nach Figur 2,
- Figur 4: zeigt eine zweite Seitenansicht der Ausführungsform nach Figur 2,
- Figur 5: zeigt eine Ansicht mit Blickrichtung auf einen Kastenkörper gem. der Ausführungsform nach Figur 2,
- Figur 6: zeigt eine Explosionsdarstellung einer dritten Ausführungsform eines erfindungsgemäßen Bestrahlungssteckstücks, das als Mundsteckstück einsetzbar ist,
- Figur 7: zeigt in perspektivischer Darstellung eine Variante eines LED-Trägers ohne Mundschutz mit weiteren Bauteilen eines Mundsteckstücks gem. der Ausführungsform nach Figur 6,
- Figur 8: zeigt das Innere des Gehäuses mit dem Batteriefach gem. der Ausführungsform nach Figur 6,
- Figur 9: zeigt in einer Schnittansicht einen Schnitt durch das Mundsteckstück gem. der Ausführungsform nach Figur 6,
- Figur 10: zeigt, wie eine vierte Ausführungsform eines erfindungsgemäßen Bestrahlungssteckstücks, insbesondere in Gestalt als Mundsteckstück, in einen Mundraum einer Person eingesetzt werden kann,
- Figur 11: zeigt eine fünfte Ausführungsform eines erfindungsgemäßen Bestrahlungssteckstücks,
- Figur 12: zeigt eine sechste Ausführungsform eines erfindungsgemäßen Bestrahlungssteckstücks,
- Figur 13: zeigt eine siebte Ausführungsform eines erfindungsgemäßen Bestrahlungssteckstücks,
- Figur 14: zeigt einen LED-Streifen auf einer Platine, die mit dem LED-Träger nach Figur 13 zu einem Bestrahlungssteckstück zusammenfügbar ist,
- Figur 15: zeigt eine achte Ausführungsform eines erfindungsgemäßen Bestrahlungssteckstücks,
- Figur 16: zeigt eine neunte Ausführungsform eines erfindungsgemäßen Bestrahlungssteckstücks,
- Figur 17: zeigt eine zehnte Ausführungsform eines Bestrahlungssteckstücks, das aufgrund einer ersten Hülle zu einem mit einem Vaginaleinführteil und aufgrund einer zweiten Hülle zu einem mit einem Analeinführteil gestaltbar ist,
- Figur 18: zeigt eine elfte Ausführungsform eines Bestrahlungssteckstücks, das aufgrund eines Satzes Hüllen zu einem mit einem Analeinführteil und wahlweise zu einem mit einem Vaginaleinführteil gestaltbar ist,
- Figur 19: zeigt die elfte Ausführungsform in Gestalt als Analbestrahlungssteckstück,
- Figur 20: zeigt eine zwölfte Ausführungsform in Gestalt als Vaginalbestrahlungssteckstück,
- Figur 21: zeigt die zwölfte Ausführungsform in einer Einzelteilansicht bzw. Explosionsansicht,
- Figur 22: zeigt eine dreizehnte Ausführungsform eines Bestrahlungssteckstücks in Gestalt eines ersten Gehörgangbestrahlungssteckstücks,
- Figur 23: zeigt eine vierzehnte Ausführungsform eines Bestrahlungssteckstücks in Gestalt eines zweiten Gehörgangbestrahlungssteckstücks und
- Figur 24: zeigt eine fünfzehnte Ausführungsform eines Bestrahlungssteckstücks in Gestalt eines Nasenloch-, genauer eines Nasenlöcherbestrahlungssteckstücks.

### Figurenbeschreibung

Ein Bestrahlungssteckstück in Gestalt eines Mundsteckstücks 1 kann derart gestaltet sein, dass es wie ein Schnuller im vorderen Mundraum, z. B. an Zunge 25 und Gaumen 26, anliegen kann. Der nicht für die Anlage vorgesehene Bereich des Mundsteckstücks 1, genauer des Mundhöhlenkörpers 2, ist für die Abgabe von Licht 15 aus einer Lichtquelle 6, die im Mundhöhlenkörper 2 angeordnet ist, vorgesehen. Mit einem solchen Mundsteckstück 1 ist es möglich, die Keimzahl, insbesondere im Rachenbereich eines Menschen, durch Lichtbestrahlung zu reduzieren.

In Versuchen zeigte sich, dass nach einer Bestrahlungsdauer von 15 Minuten die Keimzahl reduziert war. Zahlreiche Untersuchungen haben gezeigt, dass häufig Ansteckungsrisiken und Ansteckungsverläufe zu einer Virenlast bzw. Virendichte korrespondieren, sodass auch schon kürzere Anwendungszeiten positive Effekte hervorbringen können. Deutliche Keimzahlreduktionen waren nach solchen Zeiten wie z. B. 15 Minuten feststellbar (wobei die Versuche auch zeigten, dass die kummulierte Lichtenergiemenge ebenfalls einen Einfluss auf die Dauer hat).

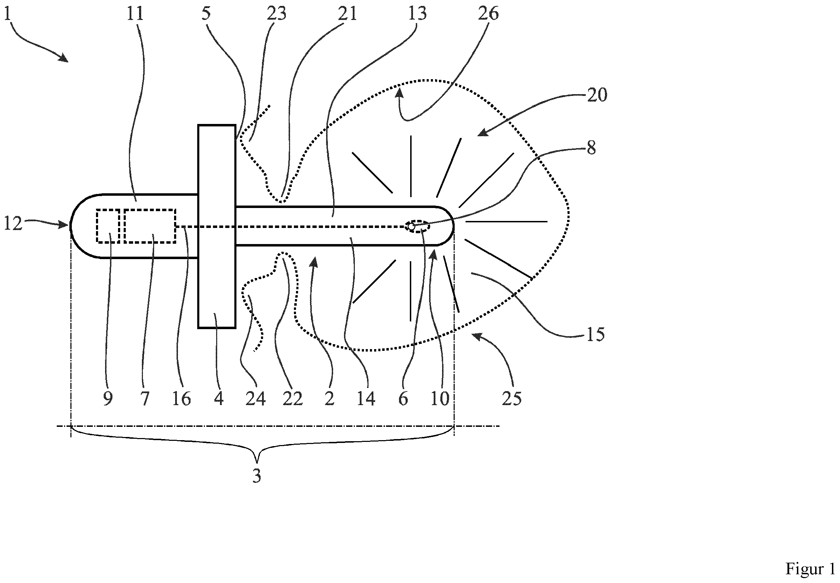

In Figur 1 ist ein Ausführungsbeispiel eines erfindungsgemäßen Mundsteckstücks 1 grob schematisch dargestellt. Es besteht aus einem knopfartigen Körper 11, der als Griff dient, einer Anlagefläche 4, die auch als Schild bezeichnet werden kann, und einem Mundhöhlenkörper 2. Der knopfartige Körper 11 hat die Funktion eines Verwahrkastens. Der knopfartige Körper 11 erfüllt zudem die Funktion eines Griffs. Im Inneren des Verwahrkastens (Körper 11) sind weitere Komponenten des Mundsteckstücks 1.

Im knopfartigen Körper 11 sind in der dargestellten Ausführung eine Energiequelle 7, z. B. eine Batterie oder ein Akkumulator, und eine Zeitsteuerung 9 untergebracht. Es ist auch ein elektrischer Anschluss 12 vorgesehen, um z. B. einen eingesetzten Akkumulator zu laden, ohne dass er entnommen werden muss.

Im Mundhöhlenkörper 2 ist als Lichtquelle 6 eine LED 8 vorgesehen. Es können auch mehrere LEDs vorhanden sein, oder auch andere Lampen. Die Lichtquelle 6 und die Energiequelle 7 sind über Kabel 16 miteinander verbunden.

Im vorderen Bereich, d. h. im Bereich der Spitze des Mundhöhlenkörpers 2, ist ein Lichtabgabebereich 10 vorgesehen. Dieser Bereich des Mundhöhlenkörpers 2 ist transluzent, sodass das von der Lichtquelle 6 abgestrahlte Licht 15 austreten kann. Ist nur der vordere Bereich transluzent, so hat dies den Vorteil, dass gezielt der dem Rachen zugewandte Bereich der Mundhöhle bestrahlt werden kann, wohingegen der Bereich der Mundhöhle 20, der hinter den Zähnen liegt, unbestrahlt bleibt. Der Mundhöhlenkörper 2 liegt im in den Mund eingesetzten Zustand auf der Zunge 25 auf und an dem Gaumen 26 an und bestrahlt den Rachenraum (nicht dargestellt).

Der Mundhöhlenkörper 2 ist in der dargestellten Ausführungsform stabförmig und im Querschnitt rotationssymmetrisch. Er besteht aus zwei Halbschalen, der ersten Halbschale 13 und der zweiten Halbschale 14. Der Mundhöhlenkörper 2 kann jedoch auch asymmetrisch gestaltet sein, wie es beispielsweise von handelsüblichen Babyschnullern bekannt ist. In allen Fällen ist zumindest der vordere Bereich, d. h. der Bereich, der von der Auflage 5 des Mundsteckstücks 1 am weitesten entfernt ist, transluzent.

Aus Figur 1 ergibt sich, wie das Mundsteckstück 1 im Mund eingesetzt ist. Mit Hilfe des knopfartigen Körpers 11 wird der Mundhöhlenkörper 2 in den Mund eingeführt. Die Anlagefläche 4, das Schild, dient dabei als Anschlag. Die Anlagefläche 4 erstreckt sich ungefähr rechtwinklig zur Längserstreckung 3 des Mundsteckstücks 1. Entsprechend liegt die Auflage 5 im eingesetzten Zustand an den Lippen 23, 24 an. Der Mundhöhlenkörper 2 ist zwischen den Zähnen 21, 22 hindurchgeführt und ragt in die Mundhöhle 20 hinein.

Die in der Figur 1 gezeigte Ausgestaltungsmöglichkeit lässt sich auch mit weiteren Aspekten der allgemeinen Beschreibung in beliebiger Form verbinden.

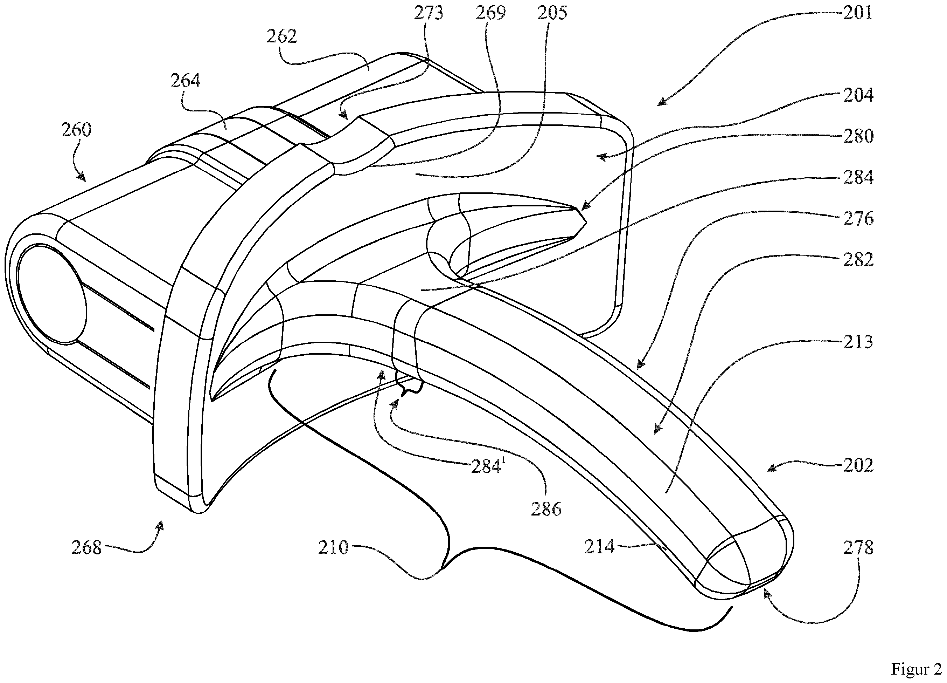

Die Figuren Figur 2 bis Figur 5 zeigen verschiedene Ansichten eines weiteren Ausführungsbeispiels eines Bestrahlungssteckstücks, das als Mundsteckstück 201 verwendbar ist.

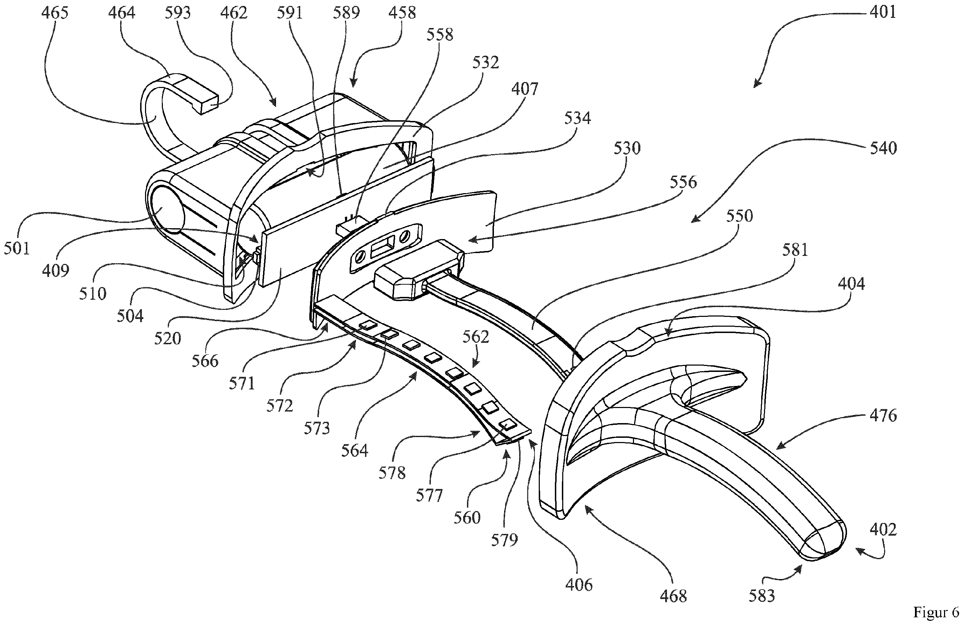

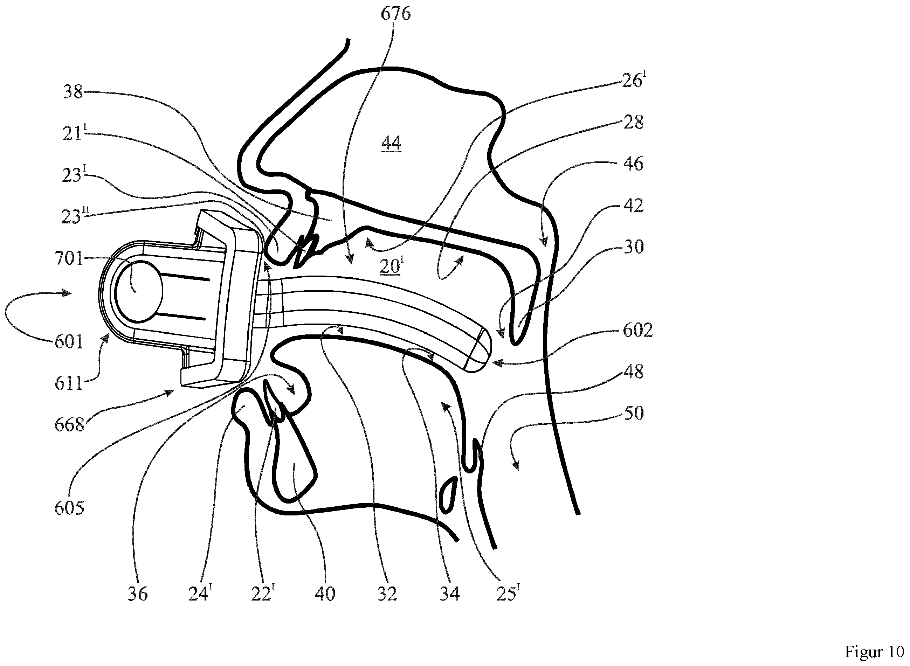

Das in Figur 2 gezeigte Mundsteckstück 201 ist aus einem Mundhöhlenkörper 202 und einem kastenartigen Körper 260 zusammengesetzt. Zwischen dem Mundhöhlenkörper 202 und dem kastenartigen Körper 260 befindet sich ein Mundschutz 268, der eine zylindermantelartig gewölbte Anlagefläche 204 hat. Die Anlagefläche 204 ist konkav dem Mundhöhlenkörper 202 zugewandt. Die Anlagefläche 204 wirkt abdeckend und wölbt sich quer zum Mundhöhlenkörper 202. Die Anlagefläche 204 bildet eine Auflage 205 für einen Gesichtsbereich eines Anwenders des Mundsteckstücks 201. Ein Lippenbezugspunkt 269 dient als Bezugspunkt für eine Eindringtiefe des Mundhöhlenkörpers 202 in eine Mundhöhle (für Details zur Eindringtiefe vgl. die Mundhöhle 20 in Figur 1 bzw. die Mundhöhle 20^{I} in Figur 10). Der Lippenbezugspunkt 269 und eine Dehnungsmulde 273 fallen örtlich zusammen. Durch die Dehnungsmulde 273 wird eine Abnahme bzw. ein Abziehen des Mundschutzes 268 von dem kastenartigen Körper 260 erleichtert. Der Mundschutz 268 ist fest mit dem Mundhöhlenkörper 202 verbunden. Der kastenartige Körper 260 hat eine Fläche, die als Griff 264 genutzt werden kann und die aus dem Gehäuse 262 hervorsteht. Vorteilhafterweise ist der Griff 264 durchsichtig und lässt Licht als eine Art und Weise der Betriebsanzeige austreten, sodass auch außerhalb des Mundes zu erkennen ist (z. B. durch einen Blick des Nutzers des Mundsteckstücks 201 nach unten), ob das Mundsteckstück 201 einwandfrei arbeitet. Das Gehäuse 262 dient u. a. zur Aufnahme eines Akkumulators und einer Steuerplatine in seinem Inneren (vgl. hierzu auch die Ausführungsvariante nach Figur 6). Das Gehäuse 262 ist so groß gestaltet, dass es als Griff 264 dienen kann und zugleich genug Platz für die elektrischen und elektronischen Bauteile zur Ansteuerung des transluzenten Körpers 276 bietet. Der Mundhöhlenkörper 202 ist durch eine erste Halbschale 213 und durch eine zweite Halbschale 214 - von außen betrachtet - abgeschlossen, die gemeinsam eine Kunststoffhülle 282 des Mundhöhlenkörpers 202 darstellen. Die erste bzw. obere Halbschale 213 ist fest mit der zweiten bzw. unteren Halbschale 214 verbunden. Der Mundhöhlenkörper 202 erstreckt sich in Gestalt eines bogenförmigen Stiels 278. Der bogenförmige Stiel 278 ist als transluzenter Körper 276 realisiert, der einen Lichtabgabebereich 210 hat. Der Lichtabgabebereich 210 erstreckt sich - in der Ausführungsvariante nach Figur 2 - über die gesamte Länge des Mundhöhlenkörpers 202. Der Lichtabgabebereich 210 weist einen Verlängerungsbereich 286 auf, der zur Verfügung steht, um in dem Verlängerungsbereich 286 den bogenförmigen Stiel 278 über eine vorgegebene Strecke auseinanderziehen zu können (im Sinne eines verlängerbaren Stiels 278). Der Lichtabgabebereich 210 ist somit in seiner Länge anpassbar. Die beiden Halbschalen 213, 214 sind eine Fortsetzung der Zahnstützflächen 284, 284^{I}, die auch als Schneidezahnbissflächen bezeichnet werden können. An die Zahnstützflächen 284, 284^{I} schließt sich in eine Richtung auf die Anlagefläche 204 hin ein Lippenanschlusskörper 280 an. Der Lippenanschlusskörper 280 besitzt ein Mundöffnungsprofil. Der Lippenanschlusskörper 280 erleichtert eine Abdichtung einer Mundöffnung unmittelbar an der Anlagefläche 204, um z. B. Speichel zurückzuhalten. Der Lippenanschlusskörper 280 erhebt sich von der Anlagefläche 204 und ist fest mit der Anlagefläche 204 und mit der oberen Zahnstützfläche 284 und der unteren Zahnstützfläche 284^{I} verbunden.

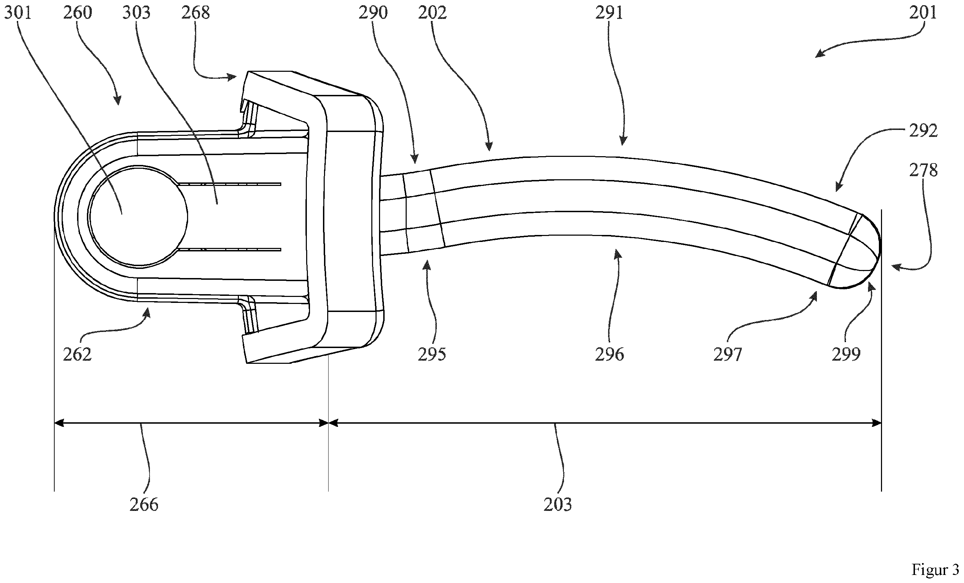

Figur 3 stellt das in Figur 2 gezeigte Mundsteckstück 201 in einer ersten Seitenansicht dar. Der bogenförmige Stiel 278 weist eine Längserstreckung 203 auf. Zur Anpassung an anatomische Gegebenheiten (siehe die anatomischen Gegebenheiten, die in Figur 10 dargestellt sind) wird der bogenförmige Stiel 278 durch mehrere bogenförmige Verläufe gebildet, die voneinander abweichende Krümmungsradien haben. Der Stiel 278 setzt sich aus einer größeren Anzahl ineinander übergehender Bögen bzw. Krümmungen zusammen. Ein erster Stielbogen 290 ist ein gaumenseitiger Stielansatzbogen. Der erste Stielbogen 290 dient der Aufnahme von oberen Schneidezähnen eines das Mundsteckstück 201 umschließenden Mundes (nicht dargestellt). Der erste Stielbogen 290 geht über in einen zweiten Stielbogen 291, der als gaumenseitiger Stielmittenbogen ausgebildet ist. Der zweite Stielbogen 291 folgt dem harten Gaumen. Der zweite Stielbogen 291 geht über in einen dritten Stielbogen 292, der als gaumenseitiger Stielendbogen ausgebildet ist. Der dritte Stielbogen 292 folgt dem weichen Gaumen eines Mundes. Ein vierter Stielbogen 295 liegt dem ersten Stielbogen 290 gegenüber und kann auch als zungenseitiger Stielansatzbogen bezeichnet werden. Der vierte Stielbogen 295 dient der Anlage der unteren Schneidezähne eines nicht dargestellten Mundes. Ein fünfter Stielbogen 296 schließt an den vierten Stielbogen 295 an und bildet einen zungenseitigen Stielmittenbogen. Der fünfte Stielbogen 296 dient zur Anlage an den anterodorsalen Bereich eines Zungenrückens. Der fünfte Stielbogen 296 geht über in einen sechsten Stielbogen 297, der auch als zungenseitiger Stielendbogen bezeichnet werden kann. Der sechste Stielbogen 297 dient zur Anlage an den posterodorsalen Bereich des Zungenrückens. Der gaumenseitige Stielendbogen 292 und der zungenseitige Stielendbogen 297 sind über einen siebten Stielbogen 299 miteinander verbunden, der das mundhöhlenseitige Ende des Mundhöhlenkörpers 202 bildet. Die Längserstreckung 203 des Mundhöhlenkörpers 202 geht bis zu dem siebten Stielbogen 299. Die Längserstreckung 203 wird von einer Längserstreckung 266 des kastenförmigen Körpers 260 fortgesetzt, die auch als außermundige Längserstreckung 266 bezeichnet werden kann.

Wie in Figur 3 zu sehen ist, ergeben die Längserstreckungen 203, 266, also die Längserstreckung 203 des Mundhöhlenkörpers 202 und die Längserstreckung 266 des kastenförmigen Körpers 260, zusammen eine Gesamtlänge des Mundsteckstücks 201. Der kastenförmige Körper 260 ist in den Mundschutz 268 eingesetzt. Seitlich an dem Gehäuse 262 des kastenförmigen Körpers 260 befindet sich ein Einschalttaster 301, der gegen eine rückstellende Tasterfeder 303 betätigbar ist. Der Einschalttaster 301 und die Tasterfeder 303 sind Teile des Gehäuses 262. Einschalttaster 301 und Tasterfeder 303 sind einstückig aus dem Gehäuse 262 heraus ausgeformt.

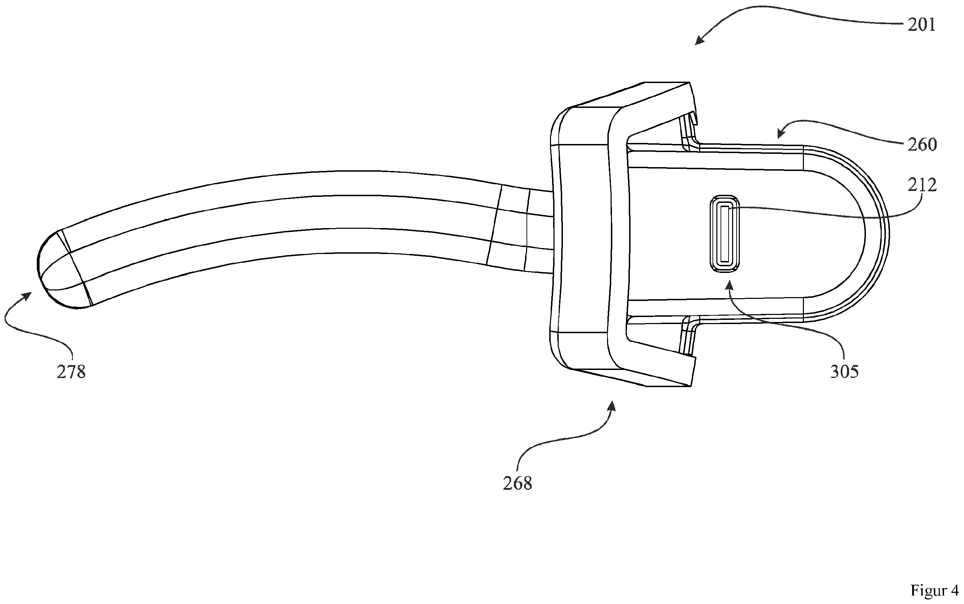

Figur 4 zeigt eine Seite des Mundsteckstücks 201, die der in Figur 3 dargestellten Seite gegenüberliegt. Der kastenartige Körper 260 bildet eine Fortsetzung des bogenförmigen Stiels 278. An dem kastenförmigen Körper 260 ist eine USB-Buchse 305 vorhanden. Die USB-Buchse 305 dient als elektrischer Anschluss 212 zur Versorgung des Mundsteckstücks mit Energie bzw. mit elektrischem Strom. Der bogenförmige Stiel 278 wird durch den Mundschutz 268 gegenüber dem elektrischen Anschluss 212 abgeschirmt. Die USB-Buchse 306 ist für eine Kleinspannung und einen Niedrigstrom ausgelegt, sodass selbst bei einer Berührung der Buchse 305 mit (nicht dargestellten) Lippen keine bemerkenswerten elektrischen Schläge auftreten können.

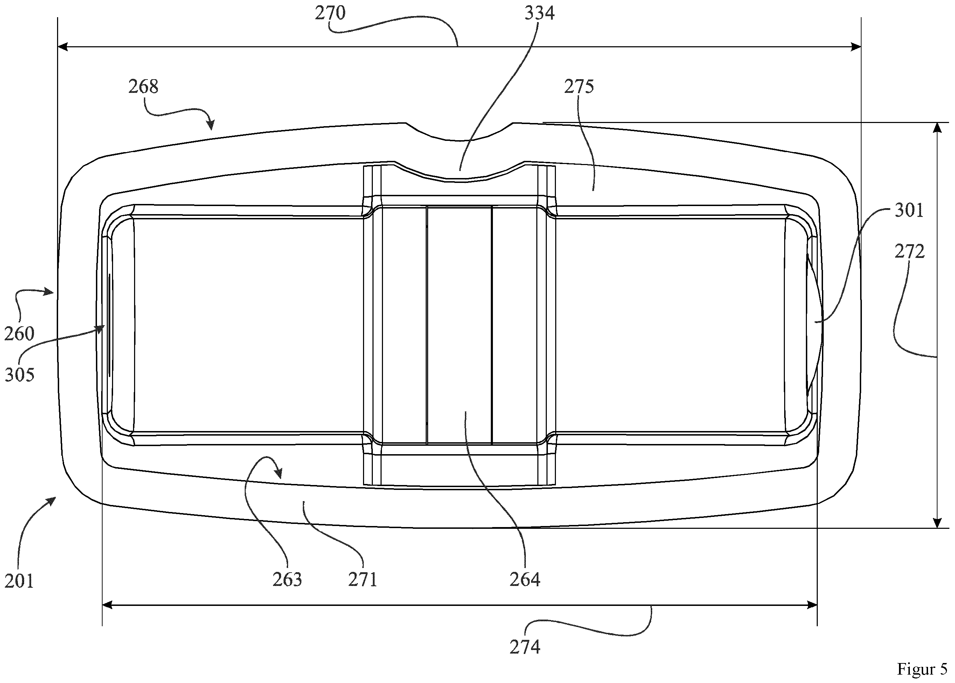

In Figur 5 ist eine schematisch dargestellte Draufsicht auf den kastenförmigen Körper 260 des Mundsteckstücks 201 gezeigt. Der Mundschutz 268 ist mit einer Anschlusslippe 271 auf eine Anschlusskante 263 des kastenförmigen Körpers 260 aufgesteckt und wird durch eine Kippnase 334, die in den Mundschutz 268 eingearbeitet ist, in Position gehalten. An dem kastenartigen Körper 260 befinden sich an dessen Schmalseiten, an jeweils einander gegenüberliegenden Seiten, zum einen die USB-Buchse 305 und zum anderen der Einschalttaster 301. Mittig zwischen USB-Buchse 305 und Einschalttaste 301 ist der Griff 264 an dem kastenförmigen Körper 260 angeordnet, der zur besseren Handhabung eine Oberflächengriffstruktur (nicht eingezeichnet) aufweist, wobei die Oberflächengriffstruktur durch eine Aufrauhung realisiert ist. In unmittelbarer Nähe zur Anschlusskante 263 bzw. zur Kippnase 334 sind in dem Mundschutz 268 Durchlüftungsöffnungen, wie die Durchlüftungsöffnung 275, eingearbeitet, die mit einem Keimfilter (von außen nicht zu sehen) ausgestattet sind. Die Durchlüftungsöffnungen 275 erleichtern einer das Mundsteckstück 201 tragendenden Person das Atmen durch den Mund, obwohl der Mund durch den Mundschutz 268 gegenüber der Umwelt verschlossen ist. Der Mundschutz 268 weist eine Breite 270 auf, die größer ist als dessen Höhe 272. Der kastenartige Körper 260 weist eine Breite 274 auf, die kleiner ist als die Breite 270 des Mundschutzes 268. Damit ist das Mundsteckstück 201 besonders günstig zwischen Nase und Kinn im Gesicht einer Person zu positionieren und kann trotzdem angenehm getragen werden.

In den Figuren Figur 6, Figur 7, Figur 8 und Figur 9 sind verschiedene Ansichten einer weiteren Ausführungsform eines Bestrahlungssteckstücks, das ebenfalls als Mundsteckstück 401 benutzbar ist, bzw. einzelne Teile dieses Mundsteckstücks 401 wie ein Gehäuse 462 gezeigt.

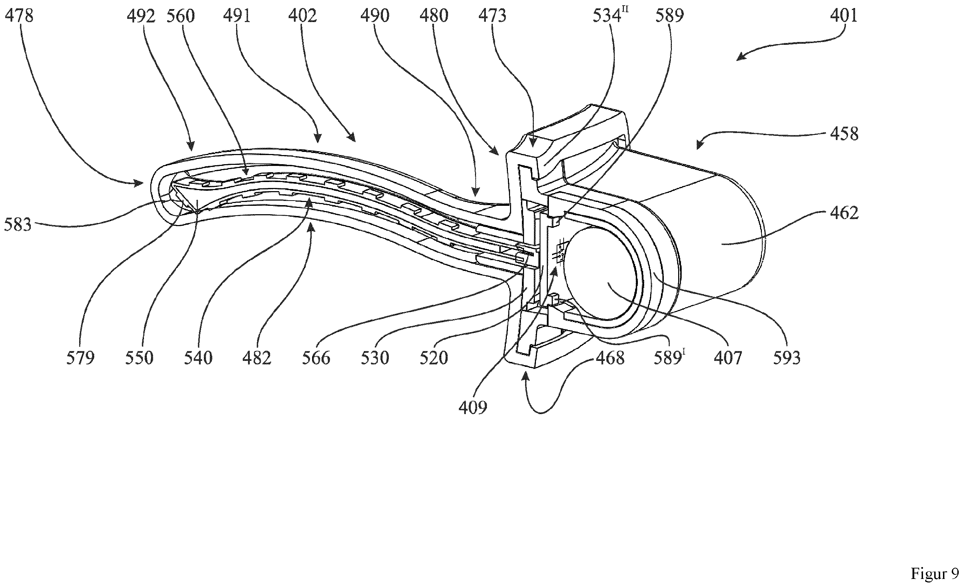

In der Figur 6 ist eine perspektivische Explosionsansicht des Mundsteckstücks 401 zu sehen. Hierdurch sind auch viele im Inneren 540 angeordnete Teile wie die LEDs 571, 572, 572, 577, 578, 579 zu sehen, die in einem zusammengebauten Zustand des Mundsteckstücks 401 (s. die Querschnittsdarstellung nach Figur 9) durch das Gehäuse 462 und den Mundhöhlenkörper 402 eingeschlossen sind. Das Gehäuse 462 ist als blockartiger Körper 458 ausgebildet, der einen Einschalttaster 501 und einen Griff 464 aufweist. Der Griff 464 ist bündig mit dem Gehäuse 462 auf dieses aufschiebbar bzw. aufklippbar, wobei eine durch den Griff 464 gebildete, somit an dem Gehäuse 462 vorhandene Betriebszustandsanzeige 593 auf diese Weise abgedeckt ist. Ein Licht, insbesondere farbiges Licht, der Betriebszustandsanzeige 593 wird über eine Reflektorfläche 465 an dem Griff 464 nach außen hin abgestrahlt bzw. in ihrer Wirkung verstärkt. Diese als Spiegelfläche wirkende Reflektorfläche kann in einer alternativen (bildlich nicht dargestellten) Ausführungsvariante auch als, vorzugsweise weißliche, Lichtstreufläche ausgebildet sein. Der blockartige Körper 458 dient somit zugleich als Verwahrkasten, in dem der Einschalttaster 501 angeordnet ist.

Im Inneren des in Figur 6 gezeigten blockartigen Körpers 458 (bzw. Verwahrkasten) ist ein Batteriefach 510 vorhanden, in das ein handelsüblicher Akkumulator 407 passt (z. B. des Typs "18650" oder des Typs "32650" oder des Typs "32700"). Der Akkumulator 407 ist mit einer Steuerplatine 520 elektrisch verbunden, die ebenfalls in das Batteriefach 510 (und somit in den Verwahrkasten) gehört. Auf der Steuerplatine 520 befindet sich seitlich ein Schalter 504, genauer gesagt, ein Mikroschalter, der durch den Einschalttaster 501 betätigbar ist. Wobei über einen Hebel (nicht dargestellt) auch ein Versatz zwischen Einschalttaster 501 und Schalter 504 ausgeglichen werden kann.

Die Steuerplatine 520 trägt zwei Betriebszustands-LEDs, wie die Betriebszustands-LED 589. Nachdem in einem betriebsbereiten Zustand des Mundsteckstücks 401 der Einschalttaster 501 betätigt wurde, wird von der Betriebszustands-LED 589 Licht in einen Lichtkanal 591 des Batteriefachs 510 an dem Akkumulator 407 vorbei auf das transluzente Fenster 593 der Betriebszustandsanzeige gestrahlt. Durch das Fenster 593 tritt Licht aus, wenn der Betriebszustand eingenommen ist, und wird von einer benachbarten Person unmittelbar erkannt oder über die Reflektorfläche 465 von der das Mundsteckstücks 401 tragenden Person gesehen.

Wie des Weiteren in Figur 6 zu sehen ist, ist über einen Steckverbinder 558 die Lichtquelle 406 an die Steuerplatine 520 elektrisch anschließbar. Der Steckverbinder 558 ermöglicht es, das Mundsteckstück 401 in zwei Teile auseinanderzunehmen, von denen ein erstes Teil der blockartige Körper 458 ist und ein zweites Teil der Mundhöhlenkörper 402 ist. Die Verbindung wird über Kippnasen, wie die Kippnase 534, zusammengehalten. Von dem blockartigen Körper 458 kann der Mundhöhlenkörper 402 abgenommen und durch einen anderen Mundhöhlenkörper, z. B. durch einen Mundhöhlenkörper mit einer größeren Länge oder durch einen Mundhöhlenkörper mit einem breiteren transluzenten Körper als dem transluzenten Körper 476, ersetzt werden. Der Steckverbinder 558 führt durch eine Abschlussplatte 530 hindurch zu einem Anschraubverbinder 556, auf dessen LED-Träger 550 die Lichtquelle 406 angeordnet ist. Die Lichtquelle 406 umfasst einen oberen LED-Strang 562 und einen unteren LED-Strang 564, die jeweils Licht in einander entgegengesetzte Richtungen abstrahlen. Die Lichtquelle 406 ist über elektrische Kontakte 566 zur Stromversorgung mit dem Steckverbinder 558 verbunden, wenn das Mundsteckstück 401 zusammengebaut ist (s. auch die Kontakte 566 in Figur 9). Durch Auflage des Mundschutzes 468 auf der Dichtfläche 532 ist das Innere 540 des blockartigen Körpers 458 flüssigkeitsdicht abgeschlossen.

Auf dem LED-Träger 550 ist ein Temperatursensor 581 vorhanden, der gemessene Temperaturwerte in Gestalt von Messdaten auf in dem LED-Träger 550 verlaufenden Datenleitungen zur Steuerplatine 520 sendet, in dem die Signale über den Steckverbinder 558 hinweg geleitet werden. Der Temperatursensor 581 ist Teil einer Temperaturüberwachung. Der Temperatursensor 581 kann sowohl eine Umgebungstemperatur, z. B. eine Fiebertemperatur im Mund eines Menschen, als auch eine LED-Betriebstemperatur erfassen. Für eine besonders zuverlässige Fiebermessung ist der Temperatursensor 581 mit einem Fühler auszustatten, der an einer Unterseite des Mundhöhlenkörpers hinaus zur Zunge reichen kann.

Die Lichtquelle 406 in Figur 6 umfasst eine größere Anzahl LEDs 571, 572, 573, 577, 578, 579, genauer gesagt 17 LEDs, die zusammen auf einem LED-Band 560 (auch als LED-Stripe bezeichnet) angeordnet sind. Die Stromversorgung dieser LEDs 571, 572, 573, 577, 578, 579 erfolgt aus dem Akkumulator 407 über die Steuerplatine 520, den Steckverbinder 558 und über LED-Stromversorgungskontakte 566 hinweg. Eine erste nach unten gerichtete Blaulicht-LED 572 dient der Bestrahlung des sublaminalen Bereichs im Mundraum. Eine auf dem LED-Band in einem endnahen Bereich angeordnete Gruppe von zwei UV-C-Licht-LEDs 577, 578 dient der Bestrahlung eines tieferliegenden Bereichs der Mundhöhle. Am Ende des LED-Trägers 550 ist eine Laser-LED 579 angeordnet, deren Licht über eine Mikrolinsenoptik 583 des Mundhöhlenkörpers 402 in Bereichen des Rachens verteilt wird (siehe auch die anatomischen Gegebenheiten in Figur 10). Zwischen den UV-C-Licht-LEDs 577, 578 und einer ersten LED 571 bzw. einer dieser LED 571 gegenüberliegenden LED 572 sind weitere Blaulicht-LEDs, wie die Blaulicht-LED 573 angeordnet, deren Licht in einem Betriebszustand des Mundsteckstücks 401 durch den transluzenten Körper 476 austritt. In einem solchen Betriebszustand ist der Mundhöhlenkörper 402 bis zu dessen Anlagefläche 404, die sich an dem Mundschutz 468 befindet, in eine Mundhöhle eingesteckt (vgl. Mundhöhle 20 in Figur 1 bzw. Mundhöhle 20^{I} in Figur 10).

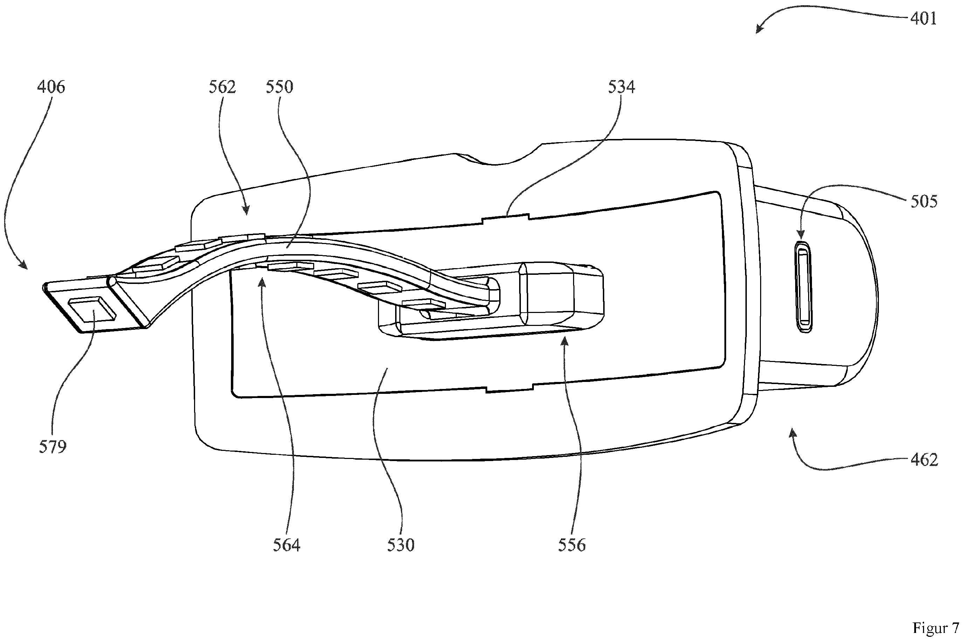

Figur 7 stellt ein teilweise zusammengebautes Mundsteckstück 401 dar, dessen Einzelteile aus Figur 6 bekannt sind. Das Gehäuse 462 weist eine USB-Buchse 505 auf. In das Gehäuse 462 ist mit Hilfe von Kippnasen, wie der Kippnase 534, die Abschlussplatte 530 eingesetzt. An der Abschlussplatte 530 ist der Schraubverbinder 556 rücklings angeschraubt, der den LED-Träger 550 trägt. Auf dem LED-Träger 550 sind der obere LED-Strang 562 und der untere LED-Strang 564 montiert, die der geschwungenen Form des LED-Trägers 550 folgen. Die Laser-LED 579 sitzt am Ende des Trägers 550 und erlaubt es, ausgehend von dem LED-Träger 550 entfernte und tiefergelegene Bereiche des Rachens mit Licht zu bescheinen. Laserlicht lässt sich besonders gut durch eine vor die Laser-LED 579 gesetzte Optik in eine gewünschte Richtung führen und dabei aufweiten (siehe z. B. die in Figur 6 gezeigte Mikrolinsenoptik 583). Um den anatomischen Gegebenheiten (s. Figur 10) gerecht zu werden, führt eine Vorzugsstrahlrichtung in einem Winkel von dem Träger 550 bzw. der Abschlussplatte 530 weg. Die beiden LED-Stränge 562, 564 und die Laser-LED 579 bilden zusammen mit dem Träger 550, der eine Orientierung der darauf angeordneten LEDs 562, 564, 469 vorgibt, die Lichtquelle 406.

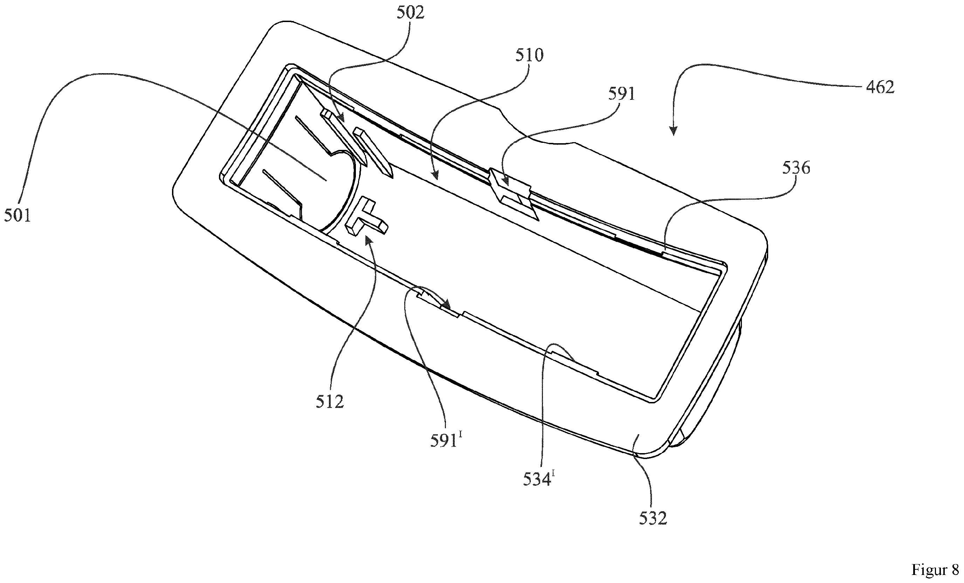

In Figur 8 ist das Gehäuse 462, das aus Figur 6 und Figur 7 bereits bekannt ist, mit einer Blickfreigabe in das Batteriefach 510 gezeigt. Das Batteriefach 510 wird durch das Gehäuse 462 gebildet. Das Gehäuse 462 ist von der Dichtfläche 532 umschlossen. An der Dichtfläche 532 befindet sich eine Dichtkante 536 mit mehreren von Kippnasen, wie z. B. die Kippnase 534^{I}, die dazu dienen, eine Abschlussplatte, wie die in Figur 7 gezeigte Abschlussplatte 530, zu halten und das Batteriefach 510 damit zu verschließen. In dem Gehäuse 462 bzw. in dessen Gehäusewand sind zwei Lichtkanäle 591, 591^{I} eingearbeitet, die der Betriebszustandsanzeige dienen. Das Gehäuse 462 weist den Einschalttaster 501 auf, neben dem sich eine Hebelführung 502 und ein Tasteranschlag 512 befinden.

Eine Querschnittsdarstellung des Mundsteckstücks 401, das bereits aus den Figuren Figur 6 bis Figur 8 bekannt ist, ist in Figur 9 in perspektivischer Ansicht gezeigt. In dem Gehäuse 462, das als blockartiger Körper 458 realisiert ist, sitzt der Akkumulator 407 und die Steuerplatine 520. Die Steuerplatine 520 ist durch die Abschlussplatte 530 abgestützt ist. Die Steuerplatine 520 trägt auf jeder ihrer Langseiten, ungefähr im mittleren Bereich, jeweils eine Seiten-LED 589, 589^{I} zur Anzeige eines Betriebszustands des Mundsteckstücks 401 mittels blauem Licht, das durch das transluzente Fenster 593 nach außen fällt. Die Abschlussplatte 530 bildet einen Schutz für die Steuerplatine 520 gegen Feuchtigkeit bzw. erhöhte Kondensatsättigung in der Luft und auch gegen mechanische Angriffe. Der Mundschutz 468 weist einen Überwurf auf, der über einen Rand des blockartigen Körpers 458 darüber gestülpt werden kann. Mit Hilfe einer Kippnase 534^{II} wird der Mundschutz 468 mit dem blockartigen Körper 458 zusammengehalten. Die Kippnase 534^{II} ist an einer Dehnungsmulde 473 manuell rückziehbar. Der Mundschutz 468 ist als ein Lippenanschlusskörper 480 ausgeformt. Von dem Lippenanschlusskörper 480 weg erstreckt sich der Mundhöhlenkörper 402 als bogenförmiger Stiel 478, der mehrere Bögen aufweist, wie einen ersten Bogen 490, einen zweiten Bogen 491 und einen dritten Bogen 492. Im Inneren 540 des Mundhöhlenkörpers 402 erstrecken sich der LED-Träger 550 mit dem LED-Band 560, wobei der LED-Träger 550 dem Verlauf der Stielbögen 490, 491, 492 folgt. Licht, das von der Laser-LED 579 ausgeht, fällt auf die Mikrolinsenoptik 583, durch die das Licht auf tieferliegende Bereiche des Rachens (vgl. Figur 10) gerichtet wird. Das LED-Band 560 wird über die LED-Stromversorgungskontakte 566 mit für die LEDs geeigneter Versorgungsspannung versorgt. Alle elektronischen Komponenten des Mundsteckstücks 401 sind von einer Kunststoffhülle 482 eingeschlossen, damit kein Strom über den Körper einer das Mundsteckstück 401 nutzenden Person fließen kann. Die Kunststoffhülle 482 ist eine elektrisch isolierende Kunststoffhülle, die aus einem körperverträglichen, formadaptiven Kunststoff, wie einem Latex oder einem Silikon, besteht.

Die Steuerplatine 520 kann mehrere Steuerfunktionen umsetzen, die von einem Steuerprozessor geleistet werden. Der Steuerprozessor arbeitet mit einem auf der Steuerplatine 520 angeordneten Datenspeicher und mit einer auf der Steuerplatine befindlichen USB-Schnittstelle (nicht dargestellt) zusammen. Die Steuerplatine 520 überwacht den Ladezustand des Akkumulators 407 sowie Lebensdaten bzw. Gesundheitsdaten des Akkumulators 407. Wenn ein Ladestromkabel angeschlossen ist (siehe die in Figur 7 gezeigte USB-Buchse 505), regelt die Steuerplatine 520 den Ladestrom. Die Steuerplatine 520 kontrolliert die Zufuhr von elektrischem Strom zu den LEDs 571, 572, 573, 577, 578, 579 des Mundsteckstücks 401.

Werden die Figuren Figur 6, Figur 7, Figur 8 und Figur 9 nun gemeinsam betrachtet, so ist zu sehen, wie die Gruppen von LEDs, wie eine Gruppe von Blaulicht-LEDs 571, 572, 573 und eine Gruppe von UV-C-LEDs 577, 578, durch die Steuerplatine 520 unabhängig voneinander mit Strom versorgbar sind. Die Steuerplatine 520 umfasst insbesondere eine Zeitsteuerung 409. Mit der Zeitsteuerung 409 können Blaulicht-LEDs 571, 572, 573 sowie UV-C-LEDs 577, 578 bzw. die Laser-LED 579 jeweils für eine unterschiedliche Zeitdauer eingeschaltet werden. Über die Zeitsteuerung 409 kann eine Zeitsperre gesetzt werden. Die Zeitsperre verhindert, dass an eine erste Bestrahlungsdauer eine zweite Bestrahlungsdauer ohne Einhaltung einer vorgegebenen Wartezeit angeschlossen wird. Die Steuerplatine 520 empfängt aktuelle Temperaturmessdaten von dem Temperatursensor 581. Wenn ein empfangener Temperaturmesswert eine in dem Steuerprozessor gesetzte Maximaltemperatur überschreitet, wird der Betrieb des Mundsteckstücks 401 durch die Zeitsteuerung 409 unterbrochen. Die Steuerplatine 520 bzw. der darauf enthaltene Steuerprozessor ist über den USB-Anschluss 505 mit den erforderlichen Daten bzw. Betriebsparametern programmierbar. Beispielsweise kann ein Behandlungsplan in den Steuerprozessor geladen werden, durch den unterschiedliche Schaltdauern für die Zeitsteuerung 409 vorgegeben werden. Die Steuerplatine 520 erfasst anhand einer Kennung, welche Art und/oder welche Größe eines Mundhöhlenkörpers, wie der Mundhöhlenkörper 402, angeschlossen ist. Anschlussdaten sowie Betriebsdaten, insbesondere Temperaturdaten, oder auch Akkumulatordaten können über den USB-Anschluss 505, z. B. durch ein Abfragegerät, wie ein Laptop, ausgelesen werden. Auf dem mobilen Abfragegerät (nicht dargestellt) kann eine Software installiert sein, die es erlaubt, einen Therapieplan zu erstellen und auf das Mundsteckstück 401 zu übertragen. Damit lässt sich der Betrieb des Mundsteckstücks 401 besonders gut überwachen. Insbesondere ist ein Therapieplan auf eine aktuell gemessene Körpertemperatur abstimmbar.

Figur 10 skizziert die anatomischen Begebenheiten im Bereich einer Mundhöhle 20^{I} einer Person, wobei die Person in ihrer Mundhöhle 20^{I} ein Mundsteckstücks 601 trägt. Das in Figur 10 gezeigte Mundsteckstück 601 ist eine weitere Variante zu dem Mundsteckstück 1 nach Figur 1, das, wie das Mundsteckstück 1 in Figur 1 es auch hat, einen knopfartigen Körper 611 aufweist. An dem knopfartigen Körper 611 des Mundsteckstücks 601 befindet sich ein Einschalttaster 701. Nach Betätigung des Einschalttasters 701 wird eine weitere Übernahme eines elektrischen Schaltimpulses von dem Einschalttaster 701 während der Dauer eines voreingestellten Zeitintervalls, d. h. während der Dauer eines zweiten Zeitintervalls, ignoriert, gesperrt oder verriegelt.

Durch einen transluzenten Körper 676 des Mundsteckstücks 601 hindurch wird für die Dauer eines ersten Zeitintervalls, das kürzer als das zweite Zeitintervall ist, Licht abgestrahlt.

Wie anhand von Figur 10 zu sehen ist, liegt eine Oberlippe 23^{I} mit dem Amorbogen 23^{II} an einer Auflage 605 eines Mundschutzes 668 des Mundsteckstücks 601 an. Der Mundschutz 668 verhindert somit, dass ein restlicher Teil des Mundsteckstücks 601, der außerhalb des Mundes verbleiben soll, an den Lippen 23^{I}, 24^{I} bzw. an den Schneidezähnen 21^{I}, 22' vorbei in den Rachen 42 hinein gelangen kann. In Figur 10 sind der Oberkiefer 38 und der Unterkiefer 40 leicht geöffnet dargestellt. Bei einem geöffneten Mund durch Beabstandung von Oberkiefer 38 und Unterkiefer 40 wird die Einführung des Mundsteckstücks 601 erleichtert. Durch Schließen von Unterkiefer 40 und Oberkiefer 38 gelangen die Schneidezähne 21^{I}, 22' in eine Position, in der die Schneidezähne 21^{I}, 22' auf dem transluzenten Körper 676 aufliegen. Ein anterodorsaler Bereich 32 und ein posterodorsaler Bereich 34 des Rückens der Zunge 25^{I} können an dem transluzenten Körper 676 zur Anlage kommen. Zwischen den unteren Schneidezähnen 22' und der Zunge 25^{I} befindet sich ein sublaminaler Bereich 36, der durch den transluzenten Körper 676 hindurch mit blauem Licht bestrahlbar ist. Von dem Mundhöhlenkörper 602 wird oberseitig durch den transluzenten Körper 676 hindurch der harte Gaumen 26^{I}, der weiche Gaumen 28 und das Gaumenzäpfen 30 bestrahlt. Außerdem wird von dem Mundhöhlenkörper 602 Licht in den Bereich des Rachens 42, insbesondere in Richtung des Kehldeckels 48 bzw. der Luftröhre 50 abgegeben. Während der Bestrahlung der Mundhöhle 20^{I} mit Licht aus dem Mundhöhlenkörper 602 kann die Person durch die Nasenhöhle 44 über den Nasenrachen 46 Luft, insbesondere durch Nasenhärchen gefiltert, einatmen. Ein Eintritt von Influenza-Viren in den Rachenbereich 42 ist erschwert, weil der Mundhöhlenkörper 602 den Eintritt behindert.

In einer nicht dargestellten Weiterbildung kann der Mundhöhlenkörper an seinem Ende zweigeteilt ausgeführt sein, um das in Figur 10 zu sehende Gaumenzäpfchen 30 seitlich zu passieren und besonders weit in den Rachen 42 des das Mundsteckstück nutzenden Menschen hineinzuleuchten.

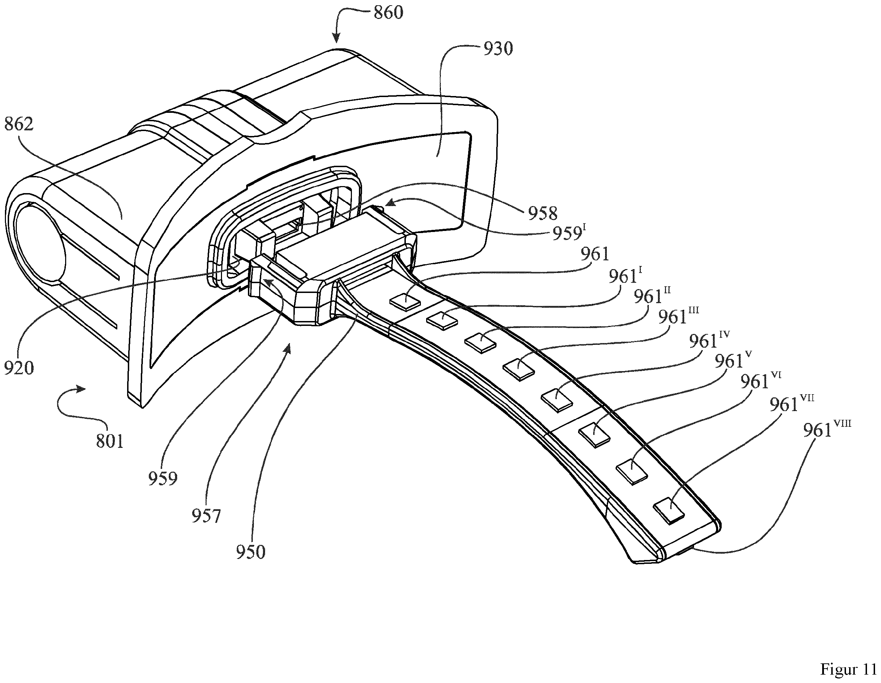

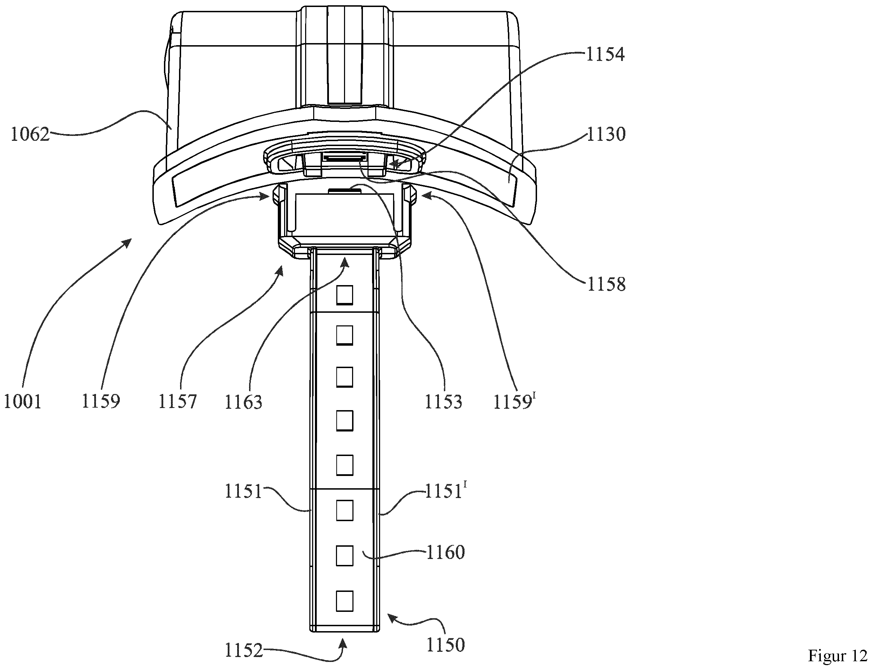

Die in den Figuren 11 und 12 gezeigten beiden Ausgestaltungsformen eines Bestrahlungssteckstücks 801, 1001 sind ohne eine abschließende Hülle, z. B. ohne die - in Figur 2 gezeigte- abschließende, die Innereien schützende Hülle 282, dargestellt, um die Anordnung der LEDs 961, 961^{I}, 961", 961^{III}, 961^{IV}, 961^{V}, 961^{VI}, 961^{VII}, 961^{VIII}, die durch die Hülle normalerweise geschützt sind, besser sehen zu können. Die in Figur 2 gezeigte Hülle 282 übernimmt in einer günstigen Ausgestaltung nicht nur die Funktion einer Schutzhülle, z. B. gegen Bisse oder gegen erhöhte Krafteinwirkungen, z. B. eines Schließmuskels, sondern auch die Funktion eines Diffusors, um das aus den LEDs, wie den LEDs 961, 961^{I}, 961", 961^{III}, 961^{IV}, 961^{V}, 961^{VI}, 961^{III}, 961^{VIII}, austretende, punktuelle Licht gleichmäßiger zu streuen.

Die in Figur 11 gezeigten LEDs 961, 961^{I}, 961", 961^{III}, 961^{IV}, 961^{V}, 961^{VI}, 961^{VII}, 961^{VIII} sind auf einem LED-Träger 950 befestigt. Der LED-Träger 950 unterstützt die flexible Platine, die die LEDs 961, 961^{I}, 961^{II}, 961^{III}, 961^{IV}, 961^{V}, 961^{VI}, 961^{VII}, 961^{VIII} über Leiterbahnen mit Strom versorgt. Der LED-Träger 950 mündet in einen Clipverbinder 957. An dem Clipverbinder 957 sind elektrische Kontakte (in der gezeigten Perspektive nicht zu sehen) vorhanden, die in einen Steckverbinder 958 einführbar sind. Der Steckverbinder 958 ist auf der Steuerplatte 920 angebracht; er ist Teil der Steuerplatine 920, die quer zum LED-Träger 950 im Gehäuse 862 liegt. Über den Steckverbinder 958 kann Strom zu allen LEDs 961, 961^{I}, 961", 961^{III}, 961^{IV}, 961^{V}, 961^{VI}, 961^{VII}, 961^{VIII} fließen, wenn seitliche Clips 959, 959^{I} an dem Clipverbinder 957 den LED-Träger 950 mit einem kastenartigen Korpus 860 zusammenhalten. Die zwei Clips 959, 959^{I} sind an einander gegenüberliegenden Seiten des Clipverbinders 957 so angeordnet, dass sie an einer Abschlussplatte 930 eines Gehäuses 862 einrasten können, wobei ein Halt durch zurückfederndes Verspreizen der zwei Clips 959, 959^{I} entsteht. Ein Benutzer (nicht dargestellt), der mit seinen Fingern einer Hand an den zwei Clips 959, 959^{I} des Clipverbinders 957 angreift, kann die zwei Clips 959, 959^{I} aufeinander zu zusammendrücken und den Clipverbinder 957 von der Abschlussplatte 930 abziehen.

Gleichartig zu dem in Figur 11 gezeigten Stecksystem können bei einer Ausführungsform in Übereinstimmung mit Figur 12 Steckkontakte 1153 an einem Clipverbinder 1157 entlang eines Kontaktführungspaars 1154 in den Steckverbinder 1158 eingeschoben werden. Der Clipverbinder 1157 wird dann durch Einrasten der Clips 1159, 1159^{I} mit der Endplatte bzw. Abschlussplatte 1130 gekoppelt. Die Endplatte bzw. Abschlussplatte 1130 ist flüssigkeitsdicht in ein Gehäuse 1062 des Bestrahlungssteckstücks 1001 eingeklebt. In den Clipverbinder 1157 ist ein LED-Träger 1150 eingesetzt. Der LED-Träger 1150 weist einen ersten Seitenschutz 1151 und einen zweiten Seitenschutz 1151^{I} auf, die eine auf dem LED-Träger 1150 angeordnete LED-Platine 1160, die auch als LED-Band 1160 bezeichnet werden kann, flankieren. Der Seitenschutz 1151, 1151^{I} bietet eine Fingergrifffläche. Außerdem bewahrt der Seitenschutz 1151, 1151^{I} die vertieft angeordnete LED-Platine 1160 mit deren auf ihr etwa äquidistant in einer Reihe angeordneten LEDs vor Verunreinigungen durch Fingerabdrücke. Eine Trägerrundung 1152 an dem LED-Träger 1150, d.h. an dessen Ende, ist als Endrundung zylindermantelabschnittartig ausgebildet.

Wird das Bestrahlungssteckstück 1001 aus seinen Einzelteilen zusammengebaut, so wird die LED-Platine 1160 um die Trägerrundung 1152 herum eng und knickfrei an den LED-Träger 1150 angelegt. Es kann also gesagt werden, dass die LED-Platine 1160 durch die Trägerrundung 1152 einen Wendebereich in ihrer Längserstreckung hat. Die LED-Platine 1160 folgt ihrer Trägerrundung 1152. Anschließend werden der Clipverbinder 1157 auf ein der Trägerrundung 1152 gegenüberliegendes Ende des LED-Trägers aufgesetzt und die Steckkontakte 1153 mit der LED-Platine 1160 verbunden. Zwei Endbereiche der einstrangigen LED-Platine 1160, wie der Platinenendbereich 1163, führen somit in den Clipverbinder 1157 hinein und somit zu den Steckkontakten 1153.

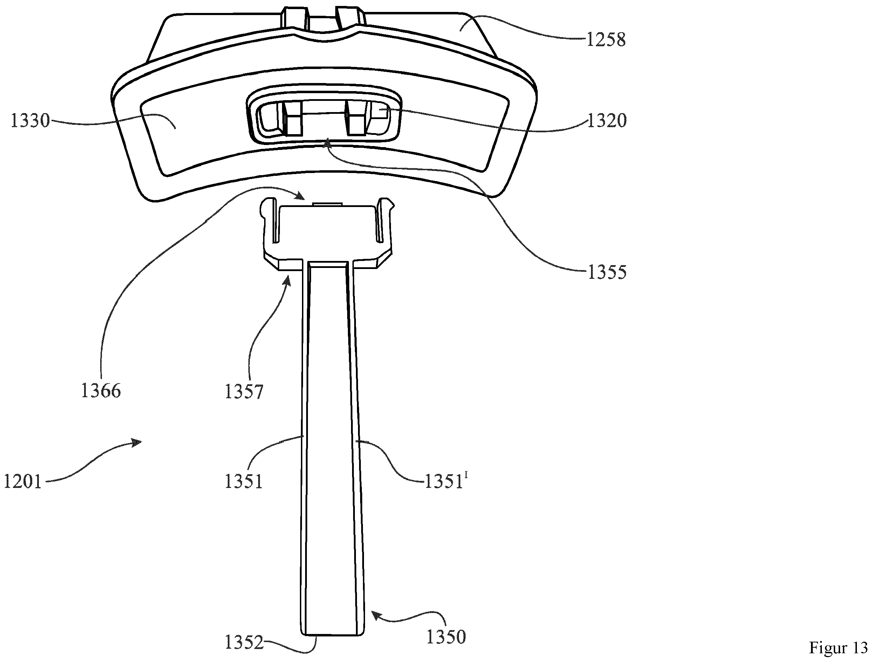

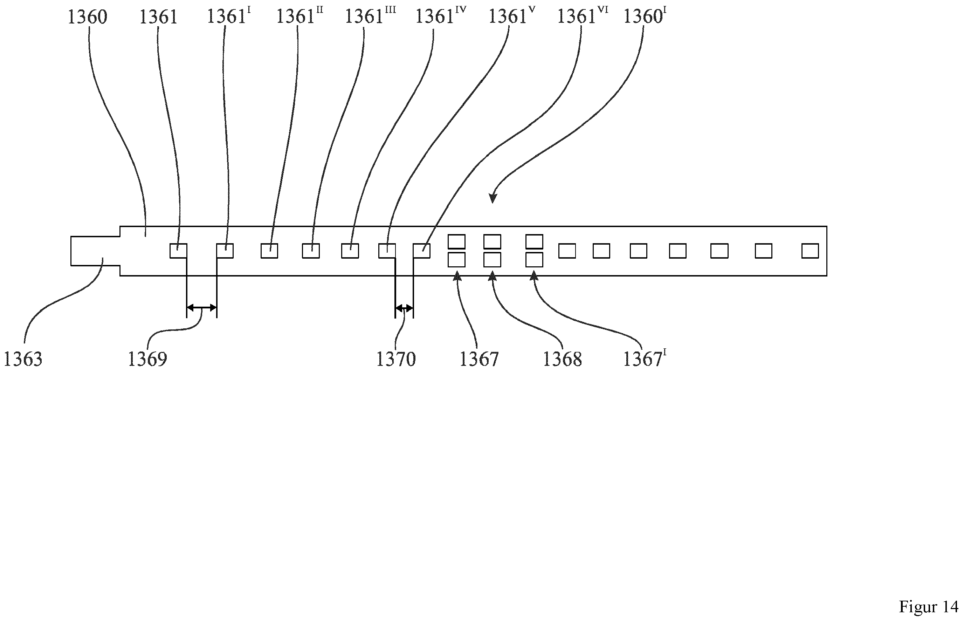

Die Figuren Figur 13 und Figur 14 stellen ein weiteres Ausführungsbeispiel für ein Bestrahlungssteckstücke vor, genauer anhand des Mundsteckstücks 1201. Fig. 13 und Fig. 14 zeigen Komponenten eines Mundsteckstücks 1201, wobei zu einer besseren Einsehbarkeit innenliegender Komponenten ein transluzenter Körper, wie der in Fig. 6 gezeigte transluzente Körper 476, weggelassen bzw. abgezogen wurde. Der transluzente Körper kann zur seiner Sterilisation von einem blockartigen Korpus 1258 des Mundsteckstücks 1201 abgenommen werden. Natürlich sind auch Anwendungsfälle vorstellbar, in denen es reicht, den transluzenten Körper 476 einfach nur abzuwaschen und nicht auch noch zu sterilisieren. Das Mundsteckstück 1201 umfasst einen LED-Träger 1350, der in einer Trägerrundung 1352 endet. Ein erster Seitenschutz 1351 und ein zweiter Seitenschutz 1351^{I}, die sich an dem LED-Träger 1350 erstrecken, bilden eine Begrenzung einer rinnenartigen Vertiefung des LED-Trägers 1350. Zwischen dem ersten Seitenschutz 1351 und dem zweiten Seitenschutz 1351^{I} ist eine in Fig. 14 gezeigte LED-Platine 1360 einlegbar. Der erste Seitenschutz 1351 und der zweite Seitenschutz 1351^{I} erstrecken sich von einem Clipverbinder 1357 des LED-Trägers 1350 bis zu der Trägerrundung 1352. Die in Fig. 14 gezeigte LED-Platine 1360 wird während der Herstellung des Mundsteckstücks 1201 auf den LED-Träger 1350, der in Fig. 13 zu sehen ist, aufgeklebt. Ein Platinenendbereich 1363, der in Fig. 14 gezeigt ist, ist bis zu einem Kontaktfeld 1366 des Clipverbinders 1357 in dem LED-Träger 1350 einfädelbar. Das in Fig. 13 gezeigte Kontaktfeld 1366 kann in eine Einstecköffnung 1355 eines blockartigen Korpus 1258 eingeführt werden. Die Einstecköffnung 1355 ist in einer Abschlussplatte 1330 des blockartigen Korpus 1258 vorhanden. Die Abschlussplatte 1330 trägt in dem blockartigen Korpus 1258 innenliegend zugleich alle Komponenten, wie eine Steuerplatine 1320, die von einem ebenfalls innenliegenden, und daher in Figur 13 nicht sichtbaren Akkumulator mit elektrischem Strom versorgt wird. In entsprechender Weise kann z. B. der LED-Träger 1350, dessen LED-Platine 1360 defekt ist, herausgenommen und ersetzt werden.

In einem Reinigungsverfahren kann ein transluzenter Körper, wie der in Fig. 6 gezeigte transluzente Körper 476, von Schmutz befreit werden. Für ca. 30 Sekunden kann der transluzente Körper in eine Desinfektionslösung, wie einen mehr als siebzigvolumenprozentigen Alkohol, ggf. mit einer Beimischung von Wasserstoffperoxid, eingetaucht werden. Alternativ kann der transluzente Körper in einer wässrigen Seifenlösung ausgekocht und anschließend mit gereinigtem Wasser gespült werden. Nach dem Trocknen kann der transluzente Körper wieder über den LED-Träger 1350 gezogen werden. Damit sind insbesondere Speichelreste oder sonstige Körperausscheidungen von dem transluzenten Körper entfernbar.

Die LED-Platine 1360, die in Fig. 14 gezeigt ist, ist biegsam. Auf der LED-Platine 1360 befinden sich mehrere vereinzelte LEDs 1361, 1361^{I}, 1361^{II}, 1361^{III}, 1361^{IV}, 1361^{V}, 1361^{VI}. Auf die Reihe von Einzel-LEDs 1361, 1361^{I}, 1361", 1361^{III}, 1361^{IV}, 1361^{V}, 1361^{VI} folgen in einem Platinenmittenbereich 1360 drei LED-Paare 1367, 1368, 1367^{I}, denen auf der LED-Platine 1360 wieder eine Reihe von Einzel-LEDs folgt, ähnlich zu den links aufgereihten LEDs 1361 bis 1361^{VI}. Ein LED-Paar 1367, 1367^{I}, 1368 umfasst zwei einzelne LEDs, die in einem nächstmöglichen Abstand in einer Querrichtung zu der Einzel-LED-Reihung auf der LED-Platine 1360 angeordnet sind. Somit sind auf der LED-Platine 1360 genau 20 LEDs befestigt und über Leiterbahnen (nicht dargestellt) mit elektrischem Strom versorgbar. Auch sind aus Übersichtsgründen solche Bauteile wie Widerstände weggelassen worden. Ein mittleres LED-Paar 1367 der drei LED-Paare 1367, 1368, 1367^{I} wird auch als Rundungs-LED-Paar 1368 bezeichnet, weil dieses LED-Paar 1368 in einem Platinenmittenbereich 1360^{I} bei der Montage der LED-Platine 1360 auf dem LED-Träger 1350 (siehe Fig. 13) der Rundung 1352 nächst kommend angeordnet wird. Durch die Anordnung der Doppel-LEDs 1367, 1368, 1367^{I} in der Nähe der Trägerrundung 1352 ist von dem Bereich der Trägerrundung 1352 eine besonders hohe Lichtstärke abstrahlbar. Die LEDs der LED-Paare 1367, 1367 sind näher zueinander angeordnet als die Einzel-LEDs 1361 bis 1361^{VI}. Zwischen einer ersten LED 1361 auf der LED-Platine 1360, die sich in einer Nähe zum Platinenendbereich 1363 befindet, und einer benachbarten, zweiten LED 1361^{I} gibt es einen Abstand 1369. Zwischen einer sechsten LED 1361^{V} und einer siebten LED 1361^{VI} existiert ein zweiter Abstand 1370, der kleiner ist als der erste Abstand 1369. Der erste Abstand beträgt 10 mm (Millimeter). Der zweite Abstand liegt bei 3 mm. Durch Wahl der Abstände 1369, 1370 lässt sich eine von den LEDs 1361 bis 1361^{VII} entlang des Mundsteckstücks 1201 bereitstellbare Leuchtstärke bzw. Lichtintensität vorgeben.

Beispielsweise sind auch solche Ausführungsformen möglich, bei denen der erste Abstand zwischen 5 mm und 12 mm groß ist und der zweite Abstand zwischen 3 mm und 6 mm groß ist. Anders gesagt ist es vorteilhaft, wenn in einer Mundhöhle (vgl. Mundhöhle 20^{I} in Fig. 10) im Bereich des Rachens 42 eine höhere Lichtintensität abgegeben wird als in einen Bereich der Schneidezähne 21^{I}, 22^{I}.

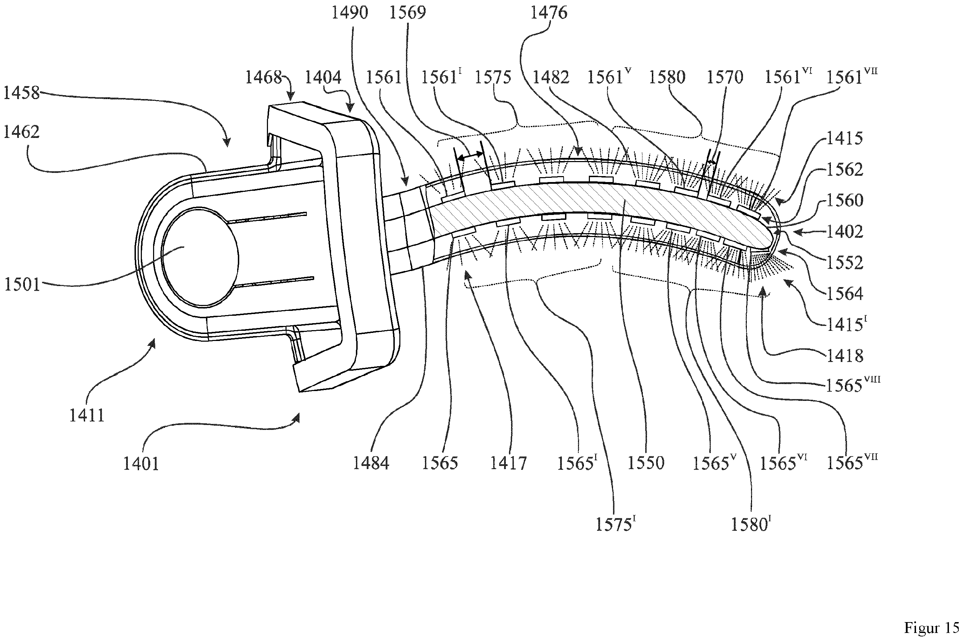

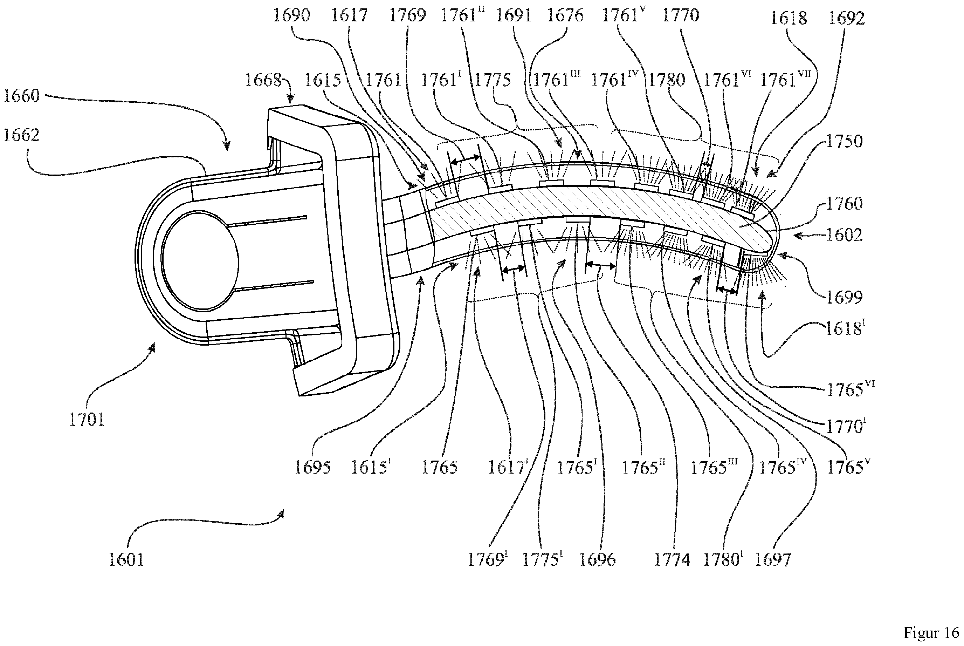

Figuren 15 und 16 zeigen zwei weitere Ausführungsbeispiele für Mundsteckstücke 1401, 1601.

Das Mundsteckstück 1401, das in Figur 15 gezeigt ist, hat einen ersten Bereich 1575, 1575^{I}, in dem der Mundhöhlenkörper 1402 mit einer ersten, d. h. niedrigeren Lichtdichte leuchtet und einen zweiten Bereich 1580, 1580^{I}, in dem der Mundhöhlenkörper 1402 mit einer zweiten, d. h. höheren Lichtdichte leuchtet. Die Helligkeit der LEDs 1561, 1561^{I}, 1561^{V}, 1561^{VI}, 1561^{VII}, 1565, 1565^{I}, 1565^{V}, 1565^{VI}, 1565^{VII}, 1565^{VIII} wird durch den elektrischen Strom bestimmt, der von der (nicht sichtbaren) Steuerplatine in dem blockartigen Korpus 1458 für jede LED 1561, 1561^{I}, 1561^{V}, 1561^{VI}, 1561^{VII}, 1565, 1565^{I}, 1565^{V}, 1565^{VI}, 1565^{VII}, 1565^{VIII} individuell eingestellt wird.

Ein erster Teil des Mundsteckstücks 1401 wird durch den knopfartigen Korpus 1411 gebildet. Es kann zudem gesagt werden, der Korpus 1411 ist ein blockartiger Korpus 1458, in dem eine Steuerungselektronik und ein elektrischer Speicher aufbewahrt sind. Dieses Gehäuse 1462, das durch den blockartigen Korpus 1458 gebildet ist, ist als Griff gestaltet. Das Gehäuse 1462 hat an einer Seite einen Einschalttaster 1501.

Der zweite Teil des Mundsteckstücks 1401 wird durch den Mundhöhlenkörper 1402 gebildet. Zwischen dem Mundhöhlenkörper 1402 und dem Gehäuse 1462 gibt es eine trennende Wand, über die ein Mundschutz 1468 ausgebildet ist. Eine Seite des Mundschutzes 1468 ist als Anlagefläche 1404 gestaltet, sodass gegen die Anlagefläche 1404 Lippen und/oder Mundpartien eines Nutzers des Mundsteckstücks 1401 anliegen können. In dem Mundhöhlenkörper 1402 sind die LEDs, wie die LEDs 1561, 1561', 1561^{V}, 1561^{VI}, 1561^{VII}, 1565, 1565^{I}, 1565^{V}, 1565^{VI}, 1565^{VII}, 1565^{VIII}, angeordnet. Aus diesem Grund ist der Mundhöhlenkörper 1402 in den beiden Bereichen 1575, 1575^{I} und 1580, 1580^{I} geschnitten dargestellt. Unmittelbar an die Anlagefläche 1404 schließt sich der erste Stielbogen 1490 an. In dem Bereich des ersten Stielbogens 1490 startet der transluzente Körper 1476, durch den die LEDs 1561, 1561^{I}, 1561^{V}, 1561^{VI}, 1561^{VII}, 1565, 1565^{I}, 1565^{V}, 1565^{VI}, 1565^{VII}, 1565^{VIII} ihr Licht 1415, 1415^{I} abgeben können. Somit gibt es ein endnahes Licht 1418 und ein mundschutznahes Licht 1417.

Der transluzente Körper 1476 ist durch die Kunststoffhülle 1482 gebildet, die Teil des Mundhöhlenkörpers 1402 ist. Im Inneren des Mundhöhlenkörpers 1402 gibt es den Lichtquellenträger, der als LED-Träger 1550 ausgestaltet ist. Der LED-Träger 1550 ist zur Aufnahme eines ersten LED-Strangs 1562 und eines zweiten LED-Strangs 1564 gestaltet. Innerhalb eines Strangs 1562, 1564 mit LEDs sind die LEDs 1561, 1561', 1561^{V}, 1561^{VI}, 1561^{VII}, 1565, 1565^{I}, 1565^{V}, 1565^{VI}, 1565^{VII}, 1565^{VIII} nicht alle gleichmäßig beabstandet. Zwischen LEDs, die im ersten Bereich 1575 des Mundhöhlenkörpers 1402 angesiedelt sind, gibt es einen ersten LED-Abstand 1569, der größer ist als ein zweiter LED-Abstand 1570, der im zweiten Bereich 1580, 1580^{I} vorzufinden ist. Der größere LED-Abstand 1569 beträgt 8 mm. Der kleine LED-Abstand 1570 beträgt weniger als die Hälfte, genauer gesagt 3 mm. Mehrere LEDs, wie z. B. die LEDs 1561^{V}, 1561^{VI}, 1561^{VII}, sind untereinander gleich beabstandet mit einem LED-Abstand 1570. Hiervon weicht der Abstand der LEDs 1561, 1561^{I} im Abschnitt unmittelbar vor der Anlagefläche 1404 ab. In dem sich oben befindlichen Bereich 1575, der auch als erster Bereich 1575 zu bezeichnen ist, und in dem sich unterhalb erstreckenden Bereich 1575^{I} sind die LEDs (z. B. LED 1561, 1561^{I}) alle gleich beabstandet. Auch die Abstände 1570 in dem zweiten Bereich 1580, 1580^{I} sind alle gleich.

Besonders vorteilhaft ist, wenn die LEDs 1561, 1561', 1561^{V}, 1561^{VI}, 1561^{VII} als LED-Band 1560 auf dem LED-Träger 1550 angeordnet sind. Der LED-Träger 1550 ist somit die Unterstützung für den ersten LED-Strang 1562 und die Halterung bzw. Unterstützung für den zweiten LED-Strang 1564. Der LED-Träger 1550 ist in seinem Endbereich mit einer Trägerrundung 1552 ausgestattet, sodass ein zusammenhängendes LED-Band 1560 auf dem LED-Träger 1550 beidseitig, genauer gesagt oben und unten, aufgelegt werden kann. Das LED-Band 1560 startet im ersten Stielbogen 1490 und läuft entlang des gesamten LED-Trägers 1550 über die Trägerrundung 1552 bis zur Zahnstützfläche 1484, die sich in Nachbarschaft zur Anlagefläche 1404 befindet.

Wird über den Einschaltaster 1501, der nur nach Ablauf einer Sperrzeit erneut betätigt werden kann, das Mundsteckstück 1401 eingeschaltet und leuchten die LEDs 1561, 1561^{I}, 1561^{V}, 1561^{VI}, 1561^{VII}, 1565, 1565^{I}, 1565^{V}, 1565^{VI}, 1565^{VII}, 1565^{VIII} im Bereich des Mundhöhlenkörpers 1402 durch die Kunststoffhülle 1482 hindurch, so stellt ein Betrachter fest, dass in dem zweiten Bereich 1580, 1580^{I} eine höhere Helligkeit abgegeben wird als in einem ersten Bereich 1575, 1575^{I}. Die LEDs 1561, 1561', 1561^{V}, 1561^{VI}, 1561^{VII}, 1565, 1565^{I}, 1565^{V}, 1565^{VI}, 1565^{VII}, 1565^{VIII} können aufgrund ihrer unterschiedlichen Ansteuerströme unterschiedlich hell leuchten.

Außerdem sind in dem zweiten Abschnitt 1580, 1580^{I} mehr LEDs 1561^{V}, 1561^{VI}, 1561^{VII}, 1565^{V}, 1565^{VI}, 1565^{VII} angesiedelt. Beide Maßnahmen tragen dazu bei, dass im Bereich des Mundsteckstücks 1401, der für die Rachenbeleuchtung vorgesehen ist, ein stärkeres Licht leuchtet als im lippennahen Bereich. Die Lippen und die Zähne sollen im Bereich der Zahnstützfläche 1484 aufliegen. Im Bereich der Zahnstützfläche 1484 reicht eine geringere Beleuchtung im Vergleich zum Rachennahenbereich bzw. dem endnahen LED-Lichtbereich 1418 aus.

An zwei Seiten des LED-Trägers 1550 sind LED-Stränge 1562, 1564 vorhanden. Die Abstände 1569, 1570 zwischen den LEDs 1561, 1561', 1561^{V}, 1561^{VI}, 1561^{VII}, 1565, 1565^{I}, 1565^{V}, 1565^{VI}, 1565^{VII}, 1565^{VIII} sind zwischen dem ersten LED-Strang 1562 und dem zweiten LED-Strang 1564 gleich verteilt. Zwischen den LEDs 1561, 1561', 1565, 1565^{I}, die im Bereich der Bissfläche 1484 liegen, ist ein größerer Abstand 1569 als zwischen den LEDs 1561^{V}, 1561^{VI}, 1561^{VII}, 1565^{V}, 1565^{VI}, 1565^{VI}, die zur Ausleuchtung des Endnahenbereichs 1418 vorgesehen sind.

Durch die Versorgung mit verschiedenen Strömen, die an die LEDs 1561, 1561', 1561^{V}, 1561^{VI}, 1561^{VII}, 1565, 1565^{I}, 1565^{V}, 1565^{VI}, 1565^{VII}, 1565^{VIII} geliefert werden, können unterschiedliche Helligkeiten in unterschiedlichen Bereichen 1575, 1575^{I}, 1580, 1580^{I} erzeugt werden. Zu LEDs, wie die LEDs 1561^{V}, 1561^{VI}, 1561^{VII}, die heller leuchten sollen, kann ein Versorgungsstrom von 40 mA fließen. LEDs, die deutlich schwächer leuchten sollen, wie die LEDs 1561, 1561^{I} in Nachbarschaft zur Anlagefläche 1404, können mit Strom in der Größenordnung von 5 mA betrieben werden.

So ist es möglich, zielgerichtet Viren, Bakterien und Pilze abzutöten, insbesondere im Rachenbereich eines Nutzers, während mit deutlich niedrigeren Dosen in anderen Mundbereichen des Nutzers eine Bestrahlung durchgeführt wird.

Das Mundsteckstück 1601, das in der Figur 16 dargestellt ist, ähnelt in weiten Teilen dem Mundsteckstück 1401, das aus Figur 15 bekannt ist. Somit können die weiter oben stehenden Beschreibungen auf das Mundsteckstück 1601 gem. Figur 16 übertragen werden. Das Mundsteckstück 1601 gem. Figur 16 unterscheidet sich aber in Bezug auf die Platzierung der LEDs 1761, 1761^{I}, 1761^{II}, 1761^{III}, 1761^{IV}, 1761^{V}, 1761^{VI}, 1761^{VII}, 1765, 1765^{I}, 1765^{II}, 1765^{III}, 1765^{IV}, 1765^{V}, 1765^{VI} auf dem LED-Träger 1750.

Eine erste LED 1761, die nahe eines ersten Stielbogens 1690 angeordnet ist, weist einen ersten Abstand 1769 zu einer zweiten LED 1761^{I} auf. Der zweiten LED 1761^{I} folgen eine dritte LED 1761", eine vierte LED 1761^{III} eine fünfte LED 1761^{IV}, eine sechste LED 1761^{V} entlang eines zweiten Stielbogens 1691. Entlang eines dritten Stielbogens 1692 befinden sich eine siebte LED 1761^{VI} und eine achte LED 1761^{VII}. Zwischen der sechsten LED 1761^{V} und der siebten LED 1761^{VI} ist ein zweiter Abstand 1770 vorhanden. Der zweite Abstand 1770 beträgt ein Drittel einer Länge des ersten Abstands 1769. Von der ersten LED 1761 wird ein mundschutznahes Licht 1617 abgestrahlt. Von der achten LED 1761^{VII} wird ein endnahes Licht 1618 ausgesendet, das heller ist als das mundschutznahe LED-Licht 1617, weil die achte LED 1761^{VII} mit einem ca. 10 % höheren Strom versorgt wird als die erste LED 1761. Auf dem transluzenten Körper 1676 des Mundhöhlenkörpers 1602 sind ein dunklerer Bereich 1775 und ein hellerer Bereich 1780 unterscheidbar. Somit wird gaumenseitig entlang der Stielbögen 1690, 1691, 1692 ein gaumenseitiges Licht 1615 abgestrahlt. Die LED-Platine 1760 ist um einen LED-Träger 1750 herum gelegt. Von zungenseitigen LEDs 1765, 1765^{I}, 1765", 1765^{III}, 1765^{IV}, 1765^{V}, 1765^{VI} wird zungenseitig Licht 1615^{I} abgestrahlt. Eine erste zungenseitige LED 1765 ist gegenüber der ersten gaumenseitigen LED 1761 und der zweiten gaumenseitigen LED 1761^{I} auf Lücke angeordnet, also deren Abstandbereich 1769 zugeordnet. Zwischen der ersten zungenseitigen LED 1765 und der zweiten zungenseitigen LED 1765^{I} ist ein Abstand 1769^{I} vorhanden. Zwischen der dritten zungenseitigen LED 1765" und der vierten zungenseitigen LED 1765^{III} ist ein Abstand 1774 vorhanden. Zwischen der sechsten zungenseitigen LED 1765^{V} und der siebten zungenseitigen LED 1765^{VI} ist ein Abstand 1770^{I} vorhanden. Der erste Abstand 1769^{I} und der dritte Abstand 1770^{I} sind etwa gleich groß. Der zweite Abstand 1774 ist um ein Drittel größer als der erste Abstand 1769^{I}, weil ein Stielmittenbogen 1696 des Mundhöhlenkörpers 1602 eine konkave Krümmung aufweist, durch die sich Abstrahlungsbereiche der dritten LED 1765^{I} und der vierten LED 1765" überschneiden. Wie bei dem gaumenseitigen Stielansatzbogen 1690 ist auch bei dem zungenseitigen Stielansatzbogen 1695 ein schwächeres Licht 1617^{I} aus der ersten, zungenseitigen LED 1765 zur Behandlung ausreichend. Von der siebten LED 1765^{VI} wird ein im Vergleich zu dem Licht 1617^{I} stärkeres bzw. helleres Licht 1618^{I} durch erhöhte Stromzufuhr zu der siebten LED 1765^{VI} abgestrahlt, sodass von einem mundhöhlenseitigen Ende 1699 des Mundhöhlenkörpers 1602 ein Eingangsbereich zum Rachen ausreichend mit Licht bestrahlt wird. Anders gesagt, liegt an dem zungenseitigen Stielendbogen 1697 ein zweiter Helligkeitsbereich 1780^{I} vor, zu dem im Vergleich ein erster Helligkeitsbereich 1775^{I}, der dem zungenseitigen Stielansatzbogen 1695 zugeordnet ist, dunkler erscheint. Das von einer LED 1761, 1761', 1761^{II}, 1761^{III} 1761^{IV}, 1761^{V}, 1761^{VI}, 1761^{VII} sowie 1765, 1765^{I}, 1765", 1765^{III}, 1765^{IV}, 1765^{V} 1765^{VI} jeweils abgegebene Licht, wie das Licht 1617, 1617', 1618, 1618^{I}, ist jeweils durch Stromzufuhr über eine Steuerplatine, die sich im inneren eines kastenförmigen Korpus 1660 befindet, festgelegt. Der kastenförmige Korpus 1660 weist ein Gehäuse 1662 auf, das an einen Mundschutz 1668 angeschlossen ist. Von dem Mundschutz 1668 bis zum siebten Stielbogen 1699 erstreckt sich der Mundhöhlenkörper 1602, der den LED-Träger 1750 enthält. Ein Einschalttaster 1701 an dem Gehäuse 1662 dient dazu, die fünfzehn LEDs 1761, 1761^{I}, 1761", 1761^{III}, 1761^{IV}, 1761^{V}, 1761^{VI}, 1761^{VII} sowie 1765, 1765^{I}, 1765", 1765^{III}, 1765^{IV}, 1765^{V} 1765^{VI} des Mundsteckstücks 1601, also acht gaumenseitige und sieben zungenseitige, gemeinsam ein- bzw. auszuschalten.

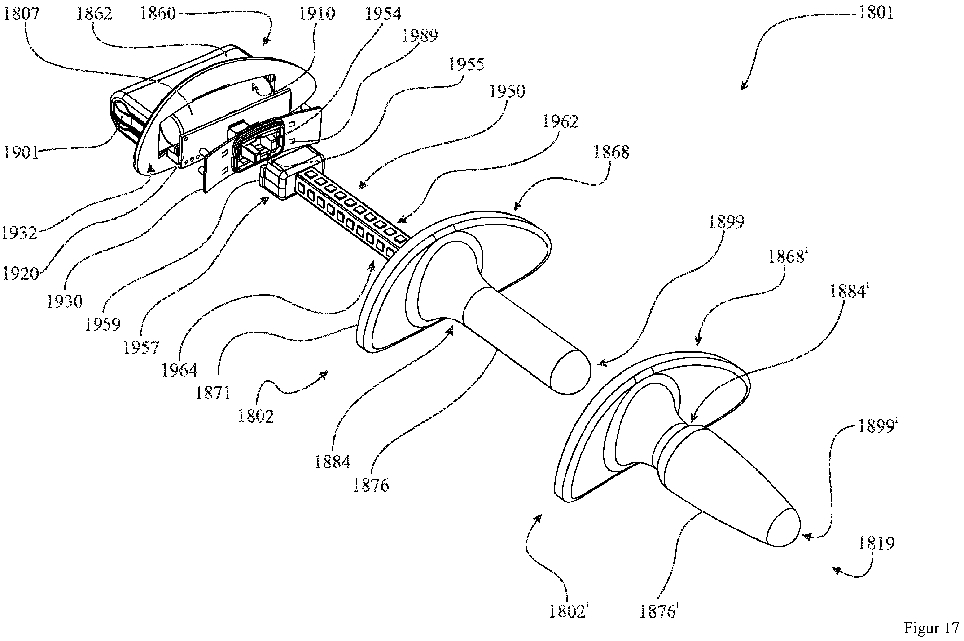

Figur 17 zeigt ein Bestrahlungssteckstück 1801, dessen einzelnen Komponenten separiert voneinander gezeichnet sind.

Das Bestrahlungssteckstück 1801 umfasst einen kastenartigen Korpus 1860, eine Steuerplatine 1920, eine Abschlussplatte 1930 und einen LED-Träger 1950, wobei der LED-Träger 1950 durch wahlweises Aufziehen einer ersten transluzenten Hülle 1876 ein Einführteil 1802 als Vaginaleinführteil oder durch Aufziehen einer zweiten transluzenten Hülle 1876^{I} ein Einführteil 1802^{I} als Analeinführteil gebildet werden kann. Die Einführteile 1802, 1802^{I} bilden ein Set 1819. Zu jedem der Einführteile 1802, 1802^{I} gehört eine eigene transluzente Hülle 1876, 1876^{I}. Zwischen einem Ende 1899, 1899^{I} des jeweiligen Einführteils 1802, 1802^{I} und dessen gegenüberliegenden Körperöffnungsschutz 1868, 1868^{I} befindet sich ein Einklemmbereich 1884, 1884^{I}, an dem das Einführteil 1802 bzw. 1802^{I} mithilfe von Körpermuskeln in einer Körperhöhle einer (nicht dargestellten) Person gehalten werden kann. Auf dem LED-Träger 1950 sind vier LED-Stränge, wie ein erster LED-Strang 1962 und ein zweiter LED-Strang 1964, angeordnet, wodurch eine Abstrahlung von Licht in vier Raumrichtungen, d. h. tetradirektional ermöglicht wird. An einem Ende geht der LED-Träger 1950 in einen Clipverbinder 1957 über, der mit mehreren seitlichen Clips wie dem seitlichen Clip 1959 in einer Einstecköffnung 1955 der Abschlussplatte 1930 einführbar und dort einrastbar ist. Das Einführen und Kontaktieren wird durch eine Kontaktführung 1954 erleichtert, wobei die Kontaktierung der LED-Stränge 1962, 1964 sowie der beiden weiteren, in der dargestellten Perspektiv nicht sichtbaren LED-Stränge an der Steuerplatine 1920 erfolgt. An der Abschlussplatte 1930 sind vier Betriebszustands-LEDs, wie die LED 1989, angeordnet, die ebenfalls über die Steuerplatine 1920 versorgt werden. Licht, das von den Betriebszustands-LEDs (z. B. der LED 1989) abgestrahlt wird, wird von dem Körperöffnungsschutz 1868 in Richtung auf eine Anschlusslippe 1871 zurückgeworfen, und kann durch diese hindurch aus dem Gehäuse 1862 austreten. An der Anschlusslippe 1871 erstreckt sich eine Dichtfläche 1932, auf der der Körperöffnungsschutz 1868 flüssigkeitsdicht zur Auflage gebracht ist, wobei der Körperöffnungsschutz 1868 an der Anschlusslippe 1871 überlappend an dem Gehäuse 1862 anschließt. Das Gehäuse 1862 umfasst einen Einschalttaster 1901, über den, wenn das Bestrahlungssteckstücks 1801 zusammengebaut ist, der Steuerplatine 1920 die Aufforderung mitgeteilt werden kann, Strom von einem Akkumulator 1807, der im Batteriefach 1910 montiert ist, den LED-Strängen, wie den Strängen 1962, 1964, zuzuführen.

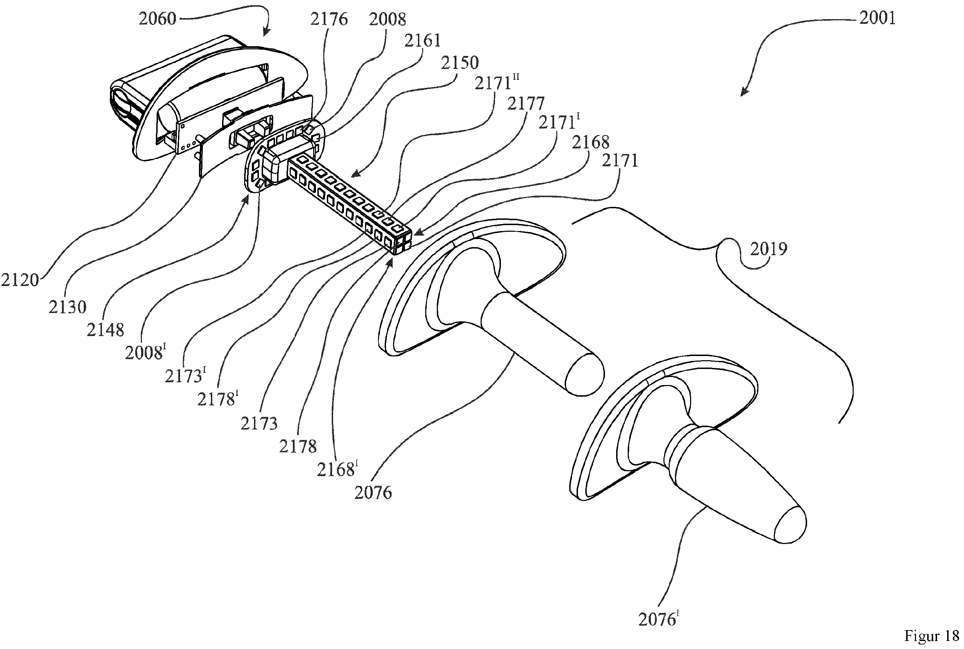

In Figur 18 ist ein weiteres Beispiel für ein Bestrahlungssteckstück 2001 gezeigt, das ein Set 2019 umfasst. Das Set 2019 enthält zwei unterschiedliche transluzente Körper, von denen ein erster transluzenter Körper 2076 für die Einführung in eine Vagina und ein zweiter transluzenter Körper für die Einführung 2076^{I} für die Einführung in einen Anus gestaltet sind. In diese transluzente Körper 2076, 2076^{I} ist jeweils wechselweise der LED-Träger 2150 des Bestrahlungssteckstücks 2001 einsteckbar. Der LED-Träger 2150 ist auf eine Abschlussplatine 2130 aufsteckbar, wobei eine elektrisch leitende Verbindung von dem LED-Träger 2150 zu einer Steuerplatine 2120 ausgebildet wird. Die Steuerplatine 2120 ist in einen kastenartigen Korpus 2060, dem Verwahrkasten, einsetzbar, der durch die Abschlussplatte 2130 verschlossen wird. Der LED-Träger 2150 weist anschlussseitig einen LED-Kragen 2148 auf, auf dem mehrere LEDs, wie die LED 2008 und die LED 2008^{I}, ringartig nebeneinander angeordnet sind. Zu diesen LEDs gehören auch Rotlicht-LEDs, wie die LED 2176, und Bestrahlungs-LEDs, wie die LED 2161, die unabhängig voneinander zur Lichtabgabe ansteuerbar sind. Beispielsweise kann eine Rotlicht-LED 2176 als Betriebszustandsanzeige geschaltet werden und eine Bestrahlungs-LED, wie die LED 2161, kann als eine Grün-LED zur Anzeige eines zweiten Betriebszustands geschaltet werden. In einem dritten Betriebszustand kann von den LEDs 2008, 2008^{I}, 2161, 2176 therapeutisch wirksames oder ein Wohlbefinden steigerndes Licht abgegeben werden. Der LED-Träger 2150 weist an seinem dem LED-Kragen 2148 gegenüberliegenden Ende vier LEDs auf, die als ein erstes End-LED-Paar 2168 und ein zweites End-LED-Paar 2168^{I} gruppiert sind. Die End-LED-Paare 2168, 2168^{I} bilden zusammen ein Quadrat. Zu den End-LEDs gehören Blaulicht-LEDs, wie die LED 2171, und UV-C-LEDs, wie die LED 2178. Weitere LEDs, wie die Blaulicht-LEDs 2171^{I}, 2171", 2173, 2173^{I} sind mit UV-C-LEDs, wie den LEDs 2177, 2178^{I}, jeweils wechselweise entlang dem LED-Träger 2150 angeordnet. Die Blaulicht-LEDs 2171^{I}, 2171", 2173, 2173^{I} sind separat von den UV-C(-Licht)-LEDs 2177, 2178^{I} für eine Blaulicht-Behandlung über die Steuerplatine 2120 einschaltbar. Für eine UV-C-Licht-Behandlung können die UV-C-LEDs 2177, 2178^{I} zusätzlich zu den Blaulicht-LEDs 2171^{I}, 2171^{II}, 2173, 2173^{I} oder auch nur separat, d. h. für sich alleine, durch die Steuerplatine 2120 mit Strom versorgt bzw. eingeschaltet werden. Somit werden durch das Bestrahlungssteckstück 2001 verschiedene Lichtbehandlungsmethoden ermöglicht, zu denen insbesondere auch ein Dauerbestrahlungsbetrieb oder wahlweise ein Pulsbestrahlungsbetrieb gehören.

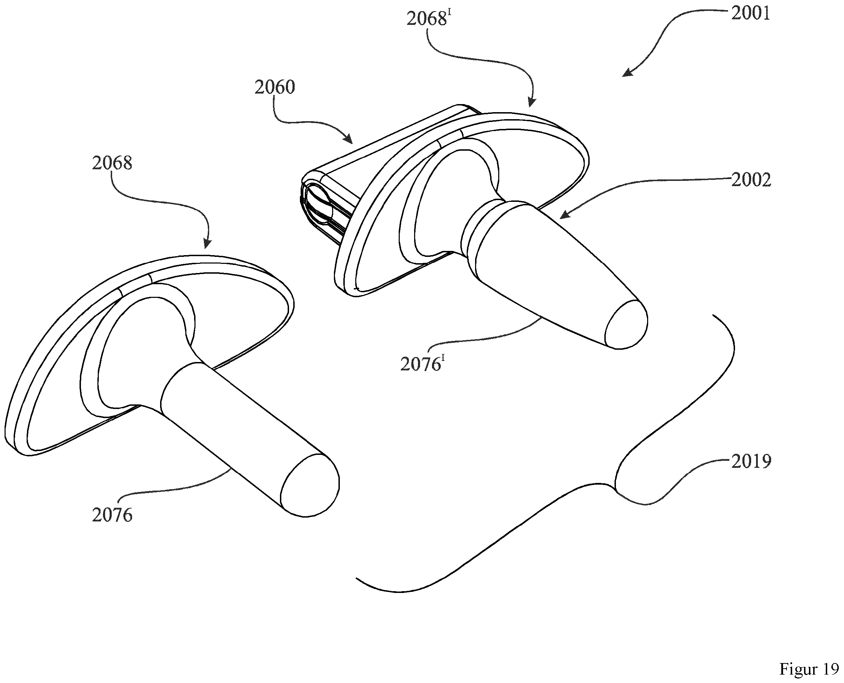

Figur 19 zeigt das Bestrahlungssteckstück 2001, das aus Figur 18 bekannt ist, in einem zusammengebauten Zustand, wobei der kastenartige Korpus 2060 (im Sinne eines Verwahrkastens für eine Steuerungsplatine bzw. für eine elektronische Steuerung des Bestrahlungssteckstücks 2001) mit dem transluzenten Körper 2076^{I} im Bereich des Körperöffnungsschutzes 2068^{I} verbunden ist. Wahlweise kann zur Ausbildung eines Einführteils 2002 der für eine Vaginaleinführung gestaltete transluzente Körper 2076 bis zu dem kastenartigen Korpus 2060 aufgezogen und über den Körperöffnungsschutz 2068 mit diesem verbunden werden. Mit dem so zur Verfügung stehenden Set 2019 lassen sich viele verschiedenartige Behandlungen an unterschiedlichen Körperöffnungen einer Person durchführen. Anders gesagt, lassen sich mit Hilfe eines Sets, wie dem Set 2019, Bestrahlungssteckstücke 2001 modifizieren, um z. B. verschiedene Durchmesser und Längen von Einzelteilen, angepasst auf die jeweilige Personenkörperöffnung, bereitzustellen.

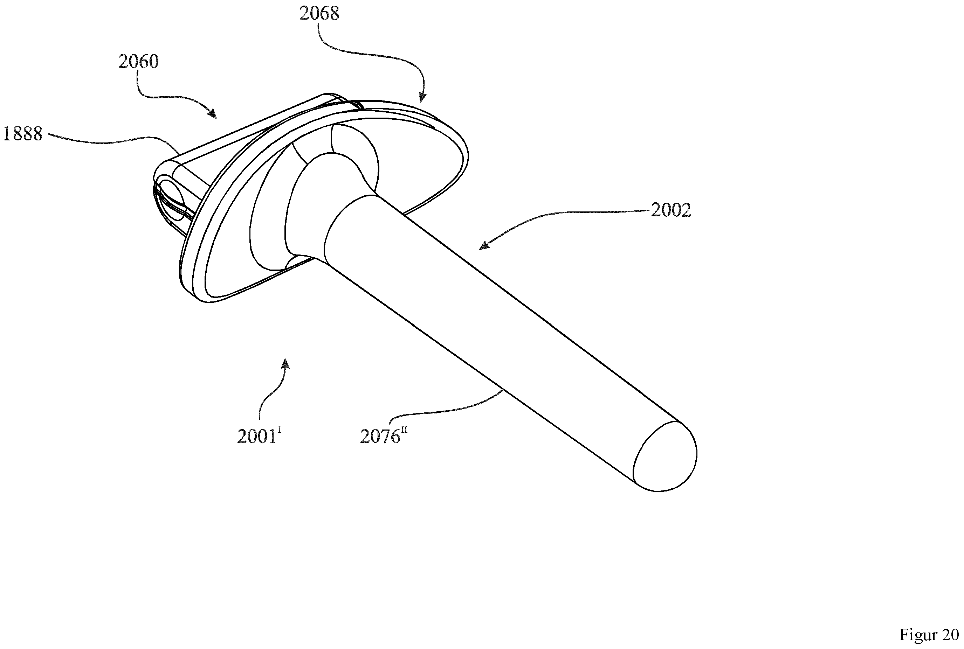

Figur 20 zeigt ein Bestrahlungssteckstück 2001^{I}, das in Bezug auf viele Teile dem aus den Figuren 18 und 19 bekannten Bestrahlungssteckstück 2001, der ein Abdo-Bestrahlungssteckstück ist, ähnelt bzw. in dem viele gleiche Teile verwendet worden sind.

Die weiter oben stehenden Beschreibungen zu den Ausführungsbeispielen der Mundsteckstücke 1 (siehe Figur 1), 201 (siehe Figuren 2 bis 5), 401 (siehe Figuren 6 bis 9), 601 (siehe Figur 10), 801 (siehe Figur 11), 1001 (siehe Figur 12), 1201 (siehe Figuren 13 und 14), 1401 (siehe Figur 15), 1601 (siehe Figur 16) lassen sich daher auf die Beschreibung des Bestrahlungssteckstücks 2001^{I} übertragen.

Das Bestrahlungssteckstück 2001^{I} hat einen länglichen, gerundeten transluzenten Körper 2076", der im Vergleich mit dem transluzenten Körper 1876 (nach Figur 17) länger ist und somit tiefer in die ihn aufnehmende Körperhöhle eindringt. Der transluzente Körper 2076" ist Teil des Einführteils 1802". Sowohl der transluzente Körper 2076" als auch eine zusätzliche Abdeckung, die eine zweite Hülle 1888 darstellt, sind aus einem Kunststoff gefertigte Hüllen, die abwaschbar, vorteilhafterweise wasserdicht bzw. wasserabweisend, sind. Der kastenartige Korpus oder Körper 2060, der Verwahrkasten wird durch einen Körperöffnungschutz 2068 auf einer seiner Seiten abgeschlossen. Der Körperöffnungschutz 2068 ist gebogen gestaltet, um am Körper einer Person anzuliegen. Dies steigert den Tragekomfort für die das Bestrahlungssteckstück 2001^{I} nutzenden Person.

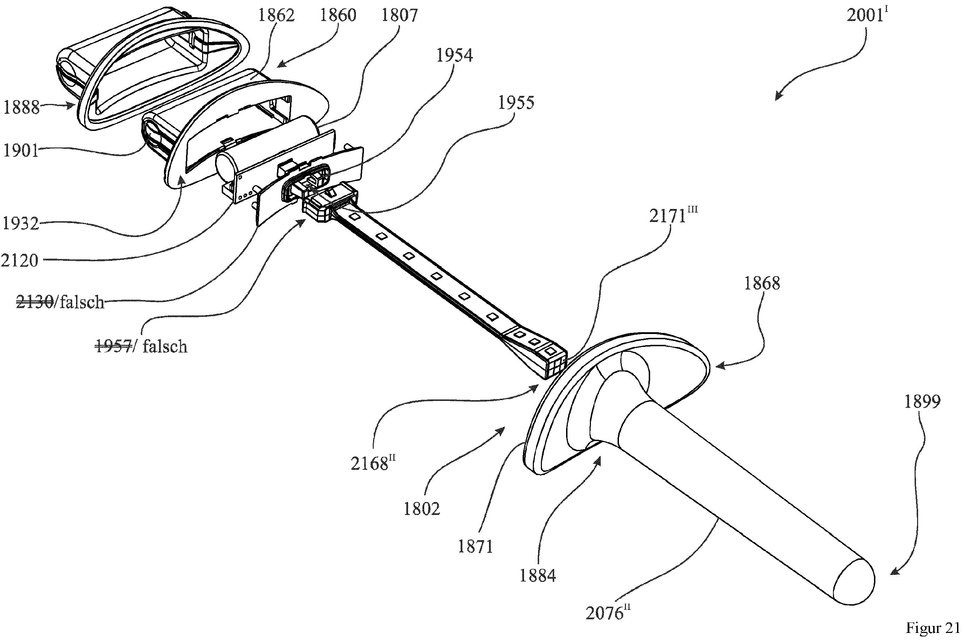

In Figur 21 wird das Bestrahlungssteckstück 2001^{I}, das anhand von Figur 20 vorgestellt worden ist, in einer Explosionszeichnung gezeigt, d. h. die einzelnen Teile sind auseinandergezogen dargestellt. Das Einführteil 1802" setzt sich im Inneren aus dem Träger 2150^{I} mit seinem Clipverbinder 2157 und mit u. a. der End-LED-Gruppe 2168" sowie außen dem transluzenten Körper 2076" zusammen. Die End-LED-Gruppe 2168" setzt sich aus mehreren Blaulicht-LEDs 2171" zusammen und ist eine nach vorne ausgerichtete Fläche, sozusagen eine Stirnfläche des Trägers 2150^{I}. Der transluzente Körper 2076" hat mehrere, von der das Aussehen wesentlich dominierenden runden, länglichen Form abweichende Teile, wie das Ende 1899, den Einklemmbereich 1884" und den Körperöffnungsschutz 1868. An dem Körperöffnungsschutz 1868 geht in die Anschlusslippe 1871 über. Aufgrund des Clipverbinders 2157, über den das Einführteil 1802" kann ein anderes Einführteil als das Einführteil 1802^{II} mit dem restlichen Gerät bzw. den restlichen Teilen des Bestrahlungssteckstücks 2001^{I} verbunden werden. Der Clipverbinder 2157 kann an der Anlagefläche 2104 anliegen und über Kontaktführungen wie die Kontaktführung 1954, die in der Einstecköffnung 1955 vorhanden sind, elektrischen Kontakt zu der Steuerplatine 2120 und der Energieversorgung aus dem Akkumulator 1807 herstellen. Die Anlagefläche 2104 hat die gleiche Biegung bzw. querverlaufende Wölbung wie eine zweite Anlagenfläche 2104^{I}, die Teil des kastenartigen Körpers 1860 ist. Die beiden Anlagenflächen 2104, 2104^{I} ergeben zusammen die Abschlussplatte 2130, sodass diese insgesamt die Dichtfläche 1932 bilden können. Die Anschlusslippe 1871 des transluzenten Körpers 2076" nimmt ein Ende der Abdeckung bzw. der zweiten Hülle 1888 auf, sodass die Abdeckung 1888 und der transluzente Körper 2076" zusammen eine dichte Hülle um die übrigen Bauteile des Bestrahlungssteckstücks 2001^{I} bilden. Das Gehäuse 1862 ist ein kastenartiger Korpus 1860, der vollständig in der Abdeckung 1888 verschwinden kann. Die Abdeckung 1888 ist genau so nachgiebig wie der transluzente Körper 2076^{II}, sodass eine Einschalttaste bzw. ein Einschalttaster 1901 durch die Abdeckung 1888 hindurch betätigt werden kann. Mithilfe des Einschalttasters 1901 kann durch ein Schaltsignal an die Steuerplatine 2120 Energie des Akkumulators 1807 an den Träger 2150^{I} und die dort vorhandenen LEDs wie die End-LED-Gruppe 2168" zur Verfügung gestellt werden. Die Dichtfläche 1932 ist so gestaltet, dass der Körperöffnungsschutz 1868 den an die Körperhöhle angrenzenden Körper vollständig abdecken kann.

Dank der Abdeckung 1888 und dank des transluzenten Körpers 2076", die beide aus einem Kunststoff gefertigt sind, kann das Bestrahlungssteckstück 2001^{I} unter laufendes Wasser, z. B. in einem Waschbecken, gehalten und abgewaschen werden.

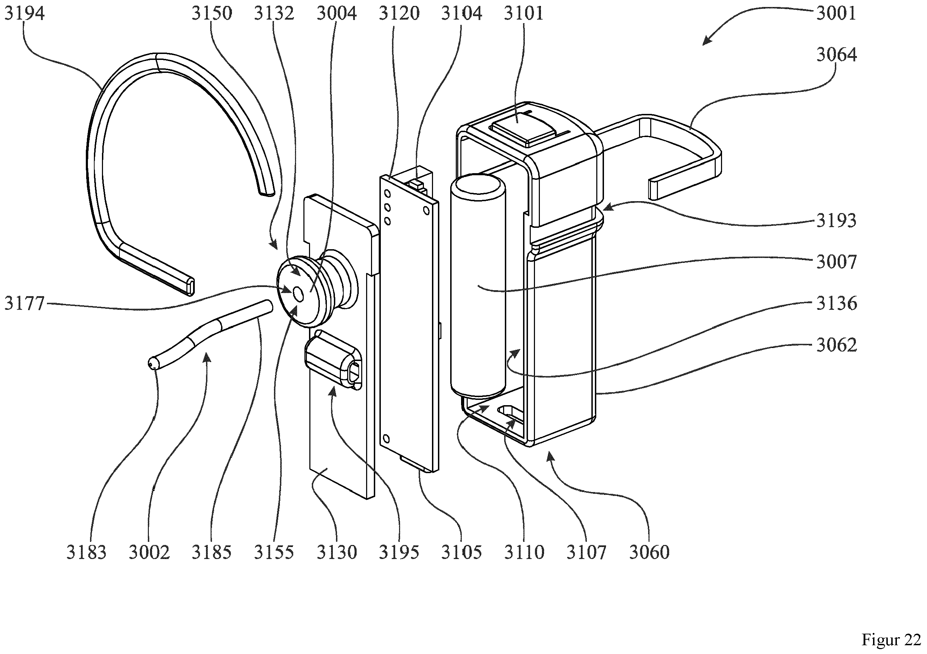

Das in Figur 22 gezeigte Bestrahlungssteckstück 3001 kann auch als Gehörgang-Bestrahlungssteckstück bezeichnet werden. Das Bestrahlungssteckstück 3001 ist in Figur 20 mit voneinander beabstandet gezeichneten Komponenten dargestellt. Das Bestrahlungssteckstück 3001 weist einen kastenartigen Korpus 3060 auf, wobei in einem Außengehäuse 3062 ein Batteriefach 3110 ausgebildet ist, das zur Aufnahme einer Batterie 3007 dient. Das Außengehäuse 3062 kann auch als Akkukasten oder als Griffeinheit bezeichnet werden. Die Batterie 3007 ist wiederaufladbar. Das Gehäuse bzw. Außengehäuse 3062 ist quaderartig geformt und weist an seinen kleinsten Flächen bzw. den einander gegenüberliegend angeordneten Flächen einen Einschalttaster 3101 und einen Buchsenschlitz 3107 auf. In das Gehäuse 3062 ist eine Steuerplatine 3120 einsetzbar. Der Einschalttaster 3101 dient zur Betätigung eines Schalters 3104 auf der Steuerplatine 3120. Auf der Steuerplatine 3120 befindet sich außerdem eine USB-Buchse 3105, die durch den Buchsenschlitz 3107 in dem Gehäuse 3062 hindurch z. B. zur Aufladung der Batterie 3007 mit einem USB-Stecker (nicht dargestellt) kontaktierbar ist. Die Steuerplatine 3120 versorgt eine LED mit elektrischem Strom aus der Batterie 3007, wobei die LED an der Abschlussplatte 3130 in einem Innenbereich eines LED-Trägers 3150 befestigt ist und daher in der in Figur 20 gezeichneten Darstellung nicht zu sehen ist. Der LED-Träger 3150 steht von der Abschlussplatte 3130 um eine Länge ab, die in etwa der Tiefe einer Ohrmuschel entspricht. Als Lichtquelle kommt eine UV-C-Licht-LED 3177 zum Einsatz, die in eine Einstecköffnung 3155 in dem LED-Träger 3150 gerichtet ist. In die Einstecköffnung 3155 ist ein Lichtleiter 3185 einführbar, der an seinem Ende transluzent ausgebildet ist und eine Mikrolinsenoptik 3183 aufweist. Der LED-Träger 3150 und der Lichtleiter 3185 bilden zusammen ein Einführteil 3002. Die Abschlussplatte 3130 weist neben dem LED-Träger 3150 einen Bügelträger 3195 auf, in den ein Ohrtragebügel 3194 einsteckbar ist. Je nach Einsteckrichtung des Ohrtragebügels 3194 lässt sich das Bestrahlungssteckstück 3001 zum Tragen an einem (nicht dargestellten) linken Ohr oder für ein rechtes Ohr (nicht dargestellt) anpassen. Das Gehäuse 3062 weist außerdem eine Betriebszustandsanzeige in Gestalt eines transluzenten Fensters 3193 auf, die mit einem einschiebbaren bzw. ausziehbaren Griff 3064 abgedeckt werden kann. Der Griff 3064 erleichtert die Handhabung des kastenartigen Korpus 3060. Eine Dichtfläche 3132 an dem LED-Träger 3150 dient beim Tragen zur Auflage in der Ohrmuschel um den Gehörgang herum. Damit wird der Gehörgang (nicht dargestellt) und das Trommelfell bzw. das Innere des Ohres bei einer Lichtbehandlung geschützt.

Das für ein Ohr bestimmte Bestrahlungssteckstück 3201 (Ohrbestrahlungssteckstück bzw. Ohrsteckstück) hat eine Anlagefläche 3004, die als Dichtfläche 3132 ausgestaltet ist. Die Anlagefläche 3004 ist dafür vorgesehen, ein Ohr von außen komplett abzudecken. Hierfür ist die Anlagefläche 3004 gerundet ausgeführt. Die Anlagefläche 3004 ist mit einem Überzug versehen, der formadaptiv, d. h. leicht nachgiebig ist.

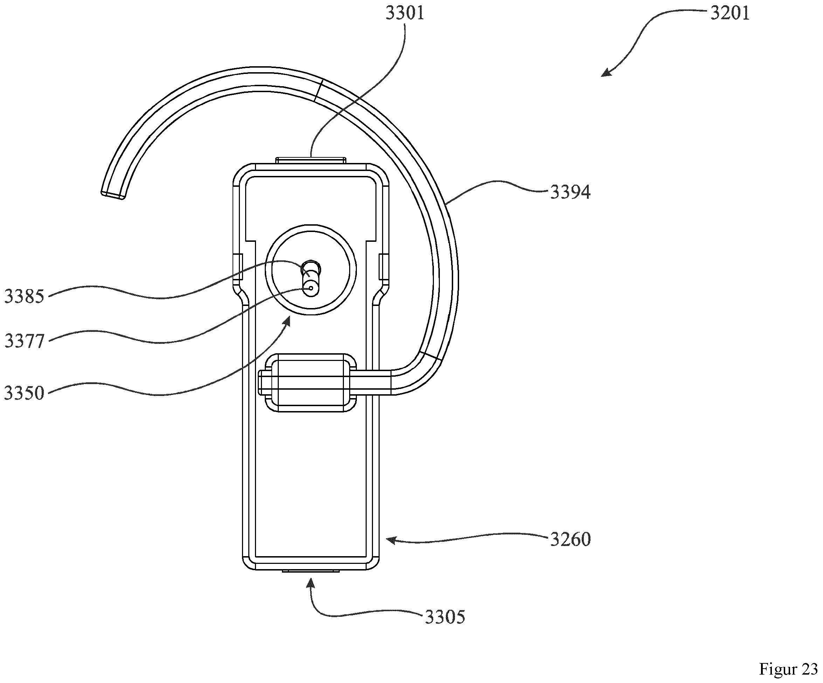

Das in Figur 23 gezeigte Bestrahlungssteckstück 3201 ist für das Tragen an einem anderen bzw. einem zweiten Ohr im Vergleich zu dem Bestrahlungssteckstück 3001 gem. Figur 22 ausgebildet. Das Bestrahlungssteckstück 3201 weist einen kastenartigen Korpus 3260 auf, an dem sich ein Einschalttaster 3301, eine USB-Buchse 3305 und ein Ohrtragebügel 3394 befinden. In einen LED-Träger 3350 des Bestrahlungssteckstücks 3201 ist ein Lichtleiter 3385 eingesetzt, der an seinem Ende eine UV-C-LED 3377 aufweist. Abgestrahltes UV-C-Licht wird großflächig und gleichmäßig entlang des Gehörgangs verteilt. Eine Person kann sich bei einer Behandlung mit einem ersten Gehörgang-Bestrahlungssteckstück 3001 und ggf. einem zweiten Gehörgang-Bestrahlungssteckstück 3201 frei im Raum bewegen, d. h. unproblematisch umherlaufen; sie ist also weiterhin mobil.

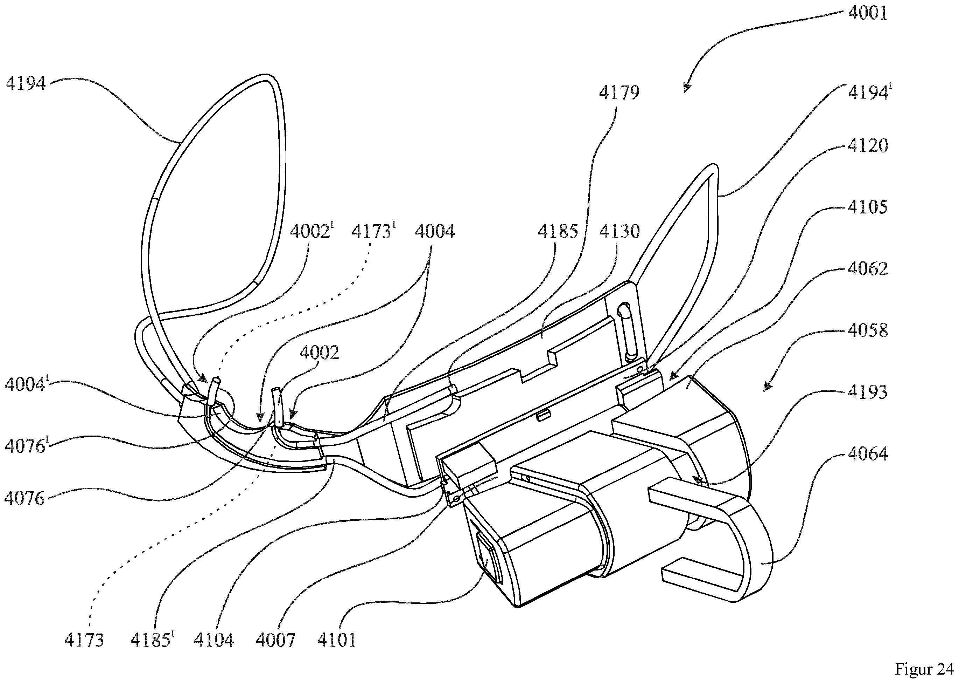

In Figur 24 ist ein Bestrahlungssteckstück 4001 gezeigt, das auch als Nasenloch-Bestrahlungssteckstück bezeichnet werden kann. Das Nasenloch-Bestrahlungssteckstück 4001 kann über zwei Ohrtragebügel 4194, 4194^{I} an einem menschlichen Kopf (nicht dargestellt) so befestigt werden, dass jeweils ein Einführteil 4002, 4002^{I} , es kann auch von einem Einführteilpaar 4002, 4002^{I} gesprochen werden, in eines der beiden Nasenlöcher (nicht dargestellt) ragt. Die Einführteile 4002, 4002^{I} weisen jeweils einen transparenten Körper 4076, 4076^{I} auf, der jeweils über einen Lichtleiter 4185, 4185^{I} mit einer Laser-LED, wie der Laser-LED 4179, verbunden ist. Wahlweise können in einer hier nicht graphisch dargestellten Ausführungsform auch statt der Laser-LEDs 4179 Blaulicht-LEDs oder UV-C-LEDs oder LEDs, die Licht in einem UV-Wellenlängenbereich bzw. in einem violetten Wellenlängenbereich abstrahlen, zum Einsatz kommen. Es ist auch möglich, in einer weiteren Ausführungsform statt dem Einbau von Laser-LEDs an den Einführteilen 4002, 4002^{I} jeweils Blaulicht-LEDs, wie die Blaulicht-LEDs 4173, 4173^{I}, vorzusehen, mit denen Licht in die Tiefe der Nasenlöcher einstrahlbar ist. Das Licht, das von den Laser-LEDs, wie der Laser-LED 4179, ausgestrahlt wird, tritt durch die transluzenten Körper 4076, 4076^{I} in die Nasenlöcher (nicht dargestellt) ein und gelangt zumindest bis in eine Nähe zur Nasenhöhle (s. Nasenhöhle 44 in Figur 10). Die Abschlussplatte 4130 ist auf eine Steuerplatine 4120 aufsteckbar und wird mit dieser elektrisch verbunden. Die Steuerplatine 4120 trägt einen Schalter 4104 und eine USB-Buchse 4105. Der Schalter 4104 ist in einem zusammengebauten Zustand des Bestrahlungssteckstücks 4001 durch den Einschalttaster 4101 betätigbar. Die Abschlussplatte 4130 kann mit einem blockartigen Korpus 4058 zusammengeschlossen werden, der in seinem Inneren einen Akkumulator 4007 schützend trägt. Das Gehäuse 4062 des blockartigen Korpus 4058 weist zusätzlich zu dem Einschalttaster 4101 eine Betriebszustandsanzeige 4193 auf, die mit Hilfe eines aufsteckbaren Griffs 4064 abgedeckt werden kann. Somit kann sich eine Person bei einer Behandlung mit einem Nasenloch-Bestrahlungssteckstück 4001 frei im Raum bewegen, d. h. sie ist weiterhin mobil.

Das Bestrahlungsteckstück 4001, das in Figur 24 gezeigt ist, hat zwei Einführteile 4002, 4002^{I}, die aus einem Körper herausstehen, der als Anlagekörper mit seinen Anlageflächen 4004, 4004^{I} bezeichnet werden kann. Der Anlagekörper ist mit Anlageflächen 4004, 4004^{I} ausgestattet. Die Anlageflächen 4004, 4004^{I} sind leicht gebogen bzw. gekrümmt. Die Anlageflächen 4004, 4004^{I} sind einer Nase im Bereich ihres Nasenstegs nachgebildet. Der Verlauf der Anlageflächen 4004, 4004^{I} ist gebogen, sodass einer typischen Nasenform (insbesondere eines Mitteleuropäers) durch die Anlageflächen 4004, 4004^{I} gefolgt werden kann und die Nasenlöcher abgedeckt werden.

Die in den beiden Bestrahlungssteckstücken 3001 (siehe Figur 22) und 4001 (siehe Figur 24), dem Bestrahlungssteckstück 3001 für eine Ohrenbestrahlung und dem Bestrahlungssteckstück 4001 für eine Nasenbestrahlung, verwendeten Einführteile 3002, 4002 sind schmaler als die entsprechenden Einführteile 2, 202, 402, 602, 1402, 1602, 1802, 1802^{I}, 1802" eines Mundsteckstücks 1, 201, 401, 601, 801, 1001, 1201, 1401, 1601 und eines Abdo-Steckstücks 1801, 2001, 2001^{I}, sodass im Inneren schmalerer Einführteile 3002, 4002 Mikro-LEDs mit Breiten zwischen 50 µm und 500 µm eingebaut sind, während in den anderen Einführteilen 2, 202, 402, 602, 1402, 1602, 1802, 1802^{I}, 1802^{II} gängige Blau-Licht-LEDs, z. B. kostengünstigere SMD-LEDs, verbaut sein können.

Die in den einzelnen Figuren gezeigten Ausgestaltungsmöglichkeiten lassen sich auch untereinander in beliebiger Form verbinden.

Basierend auf den Bestrahlungssteckstücken 1, 201, 401, 601, 801, 1001, 1201, 1401, 1601, 1801, 2001, 2001^{I}, 3001, 3201, 4001 sind weitere vorteilhafte Ausführungsformen - je nach gewünschter Behandlungsweise - möglich. Ein Bestrahlungssteckstück kann ausschließlich mit LED-Bändern mit Blaulicht-LEDs ausgestattet sein. Alternierend können Blaulicht-LEDs und Rotlicht-LEDs auf den LED-Bändern angeordnet sein oder jeweils auf einem LED-Band für eine Art LED mit lichtfarblich unterschiedlichen LED-Bändern nebeneinander eingesetzt sein. Es ist auch möglich, statt Rotlicht-LEDs Infrarot-LEDs zu verwenden, um die Körperhöhle besser zu erwärmen. Weitere vorteilhafte Ausführungsformen weisen LED-Bänder mit UV-LEDs an allen Lichtabstrahlungsflächen auf. Ein solches Bestrahlungssteckstück mit UV-C-LEDs wird vorzugsweise mit einem Bestrahlungssteckstücksack oder einer Bestrahlungssteckstückhülle (nicht dargestellt) kombiniert bzw. darin eingesetzt, um zu verhindern, dass UV-C-Licht bei einem Betrieb des Bestrahlungssteckstücks einem Betrachter bzw. einem Benutzer in die Augen fällt. Mit UV-Licht können außer therapeutischen Hautverbesserungen auch Hautbräunungen bewirkt werden.

So ist es möglich, auch noch mehr LEDs, ganz allgemein gesprochen, mehr Leuchtmittel, auf einem LED-Träger anzuordnen.

**Bezugszeichenliste**

| | |
|---|---|
| 1, 201, 401, 601, 801, 1001, 1201, 1401, 1601, 1801, 2001, 2001^{I}, 3001, 3201, 4001 | Bestrahlungssteckstück, insbesondere Mundsteckstück, aber auch Abdosteckstück, Vaginasteckstück, Ohrsteckstück und Nasensteckstück |
| 2, 202, 402, 602, 1402, 1602, 1802, 1802^{I}, 1802^{II}, 2002, 3002, 4002, 4002^{I} | Einführteil, insbesondere Mundhöhlenkörper |
| 3, 203 | Längserstreckung, insbesondere des Mundsteckstücks |
| 4, 204, 404, 1404, 2104, 2104', 3004, 4004, 4004^{I} | Anlagefläche |
| 5, 205, 605 | Auflage, insbesondere Lichtreflexionsfläche |
| 6,406 | Lichtquelle |
| 7, 407,1807, 4007 | Energiequelle, insbesondere Akkumulator |
| 8, 2008, 2008^{I} | LED |
| 9, 409 | Zeitsteuerung |
| 10, 210 | Lichtabgabebereich |
| 11, 611, 1411 | Verwahrkasten, insbesondere in Gestalt eines knopfartigen Korpus, insbesondere als knopfartiger Körper |
| 12, 212 | elektrischer Anschluss |
| 13, 213 | erste Halbschale |
| 14, 214 | zweite Halbschale |
| 15, 1415, 1415^{I}, 1615, 1615^{I} | Licht |
| 16 | Kabel |
| 1417, 1617, 1617^{I} | mundschutznahes LED-Licht |
| 1418, 1618, 1618^{I} | endnahes LED-Licht, insbesondere Licht zur Behandlung eines Rachenbereichs |
| 1819, 2019 | |
| 20, 20^{I} | Mundhöhle, insbesondere Mundinnenraum |
| 21, 21^{I} | obere Schneidezähne |
| 22, 22^{I} | untere Schneidezähne |
| 23, 23^{I} | Oberlippe |
| 23^{II} | Amorbogen |
| 24, 24^{I} | Unterlippe |
| 25, 25^{I} | Zunge |
| 26, 26^{I} | Gaumen, insbesondere harter Gaumen mit Wölbung |
| 28 | Gaumen, insbesondere weicher Gaumen mit Wölbung |
| 30 | Gaumenzäpfchen |
| 32 | Zungenrücken, insbesondere anterodorsaler Bereich einer Zungenwölbung |
| 34 | Zungenrücken, insbesondere posterodorsaler Bereich einer Zungenwölbung |
| 36 | sublaminaler Bereich |
| 38 | Oberkiefer |
| 40 | Unterkiefer |
| 42 | Rachen |
| 44 | Nasenhöhle |
| 46 | Nasenrachen |
| 48 | Kehldeckel |
| 50 | Luftröhre |
| | |
| 458, 1258, 1458, 4058 | Verwahrkasten, insbesondere in Gestalt eines blockartigen Korpus, insbesondere als blockartiger Körper |
| 260, 860, 1660,1860, 2060, 3060, 3260 | Verwahrkasten, insbesondere in Gestalt eines kastenartigen Korpus, insbesondere als kastenartiger Körper |
| 262, 462, 862, 1062, 1462, 1662, 1862, 3062, 4062 | Gehäuse, insbesondere Außengehäuse, wie eine Elektronikbox |
| 263 | Anschlusskante |
| 264, 464, 3064, 4064 | Griff |
| 465 | Reflektorfläche |
| 266 | Längserstreckung des kastenartigen Körpers |
| 268, 468, 668, 1468, 1668, 1868, 1868^{I}, 2068, 2068^{I} | Körperöffnungsschutz, insbesondere Mundschutz |
| 269 | Körperöffnungsbezugspunkt, insbesondere Lippenbezugspunkt |
| 270 | Breite des Körperöffnungsschutzes, insbesondere des Mundschutzes |
| 271, 1871 | Anschlusslippe |
| 272 | Höhe des Körperöffnungsschutzes, insbesondere des Mundschutzes |
| 273, 473 | Dehnungsmulde |
| 274 | Breite des kastenartigen Korpus, insbesondere des Körpers |
| 275 | Durchlüftungsöffnung, insbesondere mit Keimfilter ausgestattete Öffnung |
| 276, 476, 676, 1476, 1676, 1876, 1876^{I}, 2076, 2076^{I}, 2076^{II}, 4076, 4076^{I} | transluzenter Körper, insbesondere transluzente Hülle |
| 278, 478 | bogenförmiger Stiel |
| 280, 480, 1880 | Anschlussteil, insbesondere Lippenanschlusskörper |
| 282, 482, 1482 | Kunststoffhülle, insbesondere Schutzhülle des Mundhöhlenkörpers |
| 284, 284^{I}, 1484, 1884, 1884^{I}, 1884^{II} | Einklemmbereich, z. B. eine Zahnstützfläche wie eine Bissfläche oder z. B. eine Muskelanpressfläche |
| 286 | Verlängerungsbereich |
| 1888 | zweite Hülle, insbesondere Gehäusehülle,vorzugsweise aus einem abwaschbaren Kunststoff |
| 290, 490, 1490, 1690 | erster Stielbogen, insbesondere gaumenseitiger Stielansatzbogen |
| 291, 491, 1691 | zweiter Stielbogen, insbesondere gaumenseitiger Stielmittenbogen |
| 292, 492, 1692 | dritter Stielbogen, insbesondere gaumenseitiger Stielendbogen |
| 295, 1695 | vierter Stielbogen, insbesondere zungenseitiger Stielansatzbogen |
| 296, 1696 | fünfter Stielbogen, insbesondere zungenseitiger Stielmittenbogen |
| 297, 1697 | sechster Stielbogen, insbesondere zungenseitiger Stielendbogen |
| 299, 1699, 1899, 1899^{I} | siebter Stielbogen, insbesondere Ende des Einführteils, wie mundhöhlenseitiges Ende des Mundhöhlenkörpers |
| 301, 501, 701, 1501, 1701, 1901, 3101, 3301, 4101 | Einschalttaster |
| 502 | Hebelführung |
| 303 | Tasterfeder |
| 504, 3104, 4104 | Schalter, insbesondere Schalter mit Zeitsteuerung, wie ein Timer |
| 305, 505, 3105, 3305, 4105 | USB-Buchse |
| 3107 | Buchsenschlitz |
| 510, 1910, 3110 | Batteriefach bzw. Akkumulatorfach |
| 512 | Tasteranschlag |
| 520, 920, 1320, 1920, 2120, 3120, 4120 | Steuerplatine |
| 530, 930, 1130, 1330, 1930, 2130, 3130, 4130 | Abschlussplatte |
| 532, 1932, 3132 | Dichtfläche |
| 334, 534, 534^{I}, 534^{II} | Kippnase |
| 536, 3136 | Dichtkante |
| 540 | Inneres, insbesondere des Mundhöhlenkörpers |
| 2148 | LED-Kragen |
| 550, 950,1150, 1350, 1550, 1750, 1950, 2150, 2150^{I}, 3150, 3350 | LED-Träger |
| 1151, 1151^{I}, 1351, 1351^{I} | Seitenschutz |
| 1152, 1352, 1552 | Trägerrundung |
| 1153 | Steckkontakt |
| 1154, 1954 | Kontaktführung |
| 1355, 1955, 3155 | Einstecköffnung |
| 556 | Anschraubverbinder |
| 957, 1157, 1357, 1957, 2157 | Clipverbinder |
| 558, 958, 1158 | Steckverbinder |
| 959, 959^{I}, 1159, 1159^{I}, 1959 | Clip, insbesondere seitlicher Clip |
| 560,1160, 1360, 1560, 1760 | LED-Band, insbesondere LED-Platine |
| 1360^{I} | Platinenmittenbereich, insbesondere elastischer Rundungsbereich |
| 961, 961^{I}, 961^{II}, 961^{III}, 961^{IV}, 961^{V}, 961^{Vl}, 961^{VII}, 961^{VIII}, 1361, 1361^{I}, 1361^{II}, 1361^{III}, 1361^{IV}, 1361^{V}, 1361^{VI}, 1561, 1561^{I}, 1561^{V}, 1561^{VI}, 1561^{VII}, 1761, 1761^{I}, 1761^{II}, 1761^{III}, 1761^{IV}, 1761^{V}, 1761^{VI}, 1761^{VII}, 2161 | LED |
| 562, 1562, 1962 | erster LED-Strang, insbesondere obere LEDs |
| 1163, 1363 | Platinenendbereich |
| 564, 1564, 1964 | zweiter LED-Strang, insbesondere untere LEDs |
| 1565, 1565^{I}, 1565^{V}, 1565^{VI}, 1565^{VII}, 1565^{VIII}, 1765, 1765^{I}, 1765^{II}, 1765^{III}, 1765^{IV}, 1765^{V}, 1765^{VI} | LED |
| 566, 1366 | Kontaktfeld mit mehreren Kontakten, insbesondere LED-Stromversorgungskontakte |
| 1367, 1367^{I} | LED-Paar |
| 1368, 2168, 2168^{I}, 2168^{II} | End-LED-Gruppe, wie ein Rundungs-LED-Paar |
| 1369, 1569, 1769, 1769^{I} | erster LED-Abstand |
| 1370, 1570, 1770, 1770^{I} | zweiter LED-Abstand |
| 571, 2171, 2171^{I}, 2171^{II} | Blaulicht-LED |
| 572 | Blaulicht-LED, insbesondere LED zur Bestrahlung des sublaminalen Bereichs |
| 573, 2173, 2173', 4173, 4173^{I} | Blaulicht-LED |
| 1774 | LED-Abstand, insbesondere Abstand von zwei zungenseitigen LEDs im Mittenbereich des Mundhöhlenkörpers |
| 1575, 1575^{I}, 1775, 1775^{I} | erster Bereich, insbesondere Bereich mit einer ersten Helligkeit, wie ein dunklerer Lichtabgabebereich des Mundhöhlenkörpers |
| 2176 | Rotlicht-LED |
| 577, 2177 | UV-C-Licht-LED |
| 578, 2178, 2178^{I}, 3177, 3377 | UV-C-Licht-LED |
| 579, 4179 | Laser-LED |
| 1580, 1580^{I}, 1780, 1780^{I} | zweiter Bereich, insbesondere Bereich mit einer zweiten Helligkeit, wie ein hellerer Lichtabgabebereich des Mundhöhlenkörpers |
| 581 | Temperaturüberwachung, insbesondere Temperatursensor |
| 583, 3183 | Mikrolinsenoptik |
| 3185, 3385, 4185, 4185^{I} | Lichtleiter |
| 589, 589^{I}, 1989 | Betriebszustands-LED, vorzugsweise Seiten-LED |
| 591, 591^{I} | Lichtkanal |
| 593, 3193, 4193 | Betriebszustandsanzeige, insbesondere in Form eines transluzenten Fensters |
| 3194, 3394, 4194, 4194^{I} | Ohrtragebügel |
| 3195 | Bügelträger |
| | Fußstellfläche |
| | Versorgungskabel, insbesondere Strom und Datenkabel |

## Patentansprüche

1. Bestrahlungssteckstück (1, 201, 401, 601, 801, 1001, 1201, 1401, 1601, 1801, 2001, 2001', 3001, 3201, 4001),
das ein längliches Einführteil (2, 202, 402, 602, 1402, 1602, 1802, 1802^{I}, 2002, 3002, 4002, 4002') und
einen blockartigen Verwahrkasten (11, 260, 458, 611, 860, 1258, 1411, 1458, 1660, 1860, 2060, 3060, 3260, 4058) umfasst,
wobei das Einführteil (2, 202, 402, 602, 1402, 1602, 1802, 1802^{I}, 2002, 3002, 4002, 4002^{I}, 5002, 6002) aufgrund seiner Länge und Breite zur Aufnahme in einer Körperöffnung (20, 20^{I}) eines Menschen oder Tiers gestaltet ist,
und wobei zum Einführteil (2, 202, 402, 602, 1402, 1602, 1802, 1802^{I}, 2002, 3002, 4002, 4002^{I}) wenigstens eine wenigstens abschnittsweise, vorzugsweise vollständig, transluzente, insbesondere erste, Hülle (276, 476, 676, 1476, 1676, 1876, 1876^{I}, 2076, 2076^{I}, 4076, 4076^{I}) gehört,
durch die eine Abgabe eines Lichts (15, 1415, 1415^{I}, 1417, 1418, 1615, 1615^{I}, 1617, 1617', 1618, 1618^{I}),
insbesondere durch mehrere LEDs (571, 572, 573, 577, 578, 579, 961, 1361, 1367, 1368, 1561, 1565, 1761, 1765) erzeugt,
mit einer bakterizid und/oder viruzid und/oder fungizid wirkenden Wellenlänge, z. B. im Rotlichtbereich, Blaulichtbereich, Violettlichtbereich und/oder im Ultraviolettlichtbereich, ermöglicht wird,
**dadurch gekennzeichnet, dass**
das Bestrahlungssteckstück (1, 201, 401, 601, 801, 1001, 1201, 1401, 1601, 1801, 2001, 2001', 3001, 3201, 4001) mit einer,
insbesondere quer zu einer Längserstreckung (3, 203) des Bestrahlungssteckstücks (1, 201, 401, 601, 801, 1001, 1201, 1401, 1601, 1801, 2001, 2001^{I}, 3001, 3201, 4001) ausgerichteten,
Anlagefläche (4, 204, 404, 1404) ausgestattet ist,
die vorzugsweise durch ein Schild gebildet ist,
wobei die Anlagefläche (4, 204, 404, 1404) zur Auflage (5, 205, 605) zumindest auf einem Endbereich eines Körperöffnungskranzes, wie eines Amorbogens (23^{II}) oder wie eines Nasenstegs eines menschlichen Gesichts, insbesondere in abdichtender Weise, gestaltet ist,
und dass in einem Inneren (540) des Einführteils (2, 202, 402, 602, 1402, 1602, 1802, 1802^{I}, 2002, 3002, 4002, 4002^{I}) wenigstens eine Lichtquelle (6, 406) zu einer wenigstens posterioren Körperhöhlenbeleuchtung sitzt,
wobei insbesondere das Einführteil (2, 202, 402, 602, 1402, 1602, 1802, 1802^{I}, 2002, 3002, 4002, 4002^{I}) für eine umschließende Aufnahme des Einführteils (2, 202, 402, 602, 1402, 1602, 1802, 1802^{I}, 2002, 3002, 4002, 4002^{I}) in einer Körperhöhle (20, 20^{I}) gestaltet ist.

2. Bestrahlungssteckstück (1, 201, 401, 601, 801, 1001, 1201, 1401, 1601, 1801, 2001, 2001', 3001, 3201, 4001) nach Anspruch 1,
**dadurch gekennzeichnet, dass**
das Einführteil (2, 202, 402, 602, 1402, 1602) schnullerartig, stabartig oder zapfenartig gestaltet ist,
insbesondere als gebogener oder geschwungener Stab,
vorzugsweise einer Körperhöhlenwölbung (26, 26^{I}, 28) und/oder einer Körperhöhlenkrümmung (25, 25^{I}, 32, 34) in der Körperhöhle (20, 20^{I}) nachgebildet.

3. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Bestrahlungssteckstück (1, 201, 401, 601, 801, 1001, 1201, 1401, 1601, 1801, 2001, 2001^{I}, 3001, 3201, 4001) mit einer Energiequelle (7), z. B. über eine USB-Buchse (305, 505), verbindbar ist oder eine Energiequelle (407) umfasst, insbesondere für eine Niedervoltversorgung der Lichtquelle (6, 406).

4. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
die Lichtquelle (6, 406) sich aus einer oder mehreren LEDs (8, 560, 562, 564, 961, 961^{I}, 961^{II}, 961^{III}, 961^{IV}, 961^{V}, 961^{VI}, 961^{VII}, 961^{VIII}, 1361, 1367, 1368, 1561, 1565, 1761, 1765), insbesondere RGB-LEDs, Blaulicht-LEDs (571, 572, 573), Laser-LEDs (579) und/oder UV-Licht-LEDs, z. B. UV-C-Licht-LEDs (577, 578),
insbesondere aus einem Satz (560, 562, 564) unterschiedlicher LEDs wie einer UV-Licht-LED (577, 578) und mehreren Blaulicht-LEDs (571, 572, 573) und/oder aus mehreren typgleichen LEDs (961, 961^{I}, 961", 961^{III}, 961^{IV}, 961^{V}, 961^{VI}, 961^{VII}, 961^{VIII}, 1361, 1367, 1368, 1561, 1761),
durch die vorzugsweise unterschiedliche Lichtdichten durch Licht (15, 1415, 1415^{I}, 1417, 1418, 1615, 1615^{I}, 1617, 1617^{I}, 1618, 1618^{I}) aus verschiedenen Bereichen (10, 210, 1575, 1575^{I}, 1580, 1580^{I} 1775, 1775^{I}, 1780, 1780^{I}) des Einführteils (2, 202, 402, 602, 1402, 1602, 1802, 1802^{I}, 2002, 3002, 4002, 4002^{I}) herstellbar sind, z. B. durch von einer ersten LED zu einer anderen bzw. zweiten LED (1561, 1561^{VII}, 1565, 1565^{VIII}; 1761, 1761^{VII}; 1765, 1765^{VI}) abweichende Versorgungsströme und/oder z. B. durch in einem ersten Bereich dichter angeordnete (1570, 1770, 1770^{I}) und in einem zweiten Bereich weiter beabstandete (1569, 1769, 1769^{I}, 1774) LEDs,
bildet.

5. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Bestrahlungssteckstück (1, 201, 401, 601, 801, 1001, 1201, 1401, 1601, 1801, 2001, 2001^{I}, 3001, 3201, 4001) eine Zeitsteuerung (9, 409) und/oder eine Temperaturüberwachung (581) umfasst, durch die eine Beleuchtungsdauer, während der die Lichtquelle (6, 406) Licht (15), wie sichtbares oder nicht sichtbares Licht, und/oder UV-Strahlen abgibt, zeitlich und/oder leistungsmäßig begrenzt wird, insbesondere mit zwei unterschiedlichen LED-Betriebsphasen,
vorzugsweise einer längeren für LEDs (571, 572, 573),
die im sichtbaren Wellenspektrum Licht ausstrahlen, und
einer hierzu kürzeren für wenigstens eine LED (577, 578),
die eine Wellenfront oder Strahlung jenseits des sichtbaren Wellenspektrums aussendet.

6. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Bestrahlungssteckstück (1, 201, 401, 601, 801, 1001, 1201, 1401, 1601, 1801, 2001, 2001^{I}, 3001, 3201, 4001) ein Batterie- bzw. Akkumulatorfach (510) im außerhalb eines menschlichen Körpers anzusiedelnden Bereich des Bestrahlungssteckstück (1, 201, 401, 601, 801, 1001, 1201, 1401, 1601, 1801, 2001, 3001, 3201, 4001) umfasst, in das ein Akkumulator oder eine Batterie zur elektrischen Versorgung der Lichtquelle (6, 406) einlegbar oder eingebaut, z. B. eingelötet, ist.

7. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
die Lichtquelle (6, 406), insbesondere neben wenigstens einer weiteren Wellenlänge, für eine Abgabe von Licht (15, 1415, 1415^{I}, 1417, 1418, 1615, 1615^{I}, 1617, 1617^{I}, 1618, 1618^{I}) in einem Wellenlängenbereich zwischen 400 nm und 490 nm, vorzugsweise von Licht mit einer Wellenlänge von ca. 405 nm, von ca. 410 nm und/oder einem Licht mit einer Wellenlänge von ca. 453 nm und/oder einem Licht mit einer Wellenlänge von ca. 470 nm, gestaltet ist.

8. Bestrahlungssteckstück (1, 201, 401, 601, 801, 1001, 1201, 1401, 1601, 1801, 2001, 2001^{I}, 3001, 3201, 4001) nach einem der Ansprüche 1 bis 7,
**dadurch gekennzeichnet, dass**
die Lichtquelle (6, 406) für eine Abgabe von Licht (15) bzw. Strahlung in einem UV-Wellenlängenbereich, z. B. von 222 nm, gestaltet ist.

9. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Einführteil (2, 202, 402, 602, 1402, 1602, 1802, 1802^{I}, 2002, 3002, 4002, 4002^{I}) für ein gerichtet von der Lichtquelle (6, 406) abgestrahltes Licht (15) ausgestaltet ist, wobei vorzugsweise ein Spitzenbereich des Einführteils (2, 202, 402, 602, 1402, 1602, 1802, 1802^{I}, 2002, 3002, 4002, 4002^{I}) als Lichtabgabebereich (10, 210, 1580, 1580^{I}, 1780, 1780^{I}) vorgesehen ist.

10. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Bestrahlungssteckstück (1, 201, 401, 601, 801, 1001, 1201, 1401, 1601, 1801, 2001, 2001^{I}, 3001, 3201, 4001) einen knopfartigen Korpus (11, 611), einen kastenartigen Korpus (260, 860, 1660) und/oder einen blockartigen Korpus (458, 1258, 1458), insbesondere als Griff (264, 464), hat,
der jenseits der Anlagefläche (4, 204, 404) angesiedelt ist,
wobei insbesondere der Korpus, wie knopfartiger, kastenartiger oder blockartiger Korpus (11, 260, 458, 611, 1411), mit einem elektrischen Anschluss (12, 212), wie z. B. einem Kabel, eine Buchse (305, 505) oder einem Stecker, ausgestattet ist.

11. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Einführteil (2, 202, 402, 602, 1402, 1602, 1802, 1802^{I}, 2002, 3002, 4002, 4002^{I}, 5002, 6002) nachgiebig und/oder flüssigkeitsdicht ist, z. B. aufgrund einer Kunststoffhülle (282, 482, 1482) wie z. B. aus einem Silikon oder z. B. aufgrund einer Latex-Hülle
und insbesondere der Verwahrkasten (11, 260, 458, 611, 860, 1258, 1411, 1458, 1660, 1860, 2060, 3060, 3260, 4058) mit einer zweiten Hülle (1888) abgedeckt ist,
die vorzugsweise mit der ersten Hülle (276, 476, 676, 1476, 1676, 1876, 1876^{I}, 2076, 2076', 4076, 4076^{I}) eine das Bestrahlungssteckstück (1, 201, 401, 601, 801, 1001, 1201, 1401, 1601, 1801, 2001, 2001^{I}, 3001, 3201, 4001, 5001) vollständig umschließende Feuchtigkeitsbarriere bildet.

12. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Bestrahlungssteckstück (1, 201, 401, 601, 801, 1001, 1201, 1401, 1601, 1801, 2001, 2001', 3001, 3201, 4001) einen aus zwei Halbschalen (13, 14, 213, 214), z. B. oval gestalteten Querkörper, symmetrisch gestaltetes Einführteil (2, 202, 402, 602, 1402, 1602, 1802, 1802^{I}, 2002, 3002, 4002, 4002^{I}) hat,
der insbesondere aufgrund von Druck oder Unterdruck eine formadaptive Oberfläche zu einer Körperhöhle (20, 20^{I}) eines Nutzers ausbilden kann.

13. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Einführteil (2, 202, 402, 602, 1402, 1602, 1802, 1802^{I}, 2002, 3002, 4002, 4002^{I}) verschiebbar ist,
insbesondere verlängerbar und/oder deplatzierbar ist in Bezug auf einen Körperöffnungsbezugspunkt (269) des Bestrahlungssteckstücks (1, 201, 401, 601, 801, 1001, 1201, 1401, 1601, 1801, 2001, 2001^{I}, 3001, 3201, 4001),
oder das Einführteil (2, 202, 402, 602, 1402, 1602, 1802, 1802^{I}, 2002, 3002, 4002, 4002^{I}) ,
insbesondere durch ein Aufspreizen oder ein Zusammendrücken von haltenden Klippnasen (534", 959, 959^{I}, 1159, 1159^{I}), z. B. durch zwei entgegengesetzt gerichtete Bewegungen, auswechselbar ist,
wodurch vorzugsweise das Bestrahlungssteckstück (1, 201, 401, 601, 801, 1001, 1201, 1401, 1601, 1801, 2001, 3001, 3201, 4001, 5001, 6001) in seiner Länge (203) veränderbar ist.

14. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
an dem Bestrahlungssteckstück (1, 201, 401, 601, 801, 1001, 1201, 1401, 1601, 1801, 2001, 3001, 3201, 4001), insbesondere in einem seitlichen Bereich des Korpus, ein Einschaltknopf, z. B. ein zeitlich verriegelnder Einschalttaster (301, 501, 701, 1501, 1701), vorhanden ist.

15. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Bestrahlungssteckstück (1, 201, 401, 601, 801, 1001, 1201, 1401, 1601, 1801, 2001, 2001', 3001, 3201, 4001) einen LED-Träger (550, 950, 1150, 1350, 1550, 1750, 1950, 2150, 3150, 3350) aufweist, auf dem LEDs zumindest bidirektional, vorzugsweise tridirektional, tetradirektional oder pentadirektional, angeordnet sind.

16. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
an dem Einführteil (2, 202, 402, 602, 1402, 1602, 1802, 1802^{I}, 2002, 3002, 4002, 4002^{I}) ein Einklemmbereich, wie ein Bissbereich, ein Schließmuskelbereich oder ein Muskelanpassbereich, vorhanden ist, der sich insbesondere näher bei einem Körperöffnungsschutz (268, 468, 668, 1468, 1668) als bei einem Ende (299, 1699) des Einführteils (2, 202, 402, 602, 1402, 1602, 1802, 1802^{I}, 2002, 3002, 4002, 4002^{I}) befindet und an dem insbesondere das Einführteil (2, 202, 402, 602, 1402, 1602, 1802, 1802^{I}, 2002, 3002, 4002, 4002^{I}) gehalten werden kann, wobei der Einklemmbereich vorzugsweise als eine Verstärkung und/oder eine Einschnürung der transluzenten Hülle (276, 476, 676, 1476, 1676, 1876, 1876^{I}, 2076, 2076^{I}, 4076, 4076^{I}) ausgebildet ist.

17. Bestrahlungssteckstück (1, 201, 401, 601, 801, 1001, 1201, 1401, 1601, 1801, 2001, 2001^{I}, 3001, 3201, 4001) nach einem der vorhergehenden Ansprüche,
**dadurch gekennzeichnet, dass**
das Einführteil (2, 202, 402, 602, 1402, 1602, 1802, 1802^{I}, 2002, 3002, 4002, 4002^{I}) zu einem modularen Set gehört, wobei die Einführteile (2, 202, 402, 602, 1402, 1602, 1802, 1802^{I}, 2002, 3002, 4002, 4002^{I}) des Sets gegeneinander auswechselbar sind und das Set insbesondere mindestens ein Mundhöhleneinführteil, mindestens ein Analeinführteil und vorzugsweise mindestens ein Vaginaleinführteil oder mindestens ein Ohreinführteil und/oder mindestens ein Naseneinführteil umfasst, wobei vorzugsweise ein erstes Einführteil (2, 202, 402, 602, 1402, 1602, 1802, 1802^{I}, 2002, 3002, 4002, 4002^{I}) des Sets einen kleineren Einführdurchmesser aufweist als ein Einführdurchmesser eines zweites Einführteils (2, 202, 402, 602, 1402, 1602, 1802, 1802^{I}, 2002, 3002, 4002, 4002^{I}) des Sets.

18. Verfahren zur, insbesondere temporären, Verwendung eines Bestrahlungssteckstücks (1, 201, 401, 601, 801, 1001, 1201, 1401, 1601, 1801, 2001, 3001, 3201, 4001),
insbesondere eines Bestrahlungssteckstücks (1, 201, 401, 601, 801, 1001, 1201, 1401, 1601, 1801, 2001, 2001', 3001, 3201, 4001) nach einem der Ansprüche 1 bis 17,
mit einem,
vorzugsweise auf eine Körperhöhle (20) in seiner Länge abgestimmten,
Einführteil (2, 202, 402, 602, 1402, 1602, 1802, 1802^{I}, 2002, 3002, 4002, 4002^{I}),
in dem eine Lichtquelle (6, 406) im Inneren (540) vorhanden ist,
**dadurch gekennzeichnet, dass**
das Bestrahlungssteckstück (1, 201, 401, 601, 801, 1001, 1201, 1401, 1601, 1801, 2001, 3001, 3201, 4001) als mobiles Desinfektionsgerät gestaltet ist, bei dem LEDs (8, 571, 572, 573, 577, 578, 579, 961, 1361, 1367, 1368, 1561, 1565, 1761, 1765) als Lichtquelle (6, 406) ein gerichtetes Licht (15, 1415, 1415^{I}, 1417, 1418, 1615, 1615^{I}, 1617, 1617', 1618, 1618^{I}) von dem Einführteil (2, 202, 402, 602, 1402, 1602, 1802, 1802^{I}, 2002, 3002, 4002, 4002^{I}) weg abstrahlen können,
wobei
durch Austausch eines ersten Einführteils (2, 202, 402, 602, 1402, 1602, 1802, 1802^{I}, 2002, 3002, 4002, 4002^{I}) durch ein zweites Einführteil eine Länge des Bestrahlungssteckstücks (1, 201, 401, 601, 801, 1001, 1201, 1401, 1601, 1801, 2001, 3001, 3201, 4001) angepasst wird.
